# EUROPÄISCHE PATENTANMELDUNG

(11) **EP 1 336 850 A1**
(43) Veröffentlichungstag der Anmeldung: **20.08.2003**
(21) Anmeldenummer: 03004839.1
(22) Anmeldetag: 12.10.2000
(51) Int. Cl.: G01N 33/569, C07K 16/12, C07K 16/40

(54) **Verbessertes Verfahren zum Nachweis von Säure-resistenten Mikroorganismen im Stuhl**

(30) Priorität: 12.10.1999 EP 99120351; 16.03.2000 EP 00105592; 31.03.2000 EP 00107028; 10.05.2000 EP 00110110
(62) Teilanmeldung aus: 00967861.6
(71) Anmelder: Connex Gesellschaft zur Optimierung von Forschung und Entwicklung, 82152 Martinsried (DE)
(72) Erfinder: Reiter, Christian, 85757 Karlsfeld (DE); Cullmann, Gerhard, 81371 München (DE); Müller, Heidemarie, 81475 München (DE); Heppner, Petra, 82049 Pullach (DE); Haindl, Eva, 82349 Petenried (DE); Ringeis, Achim, 82166 Gräfelfing (DE)
(74) Vertreter: VOSSIUS & PARTNER

(57) **Zusammenfassung**

Die Erfindung betrifft ein Verfahren zum Nachweis einer Infektion eines Säugers mit einem Säure-resistenten Mikroorganismus, wobei man (a) eine Stuhlprobe des Säugers unter Verwendung (aa) eines Rezeptors unter Bedingungen inkubiert, die eine Komplexbildung eines Antigens aus dem Säure-resistenten Mikroorganismus mit dem Rezeptor erlauben; oder (ab) zwei unterschiedliche Rezeptoren unter Bedingungen inkubiert, die eine Komplexbildung eines Antigens aus dem Säure-resistenten Mikroorganismus mit den beiden Rezeptoren erlauben und wobei der Rezeptor gemäß (aa) oder die Rezeptoren gemäß (ab) ein Antigen spezifisch bindet/binden, das zumindest bei einem Teil der Säuger nach der Darmpassage eine Struktur aufweist, die der nativen Struktur oder der Struktur entspricht, gegen die ein Säuger nach Infektion oder Immunisierung mit dem Säure-resistenten Mikroorganismus oder einem Extrakt oder Lysat davon oder einem Protein daraus oder einem Fragment davon oder einem synthetischen Peptid Antikörper produziert; und (b) die Bildung mindestens eines Antigen-Rezeptorkomplexes gemäß (a) nachweist. Vorzugsweise ist der Säure-resistente Mikroorganismus ein Bakterium, insbesondere *Helicobacter pylori, Helicobacter hepaticus, Campylobacter jejuni* oder *Mycobacterium tuberculosis.* Ferner bevorzugt ist, daß der Rezeptor/die Rezeptoren an ein Epitop/Epitope einer Katalase bindet. Ferner betrifft die Erfindung diagnostische und pharmazeutische Zusammensetzungen und Testvorrichtungen, welche die vorgenannten Komponenten enthalten sowie diese enthaltende Verpackungen.

SEQUENZPROTOKOLL

<110> Connex Gesellschaft zur Optimierung von Forschung und Entwicklung mbH

<120> Verbessertes Verfahren zum Nachweis von
      Säure-resistenten Mikroorganismen im Stuhl

<130> D 2394 PCT

<140>
<141>

<160> 56

<170> PatentIn Ver. 2.1

<210> 1
<211> 354
<212> DNA
<213> Mus musculus

<400> 1
gaggtgcagc tgctcgagca gcctggggct gaactggcaa aacctggggc ctcagtgaag 60
atgtcctgca aggcttctgg ctacaccttt actaactact ggattcactg ggtgaaacag 120
aggcctggac agggtctgaa atggattgga tacattaatc ctgccactgg ttccacttct 180
tacaatcagg actttcagga cagggccact ttgaccgcag acaagtcctc caccacagcc 240
tacatgcagc tgaccagcct gacatctgag gactcttcag tctattactg tgcaagagag 300
gggtacgacg ggtttgactc ctggggccaa ggcaccactc tcacagtctc ctca 354

<210> 2
<211> 318
<212> DNA
<213> Mus musculus

<400> 2
gagctcgtgc tcacccagtc tccagcaatc atgtctgcat ctccagggga gaaggtcacc 60
atgacctgca gtgccagctc aagtgtaaat tacatgtact ggtaccagca gaagtcaggc 120
acctccccca aaagatggat ttatgacaca tccaaattgg cttctggagt ccctgctcgc 180
ttcagtggca gtgggtctgg gacctcttac tctctcacac tcagcagcat ggaggctgaa 240
gatgccgcca cttattactg ccagcagtgg agtagtaatc cgtacacgtt cggagggggg 300
accaagctgg agataaaa 318

<210> 3
<211> 360
<212> DNA
<213> Mus musculus

<400> 3
gaggttcagc tgcagcagtc tggggcagag cttgtgaagc ctggggcctc agtcaagttg 60
tcctgcacat cttctggctt caacattaaa gacacctatg tgcactggat gaaacagagg 120
cctgaacagg gcctggagtg gattggaaag attgatcctg cgaatggtaa aactaaatat 180
gacccgatat tccaggccaa ggccactatg acagcagacg catcctccaa tacagcctac 240
ctgcaactca gcagcctgac ttctgaggac actgccgtct attactgtgc tctccccatt 300
tattacgcta gttcctggtt tgcttactgg ggccaaggga ctctggtcac tgtctctgca 360

<210> 4
<211> 318
<212> DNA
<213> Mus musculus

<400> 4
gacattgtga tgacccagtc tcacaaattc atgtccacat cagtaggaga cagggtcagc 60
atcacctgca aggccagtca ggatgtgggt acttctgttg cctggtatca acagaaacct 120
gggcactctc ctaaattact gatttactgg acatccaccc ggcacactgg agtccctgat 180
cgcttcacag gcagtggatc tgggacagat ttcattctca ccattagcaa tgtgcagtct 240
gaagacttgg cagattattt ctgtcagcaa tatagcagct ctcccacgtt cggagggggg 300
gccaaggtgg aaataaaa 318

<210> 5
<211> 321
<212> DNA
<213> Mus musculus

<400> 5
gacatcttgc tgactcagtc tccagccatc ctgtctgtga gtccaggaga aagagtcagt 60
ttctcctgca gggccagtca gagcattggc acaagaatac actggtatca acaaagaaca 120
aatggttctc caaggcttct cataaagtat ggttctgagt ctatctctgg gatcccttcc 180
aggtttagtg gcagtggatc agggacagat tttagtctta gcatcaacag tgtcgagtct 240
gaagatattg cagattatta ctgtcaacaa agtaatacct ggccgctcac gttcggtgct 300
gggaccaagc tggagctgaa a 321

<210> 6
<211> 369
<212> DNA
<213> Mus musculus

<400> 6
gaggtgcagc tgctcgagca gtctggagct gagctggtga agcctggggc ctcagtgaag 60
atttcctgca aggcttctgg ctacgcattc agtacctcct ggatgaactg ggtgaaacag 120
aggcctggaa agggtcttga gtggattgga cggatttatc ctggagatgg agatactaac 180
tacaatggga agttcaaggg caaggccaca ctgactgcag acaaatcctc cagcacagcc 240
tacatgcaac tcaacagcct gacatctgag gactctgcgg tctacttctg tgtaagagag 300
gatgcctatt atagtaaccc ctatagtttg gactactggg gtcaaggaac ctcagtcacc 360
gtctcctca 369

<210> 7
<211> 321
<212> DNA
<213> Mus musculus

<400> 7
gagctccaga tgacccagtc tccatccagt ctgtctgcat cccttggaga cacaattacc 60
atcacttgcc atgccagtca gaacattaat gtttggttaa gctggtatca gcagaaacca 120
ggagatatcc ctaaactatt gatctataag gcttccaact tgcacacagg cgtcccatca 180
aggtttagtg gcagtggatc tggaacaggt ttcacattag tcatcagcag cctgcagcct 240
gaagacattg ccacttacta ctgtcaacag ggtcgaagtt atcctctcac gttcggtgct 300
gggaccaagc tggagctgaa a 321

<210> 8
<211> 354
<212> DNA
<213> Mus musculus

<400> 8
gaggtgcagc tgctcgagga gtctggggga ggcttagtga agcctggagg gtccctgcaa 60
ctctcctgtt cagcctctgg attcactttc agtagccatt tcatgtcttg ggttcgccaa 120
actccagaga agaggctgga gtgggtcgca tccattagta gtggtggtga cagtttctat 180
ccagacagtc tgaagggccg attcgccatc tccagagata atgccaggaa catcctgttc 240
ctgcaaatga gcagtctgag gtctgaggac tcggccatgt atttctgtac aagagactac 300

tcttggtatg ctttggacta ctggggtcaa ggaacctcag tcaccgtctc ctca 354

<210> 9
<211> 5
<212> PRT
<213> Künstliche Sequenz

<220>
<223> Beschreibung der künstlichen Sequenz: CDR

<400> 9
Asn Tyr Trp Ile His
1               5

<210> 10
<211> 17
<212> PRT
<213> Künstliche Sequenz

<220>
<223> Beschreibung der künstlichen Sequenz: CDR

<400> 10
Tyr Ile Asn Pro Ala Thr Gly Ser Thr Ser Tyr Asn Gln Asp Phe Gln
1               5                   10                  15

Asp

<210> 11
<211> 8
<212> PRT
<213> Künstliche Sequenz

<220>
<223> Beschreibung der künstlichen Sequenz: CDR

<400> 11
Glu Gly Tyr Asp Gly Phe Asp Ser
1               5

<210> 12
<211> 15
<212> DNA
<213> Künstliche Sequenz

<220>
<223> Beschreibung der künstlichen Sequenz: CDR

<400> 12
aactactgga ttcac 15

<210> 13
<211> 51
<212> DNA
<213> Künstliche Sequenz

<220>

<223> Beschreibung der künstlichen Sequenz: CDR

<400> 13
tacattaatc ctgccactgg ttccacttct tacaatcagg actttcagga c          51


<210> 14
<211> 24
<212> DNA
<213> Künstliche Sequenz

<220>
<223> Beschreibung der künstlichen Sequenz: CDR

<400> 14
gaggggtacg acgggtttga ctcc                                        24


<210> 15
<211> 10
<212> PRT
<213> Künstliche Sequenz

<220>
<223> Beschreibung der künstlichen Sequenz: CDR

<400> 15
Ser Ala Ser Ser Ser Val Asn Tyr Met Tyr
  1               5                  10


<210> 16
<211> 7
<212> PRT
<213> Künstliche Sequenz

<220>
<223> Beschreibung der künstlichen Sequenz: CDR

<400> 16
Asp Thr Ser Lys Leu Ala Ser
  1               5


<210> 17
<211> 9
<212> PRT
<213> Künstliche Sequenz

<220>
<223> Beschreibung der künstlichen Sequenz: CDR

<400> 17
Gln Gln Trp Ser Ser Asn Pro Tyr Thr
  1               5


<210> 18
<211> 30
<212> DNA
<213> Künstliche Sequenz

<220>

<223> Beschreibung der künstlichen Sequenz: CDR

<400> 18
agtgccagct caagtgtaaa ttacatgtac                                        30


<210> 19
<211> 21
<212> DNA
<213> Künstliche Sequenz

<220>
<223> Beschreibung der künstlichen Sequenz: CDR

<400> 19
gacacatcca aattggcttc t                                                 21


<210> 20
<211> 27
<212> DNA
<213> Künstliche Sequenz

<220>
<223> Beschreibung der künstlichen Sequenz: CDR

<400> 20
cagcagtgga gtagtaatcc gtacacg                                           27


<210> 21
<211> 5
<212> PRT
<213> Künstliche Sequenz

<220>
<223> Beschreibung der künstlichen Sequenz: CDR

<400> 21
Asp Thr Tyr Val His
  1               5


<210> 22
<211> 17
<212> PRT
<213> Künstliche Sequenz

<220>
<223> Beschreibung der künstlichen Sequenz: CDR

<400> 22
Lys Ile Asp Pro Ala Asn Gly Lys Thr Lys Tyr Asp Pro Ile Phe Gln
  1               5                  10                  15

Ala


<210> 23
<211> 11
<212> PRT
<213> Künstliche Sequenz

<220>
<223> Beschreibung der künstlichen Sequenz: CDR

<400> 23
Pro Ile Tyr Tyr Ala Ser Ser Trp Phe Ala Tyr
1               5                   10

<210> 24
<211> 15
<212> DNA
<213> Künstliche Sequenz

<220>
<223> Beschreibung der künstlichen Sequenz: CDR

<400> 24
gacacctatg tgcac                                                    15

<210> 25
<211> 51
<212> DNA
<213> Künstliche Sequenz

<220>
<223> Beschreibung der künstlichen Sequenz: CDR

<400> 25
aagattgatc ctgcgaatgg taaaactaaa tatgacccga tattccaggc c            51

<210> 26
<211> 33
<212> DNA
<213> Künstliche Sequenz

<220>
<223> Beschreibung der künstlichen Sequenz: CDR

<400> 26
cccatttatt acgctagttc ctggtttgct tac                                33

<210> 27
<211> 11
<212> PRT
<213> Künstliche Sequenz

<220>
<223> Beschreibung der künstlichen Sequenz: CDR

<400> 27
Lys Ala Ser Gln Asp Val Gly Thr Ser Val Ala
1               5                   10

<210> 28
<211> 7
<212> PRT
<213> Künstliche Sequenz

<220>
<223> Beschreibung der künstlichen Sequenz: CDR

<400> 28
Trp Thr Ser Thr Arg His Thr
1               5


<210> 29
<211> 8
<212> PRT
<213> Künstliche Sequenz

<220>
<223> Beschreibung der künstlichen Sequenz: CDR

<400> 29
Gln Gln Tyr Ser Ser Ser Pro Thr
1               5


<210> 30
<211> 33
<212> DNA
<213> Künstliche Sequenz

<220>
<223> Beschreibung der künstlichen Sequenz: CDR

<400> 30
aaggccagtc aggatgtggg tacttctgtt gcc                                33


<210> 31
<211> 21
<212> DNA
<213> Künstliche Sequenz

<220>
<223> Beschreibung der künstlichen Sequenz: CDR

<400> 31
tggacatcca cccggcacac t                                             21


<210> 32
<211> 24
<212> DNA
<213> Künstliche Sequenz

<220>
<223> Beschreibung der künstlichen Sequenz: CDR

<400> 32
cagcaatata gcagctctcc cacg                                          24


<210> 33
<211> 10
<212> PRT
<213> Künstliche Sequenz

<220>

<223> Beschreibung der künstlichen Sequenz: CDR

<400> 33
Gly Phe Thr Phe Ser Ser His Phe Met Ser
1               5                   10


<210> 34
<211> 16
<212> PRT
<213> Künstliche Sequenz

<220>
<223> Beschreibung der künstlichen Sequenz: CDR

<400> 34
Ser Ile Ser Ser Gly Gly Asp Ser Phe Tyr Pro Asp Ser Leu Lys Gly
1               5                   10                  15


<210> 35
<211> 9
<212> PRT
<213> Künstliche Sequenz

<220>
<223> Beschreibung der künstlichen Sequenz: CDR

<400> 35
Asp Tyr Ser Trp Tyr Ala Leu Asp Tyr
1               5


<210> 36
<211> 10
<212> PRT
<213> Künstliche Sequenz

<220>
<223> Beschreibung der künstlichen Sequenz: CDR

<400> 36
Gly Tyr Ala Phe Ser Thr Ser Trp Met Asn
1               5                   10


<210> 37
<211> 17
<212> PRT
<213> Künstliche Sequenz

<220>
<223> Beschreibung der künstlichen Sequenz: CDR

<400> 37
Arg Ile Tyr Pro Gly Asp Gly Asp Thr Asn Tyr Asn Gly Lys Phe Lys
1               5                   10                  15

Gly


<210> 38

<211> 13
<212> PRT
<213> Künstliche Sequenz

<220>
<223> Beschreibung der künstlichen Sequenz: CDR

<400> 38
Glu Asp Ala Tyr Tyr Ser Asn Pro Tyr Ser Leu Asp Tyr
1               5                   10


<210> 39
<211> 30
<212> DNA
<213> Künstliche Sequenz

<220>
<223> Beschreibung der künstlichen Sequenz: CDR

<400> 39
ggctacgcat tcagtacctc ctggatgaac                                        30


<210> 40
<211> 51
<212> DNA
<213> Künstliche Sequenz

<220>
<223> Beschreibung der künstlichen Sequenz: CDR

<400> 40
cggatttatc ctggagatgg agatactaac tacaatggga agttcaaggg c               51


<210> 41
<211> 39
<212> DNA
<213> Künstliche Sequenz

<220>
<223> Beschreibung der künstlichen Sequenz: CDR

<400> 41
gaggatgcct attatagtaa cccctatagt ttggactac                              39


<210> 42
<211> 30
<212> DNA
<213> Künstliche Sequenz

<220>
<223> Beschreibung der künstlichen Sequenz: CDR

<400> 42
ggattcactt tcagtagcca tttcatgtct                                        30


<210> 43
<211> 48
<212> DNA

<213> Künstliche Sequenz

<220>
<223> Beschreibung der künstlichen Sequenz: CDR

<400> 43
tccattagta gtggtggtga cagtttctat ccagacagtc tgaagggc 48

<210> 44
<211> 27
<212> DNA
<213> Künstliche Sequenz

<220>
<223> Beschreibung der künstlichen Sequenz: CDR

<400> 44
gactactctt ggtatgcttt ggactac 27

<210> 45
<211> 11
<212> PRT
<213> Künstliche Sequenz

<220>
<223> Beschreibung der künstlichen Sequenz: CDR

<400> 45
Arg Ala Ser Gln Ser Ile Gly Thr Arg Ile His
1               5                   10

<210> 46
<211> 7
<212> PRT
<213> Künstliche Sequenz

<220>
<223> Beschreibung der künstlichen Sequenz: CDR

<400> 46
Tyr Gly Ser Glu Ser Ile Ser
1               5

<210> 47
<211> 9
<212> PRT
<213> Künstliche Sequenz

<220>
<223> Beschreibung der künstlichen Sequenz: CDR

<400> 47
Gln Gln Ser Asn Thr Trp Pro Leu Thr
1               5

<210> 48
<211> 11
<212> PRT

<213> Künstliche Sequenz

<220>
<223> Beschreibung der künstlichen Sequenz: CDR

<400> 48
His Ala Ser Gln Asn Ile Asn Val Trp Leu Ser
1               5                   10


<210> 49
<211> 7
<212> PRT
<213> Künstliche Sequenz

<220>
<223> Beschreibung der künstlichen Sequenz: CDR

<400> 49
Lys Ala Ser Asn Leu His Thr
1               5


<210> 50
<211> 9
<212> PRT
<213> Künstliche Sequenz

<220>
<223> Beschreibung der künstlichen Sequenz: CDR

<400> 50
Gln Gln Gly Arg Ser Tyr Pro Leu Thr
1               5


<210> 51
<211> 33
<212> DNA
<213> Künstliche Sequenz

<220>
<223> Beschreibung der künstlichen Sequenz: CDR

<400> 51
agggccagtc agagcattgg cacaagaata cac                                33


<210> 52
<211> 21
<212> DNA
<213> Künstliche Sequenz

<220>
<223> Beschreibung der künstlichen Sequenz: CDR

<400> 52
tatggttctg agtctatctc t                                             21


<210> 53
<211> 27
<212> DNA

<213> Künstliche Sequenz

<220>
<223> Beschreibung der künstlichen Sequenz: CDR

<400> 53
caacaaagta atacctggcc gctcacg 27


<210> 54
<211> 33
<212> DNA
<213> Künstliche Sequenz

<220>
<223> Beschreibung der künstlichen Sequenz: CDR

<400> 54
catgccagtc agaacattaa tgtttggtta agc 33


<210> 55
<211> 21
<212> DNA
<213> Künstliche Sequenz

<220>
<223> Beschreibung der künstlichen Sequenz: CDR

<400> 55
aaggcttcca acttgcacac a 21


<210> 56
<211> 27
<212> DNA
<213> Künstliche Sequenz

<220>
<223> Beschreibung der künstlichen Sequenz: CDR

<400> 56
caacagggtc gaagttatcc tctcacg 27

## Beschreibung

In der Beschreibung dieser Erfindung ist eine Anzahl von publizierten Dokumenten genannt. Der Gegenstand dieser Dokumente ist durch Bezugnahme in die Beschreibung inkorporiert.

Die Erfindung betrifft ein Verfahren zum Nachweis einer Infektion eines Säugers mit einem Säure-resistenten Mikroorganismus, wobei man (a) eine Stuhlprobe des Säugers unter Verwendung (aa) eines Rezeptors unter Bedingungen inkubiert, die eine Komplexbildung eines Antigens aus dem Säure-resistenten Mikroorganismus mit dem Rezeptor erlauben; oder (ab) zwei unterschiedliche Rezeptoren unter Bedingungen inkubiert, die eine Komplexbildung eines Antigens aus dem Säure-resistenten Mikroorganismus mit den beiden Rezeptoren erlauben und wobei der Rezeptor gemäß (aa) oder die Rezeptoren gemäß (ab) ein Antigen spezifisch bindet/binden, das zumindest bei einem Teil der Säuger nach der Darmpassage eine Struktur aufweist, die der nativen Struktur oder der Struktur entspricht, gegen die ein Säuger nach Infektion oder Immunisierung mit dem Säure-resistenten Mikroorganismus oder einem Extrakt oder Lysat davon oder einem Protein daraus oder einem Fragment davon oder einem synthetischen Peptid Antikörper produziert; und (b) die Bildung mindestens eines Antigen-Rezeptorkomplexes gemäß (a) nachweist. Vorzugsweise ist der Säure-resistente Mikroorganismus ein Bakterium, insbesondere *Helicobacter pylori, Helicobacter hepaticus, Campylobacter jejuni* oder *Mycobacterium tuberculosis*. Ferner bevorzugt ist, daß der Rezeptor/die Rezeptoren an ein Epitop/Epitope einer Katalase, Metalloproteinase oder Urease bindet. Ferner betrifft die Erfindung diagnostische und pharmazeutische Zusammensetzungen und Testvorrichtungen, die die vorgenannten Komponenten enthalten sowie diese enthaltende Verpackungen.

Der Nachweis der Infektion eines Säugerorganismus mit einem mikrobiellen Pathogen oder Parasiten kann heute auf verschiedene, invasive, semi-invasive oder nicht-invasive Arten geführt werden. Alle invasiven Methoden setzen eine Endoskopie und Biopsie voraus. Beim Einsatz dieser Techniken wird die körperliche Integrität des Untersuchten verletzt, z.B. bei der Entnahme einer Biopsie. Eine Entnahme von Biopsie ist aufwendig, verusacht hohe Kosten und bedeutet meist eine große Belastung für den Patienten. Da die Infektion mit bestimmten Mikroorganismen, beispielsweise mit *H. pylori* nicht über die gesamte Magenschleimhaut verteilt sein muß, ist durch Biopsie-Entnahme an einer nichtinfizierten Stelle ein falsch-negatives Ergebnis möglich. Ein weiterer Nachteil aller invasiven Methoden ist die Beeinflussung der Untersuchungsergebnisse durch frühere Behandlung mit Protonenpumpen-Hemmern, Wismut oder Antibiotika.

Semi-invasive oder nicht-invasive Diagnosetechniken stellen Veränderungen in Parametern fest, die ohne einen Eingriff in den Organismus gemessen werden können. Bevorzugt werden hierzu Körperflüssigkeiten und Ausscheidungen wie Serum, Atemluft, Urin, Speichel, Schweiß oder Stuhl, beprobt und analysiert. Bei direkten Methoden wird das Vorhandensein des Pathogens oder Parasiten, seiner Bestandteile oder deren Abbauprodukte durch Elektronenmikroskopie, optische Charakterisierung, Massenspektrometrie, Messung der radioaktiven Zerfallsprodukte oder spezifische enzymatische Reaktionen nachgewiesen. Oft sind diese Verfahren jedoch mit hohem apparativem Aufwand verbunden (z.B. Atemtest). Indirekte Verfahren greifen dagegen auf den Nachweis von Reaktionen des Wirtsorganismus auf das Pathogen oder den Parasiten zurück, z.B. das Vorhandensein von Antikörpern gegen Antigene des Pathogens im Serum oder im Speichel des Wirts.

Nachdem der Eingriff in den Organismus bei invasiven Techniken für den Organismus in den meisten Fällen belastend und häufig auch mit hohem apparativen Aufwand sowie einem gesundheitlichen Risiko verbunden ist, stellen nicht-invasive Techniken durch die vergleichsweise einfache Erfassung von Proben der oben beschriebenen Körperflüssigkeiten und Ausscheidungen die Methode der Wahl dar. Da weiterhin nicht jeder Wirt in gleicher Weise auf ein bestimmtes Pathogen oder einen bestimmten Parasiten reagiert, und die Reaktion des Wirts mit Verzögerung einsetzen und zudem auch nach Entfernung des Pathogens oder Parasiten aus dem Organismus persistieren kann, sind direkte Verfahren stets vorzuziehen. Idealerweise wird also eine Diagnose durch den nicht-invasiven, direkten Nachweis des Pathogens oder Parasiten in Körperflüssigkeiten oder Ausscheidungen geführt. Dies ermöglicht im Gegensatz zu indirekten Verfahren die Bestimmung des aktuellen Infektionsstatus.

Ein diagnostisches Verfahren sollte darüber hinaus auch auf weitere Gesichtspunkte hin optimiert sein: Hohe Reproduzierbarkeit, Sensitivität und Spezifität, garantierte Verfügbarkeit der zu verwendenden Materialien in konstanter Qualität, geringe Kosten bei Herstellung und Durchführung, und einfache Anwendung unabhängig von aufwendigen Apparaturen sind die hier zu berücksichtigenden Parameter.

Aus den oben genannten Gründen nehmen Verfahren basierend auf der hohen Selektivität und Bindungsaffinität bestimmter Substanzklassen (z.B. Antikörper, Rezeptoren, Lektine, Aptamere) für molekulare Strukturen, welche so gewählt werden können, daß sie für den jeweils zu bestimmenden Stoff hochspezifisch sind, in der medizinischen Diagnostik einen zunehmend breiten Raum ein. Insbesondere die Möglichkeit der Immobilisierung dieser Substanzen an Festkörperoberflächen sowie der Kopplung radioaktiver Nuklide, von Enzymen, die mit geeigneten Substraten Farbreaktionen auslösen oder farbiger Partikel mit der hochspezifischen Bindungsaffinität (z.B. im ELISA = Enzyme linked immunosorbent assay) führte zu der Entwicklung kostengünstiger, einfacher und wenig zeitaufwendiger Nachweisverfahren für körpereigene wie körperfremde Stoffe.

In den Anfangsphasen der Entwicklung dieser Nachweisverfahren fanden ausschließlich polyklonale Antikörper Verwendung. Diese haben jedoch einige, dem Fachmann wohlbekannte Nachteile, so vor allem begrenzte Verfügbarkeit und oft auch Kreuzreaktivitäten. Die Erarbeitung von Verfahren zur Herstellung monoklonaler Antikörper (Köhler & Milstein (1975)), die Fortschritte bei der Isolierung von Rezeptoren und deren gerichtete Expression in zellulären Wirtssystemen, die Entwicklung von Lektinen mit hoher Affinität für bestimmte Kohlenhydrate sowie die Entdeckung, daß einzelsträngige Nukleinsäure-Moleküle (Aptamere) molekulare Strukturen spezifisch binden können, konnten diese Nachteile großenteils beseitigen. Heute lassen sich mit vergleichsweise einfachen Methoden die Spezifität und Sensitivität von Nachweisverfahren optimieren.

Aufgrund der hohen Spezifität eignen sich derartige Verfahren besonders zum Nachweis einzelner, definierter Substanzen wie Haptene, Peptide oder Proteine, vorausgesetzt, das erkannte Strukturelement ist konstant innerhalb der zu untersuchenden Probenpopulation und spezifisch für die nachzuweisende Substanz. Sie sind zudem für eine Messung in Körperflüssigkeiten gut geeignet, und stellen damit eine naheliegende Option für den direkten Nachweis von Pathogenen in dieser Probenmatrix dar. Entsprechend sind im Stand der Technik Verfahren zur Diagnose z.B. von *Entamoeba histolytica* (Haque (1993), J. Infect. Dis. 167: 247-9), enterohemorrhagische *Escherichia coli* (EHEC; Park (1996), J. Clin. Microbiol. 34: 988-990), *Vibrio cholerae* (Hasan (1994), FEMS Microbiol. Lett. 120: 143-148), Torovirus-ähnliche Partikel (Koopmans (1993), J. Clin. Microbiol. 31: 2738-2744) oder *Taenia saginata* (Machnicka (1996), Appl. Parasitol. 37: 106-110) aus Stuhl beschrieben.

Den oben beschriebenen Pathogenen ist gemeinsam, daß sie im Darm ihres Wirts, in allen Fällen dem Menschen, lebens- und vermehrungsfähig sind. Sie besitzen also Mechanismen, die Ihnen das Überleben und die Vermehrung in Anwesenheit der im Darm aktiven Abbau- und Verdauungssysteme erlauben. Damit ist es wahrscheinlich, daß bei der Ausscheidung mit dem Stuhl eine hohe Anzahl intakter oder fast intakter Pathogene bzw. Parasiten abgehen. Mit Nachweisreagenzien, beispielsweise Antikörpern, welche die intakten Pathogene bzw. Parasiten erkennen, können diese im Stuhl oder in aufbereiteten Stuhlproben in der Regel leicht nachgewiesen werden.

Es gibt jedoch eine Anzahl von Pathogenen und Parasiten, die einerseits aufgrund der Beziehungen der von ihnen befallenen Gewebe (z.B. Lunge, Magen, Pankreas, Duodenum, Leber) zum Magen-Darm-Trakt im Stuhl auftreten können, andererseits aber im Darm selbst nicht lebens- und/oder vermehrungsfähig sind. Zu diesen Pathogenen und Parasiten gehören beispielsweise *Helicobacter pylori (H. pylori)* und *Helicobacter hepatis, Mycobacterium tuberculosis* und andere Mycobakterien, *Chlamydia pneumoniae, Legionella pneumophilae, Pneumocystis carinii,* und andere. Einige dieser Erreger können z.B. im Sputum nachgewiesen werden, jedoch ist beispielsweise der Nachweis von *Mycobacterium tuberculosis* im Sputum nur während eines kurzen Zeitraums möglich, und zwar nachdem sich eine den Erreger enthaltende Kaverne geöffnet hat. Ein Nachweis wird ferner dadurch erschwert, daß es nicht immer möglich ist, von einem zu Untersuchenden eine Sputumprobe zu erhalten. Dies trifft z.B bei Kleinkindern, verwirrten Patienten oder Tieren zu. Andere Pathogene wie beispielsweise *Legionella pneumophilae* lassen sich anhand von Antigenen, die über die Niere in den Urin gelangen spezifisch nachweisen. Dies gelingt jedoch nur, wenn die im Urin befindliche Menge für den Nachweis ausreicht. Der Nachweis im Stuhl wäre hierzu eine begrüßenswerte Alternative. Die Darmpassage ist bei diesen Organismen jedoch mit einem starken Angriff durch die Verdauungs- und Abbaumechanismen der Darmflora verbunden. Für den betrachteten Erreger spezifische molekulare Strukturen können dabei zerstört oder in ihrer Konzentration stark herabgesetzt werden.

Die Degradation der Erreger im Darm hat sich auch bei anderen Säure-resistenten Bakterien als Problem für einen sicheren Nachweis in Stuhlproben erwiesen. Die Zahl der Keime im Magen eines Infizierten ist im Vergleich zu anderen, sich im Darm ansiedelnden Bakterien, gering. Zudem müssen Keime und Keimbruchstücke nach Verlassen des Magens einen langen Weg durch den an Proteasen reichen Darm zurücklegen. Diese Umstände haben zur Folge, daß sich nur geringe Mengen intakter Proteine im Stuhl wiederfinden lassen, wobei man nicht davon ausgehen kann, daß immer die gleichen Fragmente bestimmter Proteine den Darmtrakt unbeschadet durchlaufen. Dies bedingt auch, daß die für einen ELISA-Test nötige Kombination zweier Epitope auf einem Antigen nicht mehr notwendigerweise wie im nativen Protein gegeben ist, und nah nebeneinander liegende Epitope die größte Wahrscheinlichkeit haben, in einem Nachweis, der zwei Epitope auf dem selben Molekül benötigt, ein positives Ergebnis zu zeigen. Idealerweise wird zum Nachweis nur ein Epitop auf dem selben Molekül benötigt. Die individuell unterschiedliche Verteilung von im Stuhl von Infizierten nachgewiesenen Antigenen weist zusätzlich auf individuelle Charakteristika in der Prozessierung der Antigene beim Darmdurchgang hin. Ein erster Ansatz, dieses Problem zu verringern, wurde durch den Offenbarungsgehalt der EP-A 0 806 667 bereitgestellt. In dieser Anmeldung wurde gezeigt, daß polyklonale Antikörper mit dem Lysat eines bestimmten *H. pylori*-Stammes induziert werden konnten, die eine breitere Variabilität von Stämmen aus verschiedenen geographischen Regionen erkennen. Allerdings geht aus dieser Anmeldung nicht hervor, welche Antigene durch das Serum erkannt werden. Angesichts der Tatsache, daß Immunseren trotz aller Standardisierungsbemühungen schwanken können, muß das in der o.g. Anmeldung entwickelte Verfahren für eine breite Anwendung als suboptimal betrachtet werden. Hinzu kommt, daß für die Bereitstellung der polyklonalen Seren immer wieder neue Tiere immunisiert werden müssen. Die entsprechenden Verfahren sind sowohl zeit- als auch kostenintensiv.

Idealerweise wäre ein sicherer Nachweis der Infektion eines wie oben erweiterten Säure-resistenten pathogenen Organismus/Parasiten mit einem einzigen oder einer limitierten Anzahl für diesen pathogenen Organismus/Parasiten spezifischen Reagens/Reagentien möglich. Eine derartige Möglichkeit würde vor allem die Kosten für entsprechende Nachweisverfahren deutlich herabsetzen. Aufgabe der vorliegenden Erfindung war somit, ein entsprechendes Nachweisverfahren bzw. entsprechende Reagentien bereitzustellen.

Diese Aufgabe wird durch die in den Ansprüchen gekennzeichneten Ausführungsformen gelöst.

Somit betrifft die Erfindung ein Verfahren zum Nachweis einer Infektion eines Säugers mit einem Säure-resistenten Mikroorganismus, wobei man (a) eine Stuhlprobe des Säugers unter Verwendung (aa) eines Rezeptors unter Bedingungen inkubiert, die eine Komplexbildung eines Antigens aus dem Säure-resistenten Mikroorganismus mit dem Rezeptor erlauben; oder (ab) zwei unterschiedliche Rezeptoren unter Bedingungen inkubiert, die eine Komplexbildung eines Antigens aus dem Säure-resistenten Mikroorganismus mit den beiden Rezeptoren erlauben und wobei der Rezeptor gemäß (aa) oder die Rezeptoren gemäß (ab) ein Antigen spezifisch bindet/binden, das zumindest bei einem Teil der Säuger nach der Darmpassage eine Struktur aufweist, die der nativen Struktur oder der Struktur entspricht, gegen die ein Säuger nach Infektion oder Immunisierung mit dem Säure-resistenten Mikroorganismus oder einem Extrakt oder Lysat davon oder einem Protein daraus oder einem Fragment davon oder einem synthetischen Peptid Antikörper produziert; und (b) die Bildung mindestens eines Antigen-Rezeptorkomplexes gemäß (a) nachweist.

Der Begriff "Säure-resistenter Mikroorganismus" im Sinne dieser Erfindung umfaßt jeden Mikroorganismus, der aufgrund seiner Eigenschaften/Anpassungsmechanismen an den Wirt den physikalischen und chemischen Einflüssen des Verdauungstrakts widersteht, so daß er durch einen vorzugsweise immunologischen Nachweis oder unter Verwendung von Aptameren detektierbar ist. Beispiele für derartige Säure-resistente Mikroorganismen sind *Helicobacter pylori, Helicobacter hepaticum, Mycobacterium tuberculosis, Mycobacterium pseudotuberculosis und Mycobacterium cansassii*.

Der Begriff "Stuhlprobe des Säugers" bedeutet im Sinne dieser Erfindung jede Stuhlprobe, die für das erfindungsgemäße Nachweisverfahren eingesetzt werden kann. Insbesondere fallen darunter Stuhlproben, die nach an sich bekannten Verfahren für diagnostische Tests aufbereitet worden sind. Die Aufbereitung erfolgt beispielsweise gemäß RIDASCREEN® Entamoeba Enzymimmunoassay (R-Biopharm GmbH, Darmstadt).

"Bedingungen, die eine Komplexbildung erlauben" sind vom Fachmann ohne weiteres einstellbar; s. auch Harlow und Lane, a.a.O., und sind beispielsweise physiologische Bedingungen.

Der Begriff "nach der Darmpassage eine Struktur aufweist, die der nativen Struktur entspricht" bedeutet im Sinne dieser Erfindung, daß das Epitop eines Antigens nach der Darmpassage von einem Rezeptor, beispielsweise einem monoklonalen Antikörper, Derivat oder Fragment davon oder dem Aptamer erkannt wird, der/das gegen dasselbe Antigen/Epitop gewonnen wurde oder an dieses bindet, welches die Darmpassage nicht passiert hat. Mit anderen Worten, das Epitop/Antigen, das vom o.g. Rezeptor spezifisch gebunden wird, hat die Darmpassage hinsichtlich seiner Struktur unbeschadet oder im wesentlichen unbeschadet überstanden und ist nicht degradiert worden. Als Quelle für die native Struktur des Epitops/Antigens kann z.B. ein mit einer French-Press aufgeschlossener Bakterienextrakt, der mit üblichen Verfahren (vgl. z.B. Sambrook et al., "Molecular Cloning, A Laboratory Manual", 2. Auflage 1989, CSH Press, Cold Spring Harbor, USA) weiter aufgereinigt wurde, oder ein Bakterienlysat, das nach Standardverfahren weiter aufgereinigt wurde (z.B. Sambrook et al., a.a.O.), dienen.

Der Begriff "nach der Darmpassage eine Struktur aufweist, die der Struktur entspricht, gegen die ein Säuger nach Infektion oder Immunisierung mit dem Säure-resistenten Mikroorganismus oder einem Extrakt oder Lysat davon oder einem Protein daraus oder einem Fragment davon oder einem synthetischen Peptid Antikörper produziert" bedeutet erfindungsgemäß, daß das vom Rezeptor erkannte Epitop einem Epitop entspricht, das vom Immunsystem eines Säugers, vorzugsweise eines Menschen, präsentiert wird. Die Mechanismen der Antigen-Präsentation sowie Mechanismen, die zur Prozessierung von Antigenen führen und die daraus resultierende Antikörpervielfalt sind im Stand der Technik bekannt und beispielsweise in Janeway und Travers, Immunologie, 2. Auflage 1997, Spektrum Akademischer Verlag GmbH, Heidelberg beschrieben. Diese Epitope können sich von den nativen Epitopen unterscheiden. Der Kontakt des Säugers mit den Mikroorganismen oder den Proteinen oder Fragmenten oder den synthetischen Peptiden kann durch natürliche Infektion (außer bei den synthetischen Peptiden) oder durch Immunisierung erfolgen. Für die Immunisierung können auch Extrakte, Lysate, synthetische Peptide etc. des Mikroorganismus/Proteins herangezogen werden. Geeignete Immunisierungsschemata sind im Stand der Technik bekannt und beispielsweise beschrieben in Harlow und Lane, a.a.O. Geeignete Antikörper können beispielsweise auch durch Immunisierung und/oder Screening auf Surrogate wie synthetische Peptide, rekombinant hergestellte Proteine, Extrakte, Lysate oder partiell verdaute Proteine gewonnen werden.

"Synthetische Peptide" umfassen solche Peptide, die mindestens ein Epitop des nativen oder durch den Darm passagierten Antigens aufweisen. Die Peptide können dabei dieselbe Primärstruktur wie das Antigen oder Fragmente davon aufweisen. Sie können jedoch auch eine andere Primärstruktur (primäre Aminosäuresequenz, z.B. konservative Austausche) aufweisen.

Der Begriff "spezifisch bindet" bedeutet erfindungsgemäß, daß der Rezeptor keine oder im wesentlichen keine Reaktivität mit anderen Epitopen in Proben nicht infizierter Säuger aufweist. Üblicherweise bindet der Rezeptor nur an das eine Epitop eines Antigens, das in der Stuhlprobe auftritt.

So kann in dieser Ausführungsform der Erfindung eine aufbereitete Stuhlprobe beispielsweise an eine Festphase gebunden werden und das infizierende Agens mit dem in markierter Form vorliegenden Rezeptor nachgewiesen werden. Sofern das nach der Darmpassage vorliegende Antigen (noch) in (homo) di- oder multimerer Form vorliegt, kann der gleiche Rezeptor sowohl als Fänger wie auch als Detektor eingesetzt werden.

Von Bedeutung ist für das erfindungsgemäße Verfahren ferner, daß für einen erfolgreichen Nachweis nur ein Epitop eines antigenen Proteins im wesentlichen konsistent nach der Darmpassage nachweisbar sein muß. Dieses Epitop kann auch mehrmals auf einem Homo-Dimer oder -multimer vorkommen. Die Wahrscheinlichkeit, daß dieses Epitop in nachweisbarer Form vorzufinden ist, ist aber wesentlich höher, als wenn ein Nachweistest auf mehr als einem nachzuweisenden Epitop aufbauen muß.

Schließlich bringt das erfindungsgemäße Verfahren, das nur einen Rezeptor benötigt, Kosten- und Standardisierungsvorteile mit sich.

Basierend auf dem erfindungsgemäßen überraschenden Befund, daß bestimmte Antigene aus den genannten Mikroorganismen nach der Darmpassage eine im wesentlichen konsistent nachweisbare Epitopstruktur aufweisen, muß auch eine zweite Ausführungsform als essentiell für die Erfindung gelten. Diese Ausführungsform beruht darauf, daß verschiedene Rezeptoren an verschiedene Epitope desselben Antigens binden. Der Begriff "im wesentlichen" bedeutet dabei, daß das Epitop/die Epitope und damit eine entsprechende Infektion mit dem Mikroorganismus bei mehr als 70%, vorzugsweise mindestens 75%, stärker bevorzugt mehr als 85%, besonders bevorzugt mehr als 90%, noch stärker bevorzugt mehr als 95% und am meisten bevorzugt mehr als 98% der Betroffenen erfaßt werden kann/können. Idealerweise werden Infektionen bei 100% der Betroffenen nachgewiesen.

Überraschenderweise wurde erfindungsgemäß gefunden, daß mit einem einzigen Rezeptor, der ein Epitop eines Antigens eines Säure-resistenten Mikroorganismus spezifisch bindet, oder zwei Rezeptoren, die zwei Epitope desselben Antigens spezifisch binden, eine relativ sichere Diagnose der Infektion mit diesen Bakterien/Pathogenen durchgeführt werden kann. Die Erfindung schließt Ausführungsformen mit ein, in denen weitere Epitope, die die vorgenannten Eigenschaften aufweisen, von weiteren Rezeptoren, z.B. von monoklonalen Antikörpern oder Fragmenten oder Derivaten davon oder Aptameren erkannt werden. Letztere Ausführungsformen sind dazu geeignet, die Sicherheit bei der Stellung der Diagnose noch weiter zu erhöhen. Diese weiteren Rezeptoren können vorteilhafterweise Antikörper, Fragmente oder Derivate sein, die Urease, vorzugsweise β-Urease, das 26 kDa Protein oder Hsp 60, alle vorzugsweise aus *H*. *pylori*, spezifisch erkennen. Der Nachweis eines oder mehrerer dieser Proteine/Proteinfragmente kann im selben Test und in einem unabhängigen Test mit einem anderen Teil derselben Probe durchgeführt werden.

Die erfindungsgemäßen Ergebnisse sind vor allem deshalb überraschend, da der Stand der Technik hiervon weggelehrt hatte. So wurde beispielsweise bei *H. pylori* gefunden, daß Hauptantigene in ELISA-Tests nicht die gewünschte Spezifität und Sensitivität aufweisen; vgl. Newell et al., Serodiag. Immunother. Infect. Dis. 3 (1989), 1-6. Darüber hinaus lehrt die EP-A 0 806 667, daß ein sicherer Nachweis von *H. pylori*-Infektionen mit Rezeptoren wie monoklonalen Antikörpern aufgrund der genetischen Variabilität der *H. pylori*-Stämme nicht möglich sei.

Das erfindungsgemäße Verfahren ist gegenüber dem erwähnten Stand der Technik insbesondere deshalb von Vorteil, da mit lediglich einem Rezeptor eine relativ sichere Diagnose ermöglicht wird. Vorzugsweise werden für den Nachweis, beispielsweise im ELISA Pärchen von Rezeptoren, wie Antikörpern, Fragmenten, Derivaten davon oder Aptameren eingesetzt, wobei die beiden Rezeptoren des Pärchens dasselbe oder unterschiedliche Epitope auf demselben Antigen binden. Beispielsweise bildet *H. pylori*-Katalase multimere Strukturen aus mehreren gleichen Untereinheiten aus. Im ELISA oder anderen Assays können somit die gleichen Rezeptoren als Fängerrezeptoren wie auch als Detektionsrezeptoren eingesetzt werden. Ein weiterer Vorteil des erfindungsgemäßen Verfahrens ist seine Ausgestaltung als direktes und nicht-invasives Verfahren, was die einleitend genannten Annehmlichkeiten für den Patienten sowie die Zuverlässigkeit bei der Bestimmung des Krankheitsstadiums erhöht.

In einer bevorzugten Ausführungsform ist der Säure-resistente Mikroorganismus ein Säure-resistentes Bakterium.

Im Stand der Technik ist eine Reihe von Säure-resistenten Bakterien bekannt. In einer besonders bevorzugten Ausführungsform ist das Säure-resistente Bakterium ein Bakterium der Gattung Helicobacter, Campylobacter oder der Gattung Mycobacterium.

In einer anderen besonders bevorzugten Ausführungsform ist das Bakterium ein Bakterium der Spezies *Helicobacter pylori, Helicobacter hepaticum, Campylobacter jejuni* oder ein Bakterium der Spezies *Mycobacterium tuberculosis.*

In einer weiteren bevorzugten Ausführungsform ist der Rezeptor/sind die Rezeptoren (ein) Antikörper, (ein) Fragment(e) oder (ein) Derivat(e) davon oder (ein) Aptamer(e).

"Fragmente" oder "Derivate" von monoklonalen Antikörpern weisen im Sinne dieser Erfindung dieselbe Bindungsspezifität wie die monoklonalen Antikörper auf. Derartige Fragmente oder Derivate können nach üblichen Verfahren hergestellt werden; vgl. z.B. Harlow und Lane "Antibodies, A Laboratory Manual", CSH Press, Cold Spring Harbor, USA, 1988. Beispiele für Fragmente sind Fab-, F(ab')₂ oder Fv-Fragmente. Beispiele für Derivate sind scFv-Fragmente. Derivate können auch chemisch hergestellte Substanzen sein, die dieselben oder verbesserte Bindungseigenschaften wie die Antikörper aufweisen. Solche Substanzen können beispielsweise durch Peptidomimetics oder durch verschiedene Runden von Phage Display und nachfolgende Selektion auf verbesserte Bindungseigenschaften hergestellt werden. Unter Aptameren werden erfindungsgemäß Nukleinsäuren wie RNA, ssDNA (ss = Einzelstrang), modifizierte RNA oder modifizierte ssDNA verstanden, die eine große Vielzahl von Zielsequenzen mit hoher Spezifität und Affinität binden. Der Begriff "Aptamer" ist im Stand der Technik bekannt und definiert beispielsweise in Osborne et al., Curr. Opin. Chem. Biol. **1** (1997), 5-9, oder in Stull und Szoka, Pharm. Res. 12 (1995), 465-483 beschrieben.

Der Begriff "Antigen-Antikörperkomplex" im Sinne dieser Erfindung umfaßt nicht nur Komplexe, die das Antigen mit dem nativen Antikörper eingeht, sondern auch solche, die es mit dessen Fragmenten oder Derivaten eingeht.

Umfaßt von der Erfindung sind Ausführungsformen, bei denen nur monoklonale Antikörper oder Fragmente oder Derivate davon oder nur Aptamere eingesetzt werden, wie auch Ausführungsformen, bei denen in einem Test unterschiedliche Arten von Nachweisreagenzien eingesetzt werden. So ist es möglich, daß ein erster monoklonaler Antikörper mit einem zweiten Antikörperderivat oder ein erstes Aptamer mit einem zweiten Antikörperfragment eingesetzt wird, um nur zwei Beispiele zu nennen. Insofern bezeichnen die Begriffe "erste" und "zweite" das erste und zweite Nachweisreagens. Gemeint ist dabei nicht, daß immer zwei Antikörper, Derivate oder Fragmente davon oder immer zwei Aptamere eingesetzt werden.

Die Verwendung von monoklonalen Antikörpern, Fragmenten oder Derivaten davon oder von Aptameren gewährt einen leicht zu haltenden Standard bei der Zuverlässigkeit des Diagnoseverfahrens, was ein großer Vorteil im Vergleich zu bisher bekannten und für diesen Zweck eingeführten Diagnoseverfahren ist. Weiterhin entfällt das beispielsweise im Verfahren der EP-A 0 806 667 notwendige immer neue Immunisieren und nachfolgende Testen von Versuchstieren.

In einer weiteren bevorzugten Ausführungsform ist das Antigen das Antigen einer Katalase, vorzugsweise aus *H. pylori*. Die Katalase hat den besonderen Vorteil, daß sie in allen bisher bekannten Säure-resistenten Bakterien nachgewiesen werden konnte. Erfindungsgemäß konnte als weiterer Vorteil ermittelt werden, daß die Katalase sehr resistent gegen die Verdauung im Darmtrakt ist, was den Nachweis signifikanter Mengen vereinfacht. Schließlich liegt die Katalase oder Fragmente davon auch nach der Darmpassage oft noch höhergeordneter Struktur, z.B. in tetramerer Form vor, was den Nachweis mit nur einem Rezeptortyp erleichtert.

Überraschenderweise wurde erfindungsgemäß gefunden, daß in einer Population von Säugern, insbesondere von menschlichen Patienten, deren Stuhl auf Infektionen mit Säure-resistenten Bakterien getestet wurde, im wesentlichen alle Mitglieder dieser Population konsistent wiederkehrende Katalase-Epitope im Stuhl aufwiesen, so daß mit hoher Wahrscheinlichkeit mit nur einen entsprechenden Rezeptor, vorzugsweise monoklonalen Antikörpern, Fragmenten oder Derivaten davon oder Aptameren eine relativ sichere Diagnose gestellt werden kann. Insbesondere, da die Katalase eine tetramere antigene Struktur aufweist, kann diese Diagnose vorteilhafterweise beispielsweise im ELISA oder in ähnlich angeordneten Festsystem gestellt werden.

Besonders bevorzugt ist, daß die Katalase die Katalase von *H. pylori* ist.

In einer weiteren bevorzugten Ausführungsform ist das Antigen eine Metalloproteinase, besonders bevorzugt die Metalloproteinase aus *H. pylori*.

In einer anderen bevorzugten Ausführungsform ist das Antigen eine Urease, bevorzugt aus *H. pylori*.

In einer weiteren bevorzugten Ausführungsform wird für den Nachweis zusätzlich ein Gemisch von Rezeptoren eingesetzt, wobei das Gemisch von Rezeptoren als Fänger des Antigens fungiert, wenn der Rezeptor als Detektor des Antigens eingesetzt wird und das Gemisch als Detektor des Antigens fungiert, wenn der Rezeptor als Fänger des Antigens eingesetzt wird.

Diese Ausführungsform der Erfindung erlaubt eine besonders sichere Diagnose, insbesondere wenn das Antigen nicht in dimerer oder multimerer Konformation nach der Darmpassage vorliegt. Diese Ausführungsform erlaubt, daß nur einer der beiden in den meisten standardisierten immunologischen Nachweisverfahren eingesetzten Rezeptortypen ein monoklonaler Antikörper ist, während beispielsweise der zweite Rezeptortyp ein polyklonales Serum sein kann.

In einer besonders bevorzugten Ausführungsform ist das Gemisch von Rezeptoren ein polyklonales Antiserum.

In einer zusätzlich besonders bevorzugten Ausführungsform wurde das polyklonale Antiserum gegen ein Lysat des Mikroorganismus, vorzugsweise *H. pylori*, gewonnen.

In einer weiteren besonders bevorzugten Ausführungsform ist das Lysat ein Lysat mit angereichertem Antigen.

In einer anderen bevorzugten Ausführungsform ist das Lysat ein Lysat mit abgereichertem immundominantem Antigen.

Die vorgenannten beiden Ausführungsformen schließen auch ein, daß das Lysat ein Lysat mit angereichertem Antigen, vorzugsweise mit angereicherter Katalase wie auch mit abgereichertem immundominantem Antigen, vorzugsweise hauptantigener Urease ist. Insbesondere die genannte Kombination läßt eine gute und für das erfindungsgemäße Verfahren besonders geeignete Immunisierungsausbeute zu. Eine Art der Durchführung entsprechender Anreicherungs- bzw. Abreicherungsverfahren ist in den Beispielen näher beschrieben.

Gemäß einer weiteren besonders bevorzugten Ausführungsform wurde das polyklonale Antiserum gegen ein aufgereinigtes oder ein (semi)synthetisch hergestelltes Antigen gewonnen, das bevorzugt ein Katalase-, Urease- oder Metalloproteinase-Antigen, vorzugsweise aus *H. pylori* ist

Die Rezeptoren, vorzugsweise die monoklonalen Antikörper, Fragmente oder Derivate davon oder die Aptamere können erfindungsgemäß lineare oder Konformationsepitope erkennen und spezifisch binden. In einer weiteren bevorzugten Ausführungsform bindet mindestens einer der Rezeptoren ein Konformationsepitop.

In einer besonders bevorzugten Ausführungsform binden sämtliche Rezeptoren Konformationsepitope.

In einer besonders bevorzugten Ausführungsform weist die schwere Kette des ein Katalase-Epitop bindenden Antikörpers (HP 25.2m 2H10) mindestens eine der folgenden CDRs, vorzugsweise die CDR3 und weiter bevorzugt alle drei folgenden CDRs auf:

CDR1: NYWIH
CDR2: YINPATGSTSYNQDFQD
CDR3: EGYDGFDS

In einer weiteren besonders bevorzugten Ausführungsform weist die die schwere Kette des ein Katalase-Epitop bindenden Antikörpers (HP 25.2m 2H10) kodierende DNA-Sequenz mindestens eine der folgenden CDRs, vorzugsweise die CDR3 und weiter bevorzugt alle drei folgenden CDRs auf:

CDR1: AACTACTGGA TTCAC
CDR2: TACATTAATC CTGCCACTGG TTCCACTTCT TACAATCAGG
ACTTTCAGGA C
CDR3: GAGGGGTACG ACGGGTTTGA CTCC

In einer anderen besonders bevorzugten Ausführungsform weist die leichte Kette des ein Katalase-Epitop bindenden Antikörpers (HP 25.2m 2H10) mindestens eine der folgenden CDRs, vorzugsweise die CDR3 und weiter bevorzugt alle drei folgenden CDRs auf:

CDR1: SASSSVNYMY
CDR2: DTSKLAS
CDR3: QQWSSNPYT

Weiterhin weist in einer besonders bevorzugten Ausführungsform die die leichte Kette des ein Katalase-Epitop bindenden Antikörpers (HP 25.2m 2H10) kodierende DNA-Sequenz mindestens eine der folgenden CDRs, vorzugsweise die CDR3 und weiter bevorzugt alle drei folgenden CDRs auf:

CDR1: AGTGCCAGCT CAAGTGTAAA TTACATGTAC
CDR2: GACACATCCA AATTGGCTTC T
CDR3: CAGCAGTGGA GTAGTAATCC GTACACG

In einer besonders bevorzugten Ausführungsform weist die schwere Kette des ein Katalase-Epitop bindenden Antikörpers [HP25.6m/1B5] mindestens eine der folgenden CDRs, vorzugsweise die CDR3 und weiter bevorzugt alle drei folgenden CDRs auf:

CDR1: DTYVH
CDR2: KIDPANGKTKYDPIFQA
CDR3: PIYYASSWFAY

In einer weiteren besonders bevorzugten Ausführungsformweist die die schwere Kette des ein Katalase-Epitop bindenden Antikörpers (HP25.6m/1B5) kodierende DNA-Sequenz mindestens eine der folgenden CDRs, vorzugsweise die CDR3 und weiter bevorzugt alle drei folgenden CDRs auf:

CDR1: GACACCTATGTGCAC
CDR2: AAGATTGATCCTGCGAATGGTAAAACTAAATATGACCCGATATTC
CAGGCC
CDR3: CCCATTTATTACGCTAGTTCCTGGTTTGCTTAC

In einer anderen besonders bevorzugten Ausführungsform weist die leichte Kette des ein Katalase-Epitop bindenden Antikörpers (HP25.6m/1B5) mindestens eine der folgenden CDRs, vorzugsweise die CDR3 und weiter bevorzugt alle drei folgenden CDRs auf:

CDR1: KASQDVGTSVA
CDR2: WTSTRHT
CDR3: QQYSSSPT

Weiterhin weist in einer besonders bevorzugten Ausführungsform die die leichte Kette des ein Katalase-Epitop bindenden Antikörpers (HP25.6m/1B5) kodierende DNA-Sequenz mindestens eine der folgenden CDRs, vorzugsweise die CDR3 und weiter bevorzugt alle drei folgenden CDRs auf:

CDR1: AAGGCCAGTCAGGATGTGGGTACTTCTGTTGCC
CDR2: TGGACATCCACCCGGCACACT
CDR3: CAGCAATATAGCAGCTCTCCCACG

In einer anderen bevorzugten Ausführungsform ist der β-Urease-spezifische Antikörper der von einem der bei der Deutschen Sammlung von Mikroorganismen und Zellkulturen (DSMZ) am 23. Juni 1998 nach den Vorschriften des Budapester Vertrages unter den Hinterlegungsnummern DSM ACC2360 oder DSM ACC2362 hinterlegten Hybridomen HP8m/4H5-D4-C9 oder HP9.1m/3C2-F8-E2 produzierte Antikörper. Der in den Figuren beschriebene β-Urease-spezifische Antikörper HP8m/1H5-G2-B4 wird durch einen Tochterklon des hinterlegten Hybridoms HP8m/4H5-D4-C9 produziert. Die beiden durch Mutter- und Tochterklon produzierten Antikörper werden durch identische DNA-Sequenzen kodiert und weisen dieselben Eigenschaften auf.

In einer anderen besonders bevorzugten Ausführungsform des erfindungsgemäßen Verfahrens weist die schwere Kette des ein Epitop der β-Urease bindenden Antikörpers mindestens eine der folgenden CDRs, vorzugsweise die CDR3 und weiter bevorzugt alle drei folgenden CDRs auf:

CDR1: GFTFSSHFMS
CDR2: SISSGGDSFYPDSLKG
CDR3: DYSWYALDY
oder:
CDR1: GYAFSTSWMN
CDR2: RIYPGDGDTNYNGKFKG
CDR3: EDAYYSNPYSLDY

In einer weiterhin besonders bevorzugten Ausführungsform weist die die schwere Kette des ein Epitop der β-Urease bindenden Antikörpers kodierende DNA-Sequenz mindestens eine der folgenden CDRs, vorzugsweise die CDR3 und weiter bevorzugt alle drei folgenden CDRs auf:

CDR1:	GG CTACGCATTC AGTACCTCCT GGATGAAC
CDR2:	CGGATTTATC CTGGAGATGG AGATACTAAC TACAATGGGA
	AGTTCAAGGG C
CDR3:	GAG GATGCCTATT ATAGTAACCC CTATAGTTTG GACTAC
oder:
CDR1:	GG ATTCACTTTC AGTAGCCATT TCATGTCT
CDR2:	TCCATTAGTA GTGGTGGTGA CAGTTTCTAT CCAGACAGTC
	TGAAGGGC
CDR3:	GACTAC TCTTGGTATG CTTTGGACTA C

In einer anderen besonders bevorzugten Ausführungsform des erfindungsgemäßen Verfahrens weist die leichte Kette des ein Epitop der β-Urease bindenden Antikörpers mindestens eine der folgenden CDRs, vorzugsweise die CDR3 und weiter bevorzugt alle drei folgenden CDRs auf:

CDR1: RASQSIGTRIH
CDR2: YGSESIS
CDR3: QQSNTWPLT
oder:
CDR1: HASQNINVWLS
CDR2: KASNLHT
CDR3: QQGRSYPLT

Des weiteren weist die die leichte Kette dieses Antikörpers kodierende DNA-Sequenz bevorzugt folgende CDRs auf:

CDR1:	A GGGCCAGTCA GAGCATTGGC ACAAGAATAC AC
CDR2:	TAT GGTTCTGAGT CTATCTCT
CDR3:	CAACAA AGTAATACCT GGCCGCTCAC G
oder:
CDR1:	C ATGCCAGTCA GAACATTAAT GTTTGGTTAA GC
CDR2:	AAG GCTTCCAACT TGCACACA
CDR3:	CAACAG GGTCGAAGTT ATCCTCTCAC G

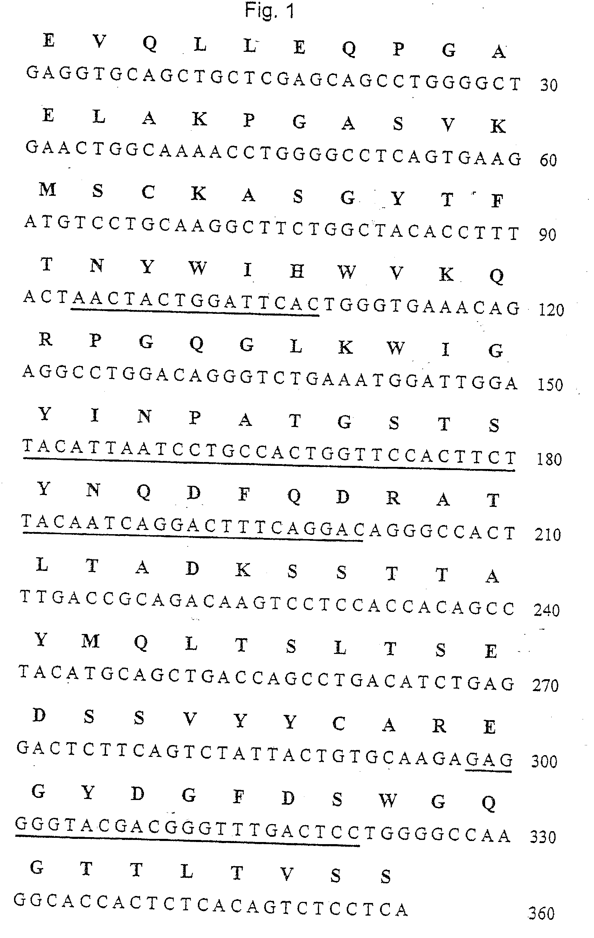

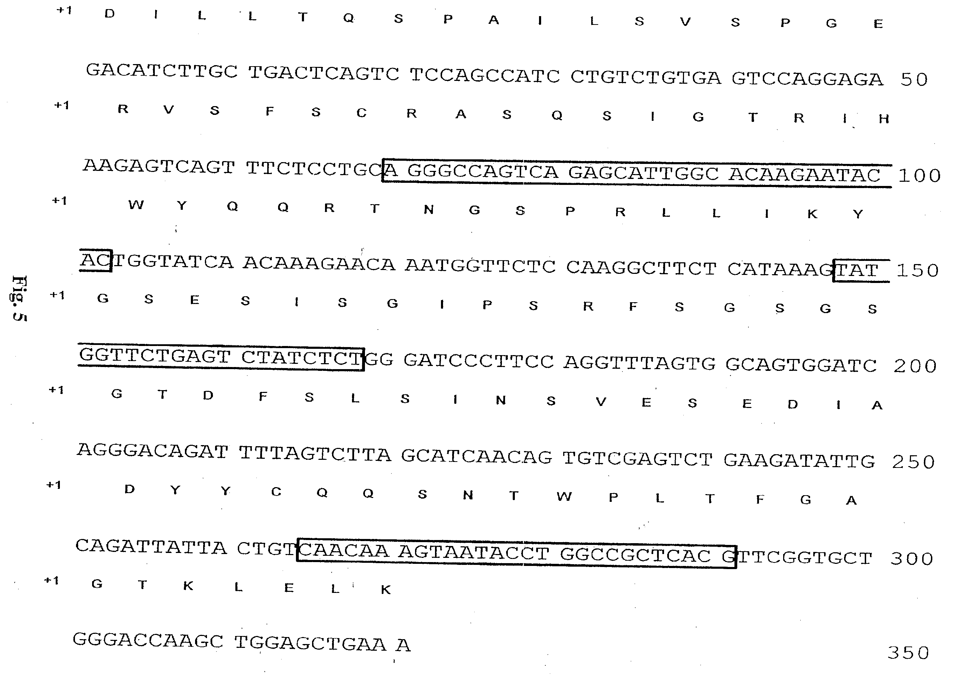

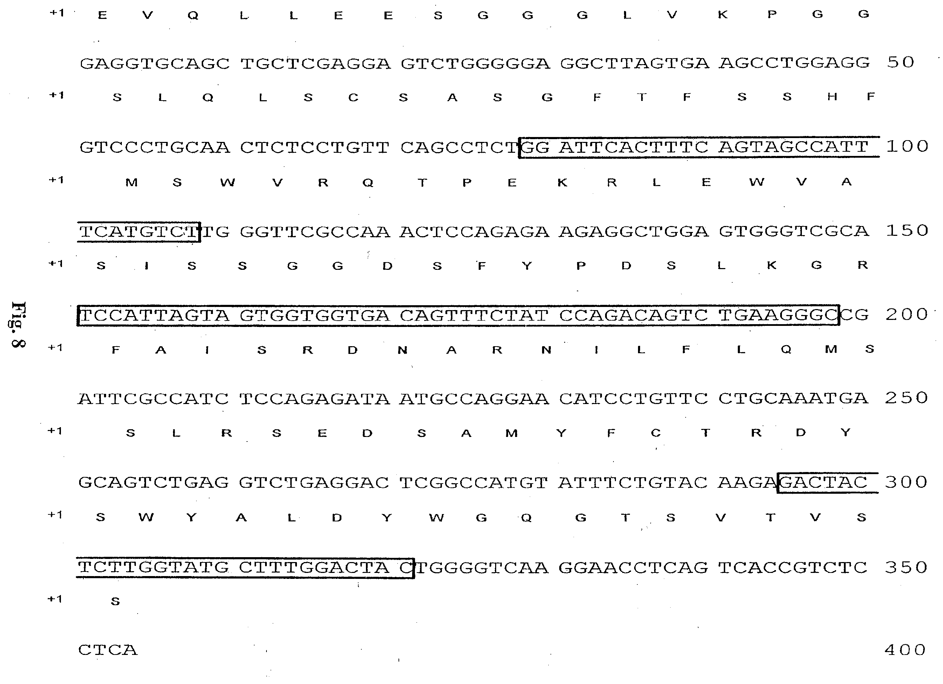

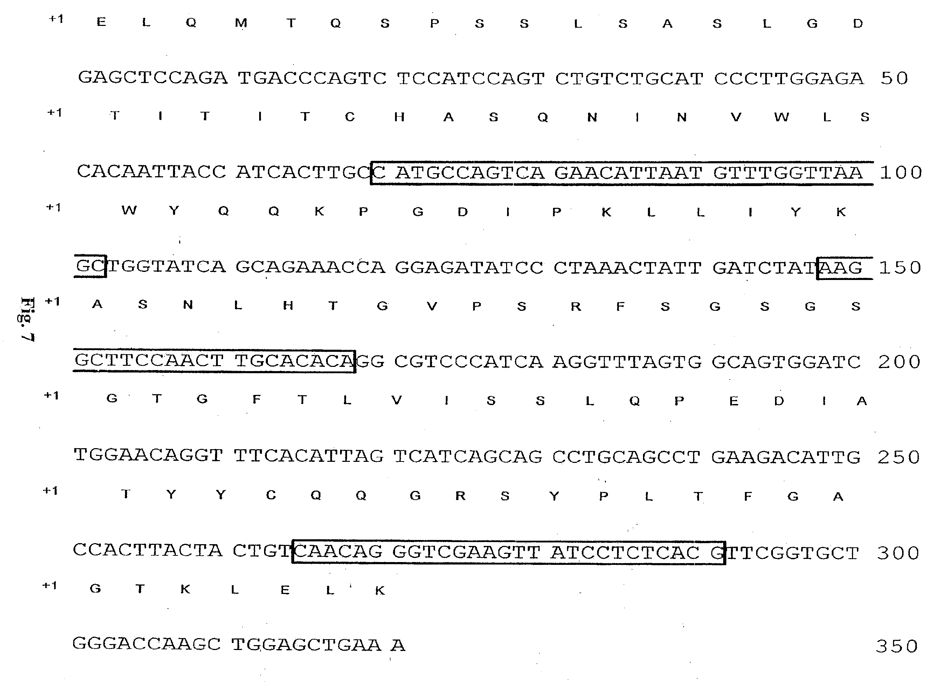

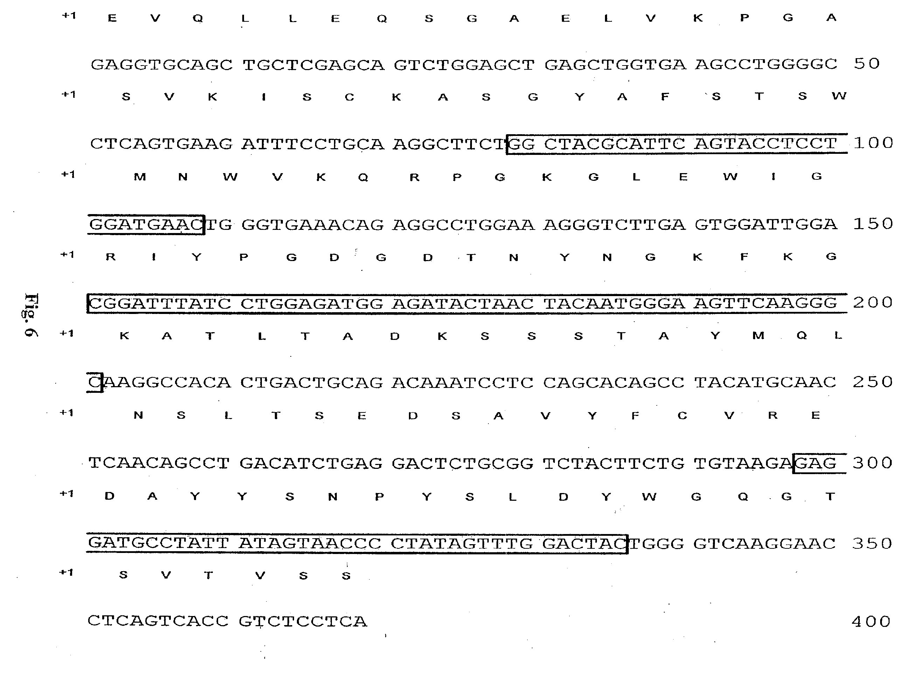

Besonders bevorzugt ist ferner, daß die schweren und leichten Ketten, die die vorstehend angegebenen CDRs aufweisen, gemeinsam in einem Antikörper, Fragment oder Derivat davon auftreten, der/das Katalase oder der/das β-Urease oder ein Fragment davon, vorzugsweise aus *H. pylori* spezifisch bindet. Die Erfindung umfaßt jedoch auch Ausführungsformen, in denen diese schweren oder leichten Ketten mit anderen leichten bzw. schweren Ketten kombiniert werden, wobei die Bindungseigenschaften im wesentlichen beibehalten oder verbessert werden können. Entsprechende Verfahren sind im Stand der Technik bekannt. Besonders bevorzugte Antikörper weisen in den variablen Regionen der leichten und schweren Ketten die in den Figuren 1 und 2, den Figuren 3 und 4, den Figuren 5 und 6 oder den Figuren 7 und 8 dargestellten Aminosäuresequenzen auf bzw. werden die Regionen von den dort dargestellten DNA-Sequenzen kodiert. Die CDRs können nach im Stand der Technik bekannten Verfahren in verschiedene FRs ("framework regions") integriert werden.

In einer bevorzugten Ausführungsform werden mit der Stuhlprobe vor der Inkubation mit den Antikörpern folgende Schritte durchgeführt: Die Stuhlprobe wird 1:3 bis 1:25, vorzugsweise etwa 1:10, besonders bevorzugt 1:5 in einem Resuspendierungspuffer resuspendiert und daraufhin auf einem Vortexmixer gemischt. Ein beispielhafter Resuspendierungspuffer enthält, 150 mM PBS, 0,1% SDS. In einer bevorzugten Ausführungsform besteht der Resuspendierungspuffer aus 150 mM PBS, 0,5% tierischem Serum und 0,1% Detergens. Hierbei kann das tierische Serum aus Rind, Maus, Ratte oder Schwein gewonnen und das Detergens aus der Gruppe ionischer (besonders bevorzugt Tween 20), nicht-ionischer (besonders bevorzugt SDS) oder zwitterionischer Detergentien (besonders bevorzugt Chaps) ausgewählt werden.

In einer anderen Ausführungsform kann der erfindungsgemäße Nachweis auch zur Detektion *von H. pylori* in Magengasen, Atemkondensat, Speichel, Zahnplaque, Schleimhautabstrichen, Biopsien, Vollblut oder Serum eingesetzt werden. Die Gewinnung von Atemgasen kann dadurch erfolgen, daß dem Patienten kalte CO₂haltige Getränke verabreicht werden, die eine Freisetzung von Magengasen in Form von "Rülpsen" verursachen. Diese Gase können in geeigneten Behältnissen aufgefangen oder entsprechend Atemkondensat in der dem Fachmann bekannter Weise gewonnen werden, beispielsweise mittels einer Vorrrichtung gemäß DE 19718925 oder einer Vorrichtung gemäß DE 19505504. Die so gewonnenen Kondensate können dann in flüssiger Form in den erfindungsgemäßen Test eingebracht werden, wobei sämtliche Schritte des erfindungsgemäßen Verfahrens wie vorstehend beschrieben durchgeführt werden mit der Ausnahme, daß anstelle einer Stuhlprobe eine wie hier beschriebene Probe eingesetzt wird. Zahnplaque und Schleimhautabstriche werden nach entsprechend den im Stand der Technik bekannten Methoden gewonnen und können wie Speichel, Vollblut und Serum in geeigneter Konzentration sowie Modifikation des Resuspendierungspuffers in den erfindungsgemäßen Nachweis eingesetzt werden.

In einer weiteren bevorzugten Ausführungsform erfolgt der Nachweis der Bildung des mindestens einen Antigen-Rezeptorkomplexes/Antigen-Rezeptor-Rezeptorgemischkomplexes in Schritt (b) mittels eines immunologischen Verfahrens.

In einer anderen bevorzugten Ausführungsform erfolgt der Nachweis der Bildung des mindestens einen Antigen-Rezeptorkomplexes/Antigen-Rezeptor/Rezeptorgemischkomplexes in Schritt (b) mittels ELISA, RIA, Western Blot oder eines immunchromatographischen Verfahren. Derartige Verfahren sind an sich im Stand der Technik bekannt; vgl. Harlow und Lane, a.a.O.

In einer besonders bevorzugten Ausführungsform des erfindungsgemäßen Verfahrens wird im immunologischen Verfahren, insbesondere im RIA oder im ELISA derselbe Rezeptor zur Bindung an die Festphase wie auch zum Nachweis des Epitops eingesetzt. Während der Fängerrezeptor in unmodifizierter Form an die Festphase, beispielsweise eine Mikrotiterplatte, gebunden werden kann, ist der zur Detektion eingesetzte Rezeptor gegebenenfalls mit einer Markierung versehen. Andererseits kann dieser Rezeptor ebenfalls nicht markiert sein und damit das Epitop des Mikroorganismus, vorzugsweise das bakterielle Epitop auch über einen dritten markierten Rezeptor nachgewiesen werden, wobei dieser Rezeptor vorzugsweise ein Antikörper, Fragment oder Derivat davon oder ein Aptamer ist, der/das ein speziesspezifischer oder Ig-klassenspezifischer Antikörper oder ein entsprechendes Aptamer sein kann. Markierungen von Antikörpern, beispielsweise mit radioaktiven oder fluoreszierenden Markern sind im Stand der Technik bekannt; vgl. Harlow und Lane a.a.O. Entsprechendes gilt für Aptamere. Die vorstehend beschriebenen Ausführungsform ist besonders günstig zum Nachweis der Katalase, die ggf. auch nach der Darmpassage noch als Tetramer vorliegt. Selbstverständlich können auch in dieser Ausführungsform Kombinationen von Antikörpern, Fragmenten, Derivaten und Aptameren eingesetzt werden, z.B. Kombinationen von Antikörpern etc., die an unterschiedliche Epitope desselben Antigens binden.

Unter einem Dreischritt-ELISA ist ein Verfahren zu verstehen, daß die Schritte Beschichtung der ELISA-Platte mit dem Fänger-Antikörper, Zugabe der Probe und Konjugatzugabe (wie beispielsweise markierter Detektorantikörper) sowie dazwischen geschaltete Waschschritte umfaßt. Der Einschritt-ELISA unterscheidet sich vom Dreischritt-ELISA dadurch, daß die Zugabe der Probe und die Zugabe des Konjugats auf eine mit dem Fänger-Antikörper vorbeschichtete ELISA-Platte in einem Schritt erfolgt.

In einer anderen bevorzugten Ausführungsform des erfindungsgemäßen Verfahrens ist der monoklonale Antikörper ein Maus-Antikörper.

Des weiteren sind in einer bevorzugten Ausführungsform die Rezeptoren an einen Träger fixiert.

Die Fixierung der Rezeptoren, vorzugsweise der Antikörper, Fragmente oder Derivate davon oder der Aptamere an einen Träger ist besonders vorteilhaft für die Durchführung von Routinechecks. Die Kombination Antikörper-Träger/Aptamer-Träger läßt sich ferner gut als Testbesteck oder in Kitform verpacken.

In einer besonders bevorzugten Ausführungsform ist das Trägermaterial ein poröses Trägermaterial.

In einer weiteren besonders bevorzugten Ausführungsform ist das Trägermaterial ein Teststreifen.

Zusätzlich besteht in einer bevorzugten Ausführungsform das Trägermaterial aus Zellulose oder einem Zellulosederivat.

Der Säuger, dessen Stuhl, Magengas, Atemkondensat etc. mit dem erfindungsgemäßen Verfahren untersucht werden kann, kann ein Tier, beispielsweise ein Haustier wie eine Katze oder ein Hund, ein Nutztier, z.B. ein Schwein oder ein sonstiges Tier wie eine Maus, ein Tiger, ein Gerbil oder ein Frettchen sein.

In einer bevorzugten Ausführungsform ist der Säuger ein Mensch.

In einer weiteren bevorzugten Ausführungsform ist das erfindungsgemäße Verfahren ein automatisiertes Verfahren. Ein automatisiertes Verfahren kann beispielsweise mittels eines Roboters durchgeführt werden, wobei der Roboter einen Teil der oder sämtliche Verfahrensschritte durchführt. Entsprechende Roboter sind im Stand der Technik bekannt.

Darüber hinaus betrifft die Erfindung einen monoklonaler Antikörper, ein Fragment oder Derivat davon, der/das eine V-Region aufweist, die eine Kombination der vorstehend dargestellten CDRs aufweist oder der von einem der vorstehend dargestellten Hybridomen produziert wird.

Bevorzugt ist dabei ein monoklonaler Antikörper, Fragment oder Derivat davon, der/das mindestens eine der in den Figuren 1 und 2, 3 und 4, 5 und 6 bzw. 7 und 8 dargestellten V-Regionen aufweist. Vorzugsweise weist dieser Antikörper zwei der in den Figuren 1 und 2, 3 und 4, 5 und 6 bzw. 7 und 8 dargestellten V-Regionen auf. Auch ist bevorzugt, daß diese V-Regionen von den in den Figuren 1 und 2, 3 und 4, 5 und 6 bzw. 7 und 8 dargestellten DNA-Sequenzen kodiert werden.

In einer besonders bevorzugten Ausführungsform der Erfindung ist der monoklonale Antikörper, das Fragment oder Derivat davon ein Maus-Antikörper oder ein Fragment oder Derivat davon oder ein chimärer, vorzugsweise ein humanisierter Antikörper oder ein Fragment oder Derivat davon. Das Derivat kann auch ein Fusionsprotein sein. Weiter bevorzugt ist, daß der Antikörper markiert ist, beispielsweise mit einem Kolloid, mit einer radioaktiven, fluoreszierenden, phosphoreszierenden oder chemiluminiszierenden Markierung.

Die Herstellung von chimärisierten humanisierten und humanen Antikörpern und der anderen Derivate ist im Stand der Technik wohlbekannt (z.B. Vaughan et al.,1998; Orlandi et al., 1989, Harlow und Lane, a.a.O.).

Die Erfindung betrifft auch ein Aptamer, das dasselbe Epitop wie der monoklonale Antikörper, das Fragment oder Derivat davon spezifisch bindet. Die Herstellung derartiger Aptamere kann mit im Stand der Technik bekannten Verfahren erfolgen.

Weiterhin betrifft die Erfindung ein Epitop, das von einem der vorstehend beschriebenen monoklonalen Antikörper, Fragment oder Derivat davon oder Aptamer spezifisch gebunden wird.

Darüber hinaus betrifft die Erfindung weitere Antikörper, Derivate oder Fragmente davon, die das erfindungsgemäße Epitop spezifisch binden. Diese Antikörper können beispielsweise monoklonale Antikörper sein, die unter Verwendung des Epitops als Hapten/Bestandteil eines Antigens nach üblichen Verfahren hergestellt werden können.

Die vorliegende Erfindung betrifft darüber hinaus eine diagnostische Zusammensetzung enthaltend mindestens einen Rezeptor, bevorzugt mindestens einen monoklonalen Antikörper, Fragmente oder Derivate davon oder Aptamere wie oben stehend definiert, gegebenenfalls fixiert an ein Trägermaterial.

Des weiteren betrifft die vorliegende Erfindung eine Testvorrichtung zum Nachweis mindestens eines wie oben stehend definierten Epitops, umfassend (a) mindestens einen Rezeptor, der vorzugsweise ein monoklonaler Antikörper, Fragmente oder Derivate davon oder ein Aptamer ist wie oben stehend definiert, fixiert an ein Trägermaterial; (b) eine Vorrichtung zur Aufbereitung und Analyse von Stuhlproben; und gegebenenfalls (c) ein Gemisch von Rezeptoren wie oben stehend definiert.

Die Erfindung hat ferner zum Gegenstand eine Testvorrichtung enthaltend (a) mindestens einen Rezeptor, vorzugsweise einen monoklonalen Antikörper, Fragmente oder Derivate davon oder ein Aptamer wie oben stehend definiert, wobei der Rezeptor konjugiert ist mit kolloidalem Gold, Latexpartikeln oder anderen farbgebenden Partikeln, deren Größe typischerweise im Bereich zwischen 5nm und 100nm, vorzugsweise zwischen 20nm und 60nm liegt (besonders bevorzugt ist eine Partikelgröße für Gold zwischen 40nm und 60nm und für Latex zwischen 200nm und 500nm); (b) eine Vorrichtung zur Aufbereitung und Analyse von Stuhlproben; und gegebenenfalls (c) ein Gemisch von Rezeptoren wie oben stehend definiert.

Darüber hinaus betrifft die vorliegende Erfindung einen Kit enthaltend (a) mindestens einen Rezeptor, der vorzugsweise ein monoklonaler Antikörper, Fragmente oder Derivate davon oder ein Aptamer wie oben stehend definiert ist, gegebenenfalls fixiert an ein Trägermaterial; gegebenenfalls ferner (b) eine Vorrichtung zur Aufbereitung und Analyse von Stuhlproben; und gegebenenfalls (c) ein Gemisch von Rezeptoren wie oben stehend definiert.

Alternativ zu den Vorrichtungen zur Aufbereitung und Analyse von Stuhlproben können die Zusammensetzungen, Testvorrichtungen und Kits auch Vorrichtungen zur Aufbereitung (sofern erforderlich) und Analyse von Magengasen, Atemkondensat, Speichel etc. aufweisen.

Die Erfindung betrifft auch eine Zusammensetzung enthaltend mindestens einen der vorstehend beschriebenen Rezeptoren, gegebenenfalls in Kombination mit einem pharmazeutisch verträglichen Träger und/oder Verdünnungsmittel. Die Zusammensetzung ist vorzugsweise ein Arzneimittel.

Beispiele für geeignete pharmazeutisch verträgliche Träger sind dem Fachmann bekannt und umfassen Phosphat-gepufferte Kochsalzlösungen, Wasser, Emulsionen wie z.B. Öl/Wasser-Emulsionen, verschiedene Arten von Detergensien, sterile Lösungen, etc. Arzneimittel, die solche Träger umfassen, können mittels bekannter konventioneller Methoden formuliert werden. Diese Arzneimittel können einem Individuum in einer geeigneten Dosis verabreicht werden, z.B. in einem Bereich von 1µg bis 100 mg pro Tag und Patient. Die Verabreichung kann auf verschiedenen Wegen erfolgen, z.B. intravenös, introperitoneal, subkutan, intramuskulär, lokal oder intradermal. Die Art der Dosierung wird vom behandelnden Arzt entsprechend den klinischen Faktoren bestimmt. Es ist dem Fachmann bekannt, daß die Art der Dosierung von verschiedenen Faktoren abhängig ist, wie z.B. der Größe, der Körperoberfläche, dem Alter, dem Geschlecht oder der allgemeinen Gesundheit des Patienten, aber auch von dem speziellen Mittel, welches verabreicht wird, der Dauer und Art der Verabreichung und von anderen Medikamenten, die möglicherweise parallel verabreicht werden.

Schließlich betrifft die Erfindung eine Packung enthaltend die erfindungsgemäße diagnostische Zusammensetzung, die erfindungsgemäße Testvorrichtung oder den erfindungsgemäßen Kit.

Die Bestandteile der erfindungsgemäßen diagnostischen Zusammensetzung, der erfindungsgemäßen Testvorrichtung und/oder des erfindungsgemäßen Kits können in Behältern, wie beispielsweise Fläschchen oder Röhrchen, gegebenenfalls in Puffern und/oder Lösungen verpackt sein. Unter Umständen können eine oder mehrere der Bestandteile in ein- und demselben Behälter verpackt sein. Die Figuren zeigen:
Fig. 1: Klonierte DNA-Sequenz, die für die V-Region der schweren Kette eines für Katalase spezifischen monoklonalen Antikörpers (HP 25.2m 2H10) kodiert. Die kodierte Aminosäuresequenz ist im Einbuchstaben-Code dargestellt. Die nach Kabat et al. bestimmten CDR-Regionen 1-3 sind durch Unterstreichung hervorgehoben.
Fig. 2: Klonierte DNA-Sequenz, die für die V-Region der leichten Kette eines für Katalase spezifischen monoklonalen Antikörpers (HP 25.2m 2H10) kodiert. Die kodierte Aminosäuresequenz ist im Einbuchstaben-Code dargestellt. Die nach Kabat et al. bestimmten CDR-Regionen 1-3 sind durch Unterstreichung hervorgehoben.
Fig. 3: Klonierte DNA-Sequenz, die für die V-Region der schweren Kette eines für Katalase spezifischen monoklonalen Antikörpers (HP25.6m/1B5) kodiert. Die kodierte Aminosäuresequenz ist ebenfalls dargestellt. Die nach Kabat et al. bestimmten CDR-Regionen 1-3 sind durch Unterstreichung hervorgehoben.
Fig. 4: Klonierte DNA-Sequenz, die für die V-Region der leichten Kette eines für Katalase spezifischen monoklonalen Antikörpers (HP25.6m/1B5) kodiert. Die kodierte Aminosäuresequenz ist ebenfalls dargestellt. Die nach Kabat et al. bestimmten CDR-Regionen 1-3 sind durch Unterstreichung hervorgehoben.
Fig. 5: DNA-Sequenz, die für eine leichte Kette eines ersten für Urease spezifischen monoklonalen Antikörpers (DMS ACC2360) kodiert. Die kodierte Aminosäuresequenz ist ebenfalls dargestellt. Die nach Kabat et al. bestimmten CDR-Regionen 1-3 sind durch Einrahmung hervorgehoben.
Fig. 6: DNA-Sequenz, die für eine schwere Kette eines ersten für Urease spezifischen monoklonalen Antikörpers (DMS ACC2360) kodiert. Die kodierte Aminosäuresequenz ist ebenfalls dargestellt. Die nach Kabat et al. bestimmten CDR-Regionen 1-3 sind durch Einrahmung hervorgehoben.
Fig. 7: DNA-Sequenz, die für eine leichte Kette eines zweiten für Urease spezifischen monoklonalen Antikörpers (DMS ACC2362) kodiert. Die kodierte Aminosäuresequenz ist ebenfalls dargestellt. Die nach Kabat et al. bestimmten CDR-Regionen 1-3 sind durch Einrahmung hervorgehoben.
Fig. 8: DNA-Sequenz, die für eine schweren Kette eines zweiten für Urease spezifischen monoklonalen (DMS ACC2362) kodiert. Die kodierte Aminosäuresequenz ist ebenfalls dargestellt. Die nach Kabat et al. bestimmten CDR-Regionen 1-3 sind durch Einrahmung hervorgehoben.
Fig. 9: Verlauf einer Eradikationsbehandlung eines *H. pylori*-positiven Patienten nach Einnahme von Omeprazol, Metronidazol und Clarithromycin.

Die Beispiele erläutern die Erfindung.

### Beispiel 1: Isolierung von H. pylori Antigenen

### 1.1 Kultivierung von H. pylori

*H. pylori* (Stamm NCTC 11637) wurde in Petri-Schalen auf Wilkins-chalkern Agar unter Zusatz von 10% Pferdeblut sowie Amphotericin B, Vancomycin und Cefsoludin (Sigma Chemicals) ausgestrichen und 1-2 Tage unter mikroaerophiler Atmosphäre (Anaerocult GasPAk, Merck) bei 37ºC inkubiert. Der Inhalt von 2 Schalen wurde in 350ml BHIB-Medium unter Antibiotika-Zusatz wie oben in einer 1I Flasche (Schott) suspendiert, das Medium für 4-8 min mit einem Gasgemisch aus 10% CO₂, 5% O₂, 85% N₂ begast und die Flasche verschlossen. Die Kultur wurde 2 Tage bei 37 ºC auf einem Rundschüttler geschüttelt. Der Inhalt der Flasche wurde anschließend steril in eine 10l Flasche überführt und mit 4,7l BHIB-Medium aufgefüllt. Die Flasche wurde dann weitere 2 Tage bei 37ºC auf einem Rundschüttler inkubiert. Das gesamte Volumen wurde daraufhin bei 5000g für 15min zentrifugiert, Überstand dekantiert und das Bakterienpellet gewogen. Zur Lagerung wurde das Pellet in einer physiologischen Kochsalzlösung unter Zusatz von 15% Glycerin im Verhältnis 2:1 (w / v) resuspendiert und bei -80ºC eingefroren. Um die Identität der kultivierten Bakterien zu überprüfen wurde eine mikroskopische Inspektion der Bakterien sowie Tests auf Urease-, Oxidase und Katalase-Aktivität durchgeführt.

### Beispiel 2: Präparation von H. pylori Antigenen

### Präparation von H. pylori-Lysat

*H. pylori* Bakterienpellet (Beispiel 1) wurde 1:10 mit PBS, pH 7,5 versetzt und auf Eis resuspendiert. Die Bakterienzellen wurden auf Eis mit der kleinen Sonde eines Ultraschallgerätes (Sonifier, Branson), bei 25 - 30 % Intensität 10 x 60s mit jeweils 60s Pause beschallt. Die aufgeschlossenen Bakterienzellen wurden 2 x 20 min, bei 4°C und 10.000 UpM zentrifugiert (Sorvall, SS34). Der Überstand wurde als Antigenpräparation für die Produktion von polyklonalen Antiseren verwendet.

### Präparation von H. pylori Katalase

Gefrorenes Bakterienpellet wurde im Verhältnis 1:2 (w / v) mit Aufschlußpuffer (20 mM Tris HCI pH 7.0, 1mM EDTA, 1 mM Phenyl-Methyl-Sulfonyl-Flourid (PMSF), 0,05% Natriumazid und 10% (v / v) Isobutanol) versetzt und bei Raumtemperatur (RT) auf einem Über-Kopf-Mischer bis zum vollständigen Auftauen und zusätzlich weitere ca. 15 min geschüttelt. Nach Zentrifugation bei 20.000g, 4°C für 20 min, wurde der Überstand abdekantiert und über ein 0,45 µm Filter filtriert.

Der klare Überstand wurde im Verhältnis 1:3 mit Puffer A (20 mM Tris HCI, pH 7.0, 1mM EDTA, 1 mM PMSF, 0,05% Natriumazid) verdünnt und auf eine mit Puffer A äquilibrierte SourceQ-Säule (16/10) (Pharmacia) überführt. Der Durchlauf von der SourceQ-Säule enthielt das Enzym Katalase und war frei von *H. pylori* Hauptantigenen wie Urease, HSP60 und Alkylhydroperoxid-Reduktase.

Zur Isolierung der Katalase wurde der Durchlauf von der SourceQ-Säule einer Molekularsieb-Chromatographie (Superdex 200) (16/60) unterzogen. Die Katalase wurde dabei zusammen mit einem andern ca. 150 kDa großen Protein (Neutrophil Activating Protein, NAP) in etwa gleichen Anteilen isoliert.

In höherer Reinheit wurde Katalase erhalten, wenn der Durchlauf von der SourceQ-Säule mit einer 2 M Natriumacetat-Lösung, pH 4.9 auf 40 mM Natriumacetat gebracht und auf eine SourceS-Säule (8/28) überführt wurde. Nach einem Waschschritt mit Puffer A zur Entfernung nicht gebundener Proteine wurde die Katalase mit Puffer B (40 mM Natriumacetat, 1 M NaCI, pH 4.9) unter Verwendung eines linearen NaCI-Gradienten (Puffer A plus 0% bis 100% Puffer B) eluiert. Katalase eluiert bei ca. 370 mM NaCI.

### Beispiel 3: Charakterisierung der Katalase:

Das gereinigte Protein wies unter reduzierenden Bedingungen im SDS-PAGE ein Molekulargewicht von ca. 58 kDa und eine Reinheit von ≥ 90% auf.

Zur Identifizierung des isolierten Proteins wurde eine Mikrosequenzierung durchgeführt. Das Protein wurde im SDS-PAGE Gel mit LysC Protease gespalten. Das extrahierte Proteingemisch wurde über RP-HPLC aufgetrennt. Die Sequenzanalyse des LysC Peptides ergab folgende Aminosäure-Sequenz:

**ERLHDTIGESLAHVTHK**

Diese Sequenz ist identisch mit dem entsprechenden LysC-Peptid aus *H. pylori* Katalase (Manos J. et al. (1998) Helicobacter 3 (1), 28-38; Genbank Accession No AAC16068.1)

### Beispiel 4: Herstellung polyklonaler und monoklonaler Antikörper (pAk; mAk)

### Herstellung polyklonaler Antiseren:

Polyklonale Antiseren gegen *H. pylori*-Lysat, *H. pylori*-Lysat mit abgereicherten Hauptantigenen wie beispielsweise Urease, HSP60 und Alkylhydroperoxid-Reduktase (siehe Beispiel 2: Isolierung und Reinigung), *H. pylori*-Lysat mit angereicherter Katalase (beispielsweise durch Zufügen von Katalase zum Lysat) sowie polyklonale Antiseren gegen gereinigte Katalase können durch Immunisierung eines ausgewählten Säugetieres (z.B. Maus, Kaninchen, Ziege, etc.) mit den entsprechenden Katalase-Epitope enthaltenden immunogenen Präparationen erhalten werden.

Die Antikörper können mittels Protein A Affinitäts-Chromatographie aus Seren gereinigt und als Fang-Antikörper im Sandwich-ELISA (siehe Beispiel 9) zur Beurteilung der Eignung monoklonaler Antikörper für die Antigen-Detektion in Patientenstuhl eingesetzt werden.

Polyklonale Kaninchen-Antiseren wurden von pab Productions (Herbertshausen) aus *H. pylori*-Lysat hergestellt. Aus diesen Antiseren wurden mittels Protein A Affinitäts-Chromatographie polyklonale Antikörper aufgereinigt und als Fang-Antikörper im Sandwich-ELISA (siehe Beispiel 9) zur Beurteilung der Eignung monklonaler Antikörper für die Antigen-Detektion in Patientenstuhl verwendet.

### Herstellung monoklonaler Antikörper:

Die Herstellung monoklonaler Antikörper erfolgt nach dem Fachmann bekannten Methoden (Harlow & Lane, 1988; Peters & Baumgarten, 1990).

### Immunisierung

Aus *H. pylori*-Lysat hergestellte Antigenpräparationen (siehe Beispiel 2), wurden zur Immunisierung von Mäusen (BALB/c x C57/Black, F1-Generation, 8-12 Wochen alt) verwendet. Als Grundimmunisierung wurden 50 µg Antigen 1:1 mit komplettem Freundschem Adjuvans (Difco), emulgiert und intraperitoneal injiziert (200 µl/Maus). Bei 4-monatlichen Auffrischungen erhielten die Mäuse jeweils 25 µg Antigen mit inkomplettem Freundschem Adjuvans. Aus retroorbital entnommenem Blut wurde Antiserum als Positivkontrolle im ELISA (siehe Fusionsscreening) gewonnen.

### Fusion

Zwei Tage nach der letzten Immunisierung wurden den Mäusen die Milzen entnommen und die Milzzellen mit den Myelomzellen P3x63Ag8.653 (ATCC CRL-1580; Kearney et al., 1979) im Verhältnis 5:1 mit Polyethylenglykol 4000 fusioniert. Die fusionierten Zellen wurden in HAT-Medium (Klonierungsmedium (= RPMI 1640 Medium, 20% FCS, 200 U/ml rhlL-6) mit Hypoxanthin-Aminopterin-Thymidin-Supplement (100x Konzentrat, Sigma)) suspendiert und mit einer Zelldichte von 2-6x10⁴ Zellen/Napf in 96-Napf-Mikrotiterplatten ausplattiert. Die Kultivierung der Hybridome erfolgte bei 37°C, 5% CO₂ und 95% relativer Luftfeuchtigkeit.

### Fusionsscreening mittels direktem ELISA

Das Screening der antikörperhaltigen Kulturüberstände aus bewachsenen Näpfen (ca. 10 Tage nach der Fusion) erfolgte im direkten ELISA auf 96-Napf Mikrotiterplatten (MaxiSorb, Nunc):

Die ELISA-Platten wurden mit 2 µg/ml Immunisierungsantigen in Carbonatpuffer, pH 9,6 beschichtet (100 µl/Napf, über Nacht, 5°C). Die Beschichtungslösung wurde abgesaugt und noch freie Bindungstellen mit 2% Magermilchpulver in PBS (w / v) geblockt (200 µl/Napf 1h, Raumtemperatur). Nach zweimaligem Waschen der Platte mit PBS pH 7,3 mit 0,025% Tween 20 (v / v) wurden die Kulturüberstände der Primärklone unverdünnt in die Näpfe pipettiert (100 µl/Napf) und die Platten 1-2 Stunden bei Raumtemperatur inkubiert. Als Positivkontrolle wurde Antiserum, als Negativkontrolle Medium verwendet. Nach erneutem Waschen erfolgte die Detektion der gebundenen Antikörper mit einem Peroxidase-markierten Sekundärantikörper (Kaninchen-anti-Maus Ig-POD (DAKO) in PBS mit 0,1% Rinderserumalbumin, 20min, Raumtemperatur). Die Peroxidase setzt im nachfolgenden Schritt das farblose Substrat Tetramethylbenzidin (TMB, Sigma) zu einem farbigen Komplex um. Nach viermaligem Waschen und Ausklopfen der Platte wurde Substratlösung (K-Blue, Neogen oder Zitronensäurepuffer, pH 4,5 mit TMB + H₂O₂) zugegeben und die Reaktion nach 10min durch Zugabe von 1 N Schwefelsäure abgestoppt. Kulturüberstände von Klonen, die antigenspezifische Antikörper produzieren, zeigten eine deutliche Färbung, gegenüber den farblosen negativen Kulturüberständen.

### Etablierung und Kultivierung der Hybridome

Positive Klone wurden zweimal nach dem Prinzip der Grenzverdünnung rekloniert, um Monoklone zu erhalten (Coller & Coller, 1983). Die erste Reklonierung erfolgte in Klonierungsmedium mit Hypoxanthin-Thymidin-Supplement (100x Konzentrat, Sigma), die zweite in Klonierungsmedium. Die Reklone wurden wiederum mittels direktem ELISA auf Antigenspezifität hin überprüft. Der Endklon wurde schließlich in Flachflaschen an Produktionsmedium (RPMI 1640 Medium mit 5% IgGreduziertem FCS) adaptiert. Die Zellen wurden kryokonserviert und Kulturüberstand für die Antikörperreinigung produziert.

### Beispiel 5: Charakterisierung der Antikörper aus Kulturüberstand

Aus einem Repertoire von 30 spezifischen (gegen das Immunisierungs-Antigen Antikörper-produzierend) Klonen wurden 10 anhand guter Reaktivität auf Stuhlproben *H. pylori*-infizierter Patienten im Sandwich-ELISA ausgewählt (sieheTabelle 2).

### Isotypbestimmung

Bei den etablierten Klonen wurde im Kulturüberstand eine Isotypbestimmung des monoklonalen Antikörpers mit dem Isotyping Kit IsoStrip (Roche Diagnostics) durchgeführt. Dies ergab 8 Klone des Typs IgG1 und einen Klon IgG2a (siehe Tabelle 3).

### Westernblot

Die Kulturüberstände wurden im Westernblot auf die Fähigkeit überprüft, das Immunisierungs-Antigen spezifisch zu erkennen. Pro Gel wurden 15 µg gereinigtes Antigen in reduzierendem Probenpuffer (Laemmli, 1970) gekocht und auf ein 12%iges SDS-Poplyacrylamid-Minigel (8,6cm x 7,7cm x 0,1cm, Biometra) aufgetragen. Nach elektrophoretischer Auftrennung bei 25-30 mA wurden die Proteine (Antigen) mittels Semidry-Blot-Verfahren auf einer Nitrozellulose-Membran immobilisiert.

Die Membran wurde mit 2% Magermilchpulver in PBS geblockt (30min, Raumtemperatur) und dreimal 5min. mit TBS/Tween 20 (0,2%) gewaschen. Für den folgenden Inkubationsschritt wurde die Membran in eine Accutran Cross-Blot-Screening-Einheit (Schleicher und Schüll) eingespannt, unter Verwendung einer Gitterplatte mit 34 Querkanälen. In jede der entstandenen Spuren wurden 250µl TBS/Tween 20 vorgelegt und je 250µl der zu testenden Hybridomakulturüberstände zugegeben. Die Inkubation erfolgte 2h bei Raumtemperatur unter Schütteln.

Nach dreimaligem Waschen TBS/Tween 20 wurde die Membran 1h mit dem PODkonjugierten Sekundärantikörper (Kaninchen-anti-Maus Ig-POD, DAKO) inkubiert.

Die Membran wurde dreimal gewaschen und der Immunkomplex durch Zugabe der 3,3-Diaminobenzidine-Substratlösung (DAB, Sigma) visualisiert. Die antikörperbindenden Proteinbanden wurden anschließend durch ein unlösliches Peroxidasesubstrat sichtbar gemacht.

6 Hybridomakulturüberstände zeigten eine der Katalase entsprechende Bande (58 kDa), 3 waren im Westernblot negativ, zeigen jedoch eine positive Reaktion mit nativem Antigen im ELISA. Wahrscheinlich erkennen sie ein Konformationsepitop. Die Ergebnisse sind in Tabelle 3 zusammengefaßt.

### Beispiel 6: Reinigung von mAk aus Hybridomakulturüberständen

Die Reinigung von mAk aus serumfreien Hybridomakulturüberständen erfolgt mittels einer modifizierten Protein-G Affinitätschromatographie (Pharmacia Biotech, 1994).

Die filtrierten (0,45µm) Kulturüberstände wurden direkt über eine Protein G Matrix geleitet. Der Proteinnachweis im Durchlauf bzw. Eluat erfolgte über die Messung der optische Dichte bei 280nm. Nach einem Waschschritt mit 150 mM PBS, pH 7,2 bis zum Erreichen des Detektor-Hintergrundwertes wurde mit 0,1M Glycin/HCl, pH 3,3 eluiert. Die Regeneration der Protein G Matrix erfolgte mit 0,1 M Glycin/HCl, pH 2,7.

### Beispiel 7: Herstellung von Konjugaten

### Kopplung von mAK an Peroxidase (POD) zur Verwendung im ELISA

Die Kopplung der mAK an Peroxidase (POD) erfolgte extern. Poly-POD-Konjugate wurden von der Firma MicroCoat (Bernried, Deutschland), HPR (horseraddish peroxidase)-Dextran-Konjugate von der Firma DAKO (Kopenhagen, Dänemark) bezogen.

### Kopplung von mAk an Biotin zur Verwendung im ELISA

Die monoklonalen Antikörper wurden im Anschluß an die Reinigung biotinyliert, um sie im ELISA als Detektionsantikörper einsetzen zu können. Die Kopplung monoklonaler Antikörper an Biotin und POD erfolgte nach bekannten Methoden (Harlow & Lane, 1988).

Die monoklonalen Antikörper wurden bei einer Konzentration von ca. 1-2 mg/ml konjugiert. Vor der Kopplung wurden die Antikörper durch Dialyse in 0,1 M Natrium-Acetat-Puffer, pH 8,3 bzw. 0,1 M Natriumhydrogencarbonat-Puffer, pH 8,3, umgepuffert. Zu je 1 mg Antikörper wurden 50 µg N-Hydroxysuccinimidobiotin (NHS-d-Biotin; Sigma) in DMSO pipettiert und vermischt. Die Mischung wurde 1 Stunde bei Raumtemperatur inkubiert. Danach wurden die biotinylierten Antikörper von ungekoppeltem NHS-d-Biotin durch extensive Dialyse gegen 0,15 M PBS, 0,05 % NaN₃, pH 7,5 befreit.

### Kopplung von mAk an kolloidales Gold zur Verwendung in immunologischen Schnelltests

Für eine Verwendung in immunologischen Schnelltests wurde der monoklonale Antikörper (mAk) an kolloidales Gold konjugiert. Dies erfolgte nach bekannten Standardmethoden (Frens, 1973; Geoghegan und Ackerman, 1977; Slot et al., 1985). Zur Herstellung von kolloidalem Gold wurden 200 ml einer 0,01 % Goldchlorid- (HAuCl₄)-Lösung bis zum Kochen erhitzt und durch Zugabe von 2 ml 1 % Natrium-citrat (Na₃C₆H₅O₇) unter weiterem Kochen reduziert.

Zur Kopplung von mAk an kolloidales Gold wurde eine zur Stabilisierung notwendige Menge des IgG mit der Goldlösung vermischt und 15 min bei Raumtemperatur inkubiert. Die optimale IgG-Konzentration und der geeignete pH-Wert für die Kopplung wurden für jeden mAk individuell bestimmt. Zur Stabilisierung des Gold-lgG-Konjugats wurden Polymere oder Protein, beispielsweise bovines Serumalbumin (BSA) in einer Konzentration von 1 % zum Kopplungsansatz zugegeben. Nicht mit IgG gekoppeltes Goldkolloid und freies IgG wurden anschließend durch Zentrifugieren vom Gold-IgG-Konjugat aus dem Kopplungsansatz entfernt. Zur Lagerung, die bevorzugt bei 4 °C erfolgt, wurde dem Lösungspuffer des Gold-lgG-Konjugats 0,05 % NaN₃ zugegeben.

### Beispiel 8: Charakterisierung der gereinigten monoklonalen Antikörper

### Charakterisierung von Antikörper-Antigen-Wechselwirkungen mittels Oberflächenplasmonresonanz-Spektroskopie (SPR-Spektroskopie)

Mit der SPR-Spektroskopie können die Affinitätskonstanten der monoklonalen Antikörper bestimmt werden. Dadurch lassen sich geeignete Antikörper für die Entwicklung von ELISA und Schnelltest finden.

### Durchführung der Oberflächenplasmonresonanz-Spektroskopie am Pharmacia BIAcore

Alle Schritte wurden auf einer Pharmacia BlAcore Processing Unit CA 186 nach Vorschrift des Herstellers durchgeführt (BlAcore Methods Manual).

Katalase wurde dabei über Aminkopplung auf der Dextranmatrix des BlAcore CM5 Sensorchips immobilisiert. Zur Aktivierung der Dextranmatrix wurden 45 µl einer 1:1-Mischung aus 0,05 M N-Hydroxysuccinimid (NHS) und 0,2 M 1-Ethy-3-(3-dimethylaminopropyl)carbodiimid (EDC)-Lösung bei einer Flußrate von 5 µl/min über den Sensorchip geleitet. Anschließend wurde Katalase (35 µl; 50 µg/ml in 10 mM Natrium-Acetat pH 5,0) an die Dextranmatrix gebunden. Verbleibende NHS-Ester wurden mit 1 M Ethanolamin (35 µl) deaktiviert. Nicht-kovalent an die Dextranmatrix gebundene Katalase wurde durch Regeneration des Sensorchips mit HCI (10 mM; 15 µl) entfernt.

Durch Zugabe der Katalase-spezifischen monoklonalen Antikörper wurden diese mit immobilisierter Katalase zur Reaktion gebracht und die Massenanlagerung am Detektor gemessen. Es wurden Antikörper-Lösungen unterschiedlicher Konzentration im Bereich zwischen 20 und 670 nM eingesetzt. Diese wurden mit einer Flußrate von jeweils 25 µl/min über die auf dem Sensorchip CM5 immobilsierte Katalase injiziert.

### Ergebnisse

Aus dem zeitlichen Verlauf des Resonanzsignals lassen sich die Werte für die Geschwindigkeitskonstanten der Adsorption (kₒₙ) und Desorption (k_{off}) des Antikörpers errechnen (BlAevaluation software 3.0). Es wurden 6 monoklonale Antikörper gegen Katalase bezüglich ihrer Affinitäten getestet:

**Tabelle 1:**

| Ergebnisse der Affinitätsbestimmung der Katalase-mAk | | | |
|---|---|---|---|
| **mAk** | **k**_{**on**} **[M**^{**-1**} **s**^{**-1**}**]** | **k**_{**off**} **[s**^{**-1**}**]** | **K**_{**D**} **[M]** |
| HP25.2m/2H10 | 1,44E+05 | 3,90E-05 | 2,71E-10 |
| HP25.6m/1G4 | 1,41E+05 | 2,52E-05 | 1,79E-10 |
| HP25.6m/1B5 | 5,67E+04 | 3,86E-05 | 6,81E-10 |
| HP25.6m/4E3 | 4,92E+04 | 5,96E-05 | 1,21E-09 |
| HP25.6m/1A5 | 3,91E+04 | 4,77E-05 | 1,22E-09 |
| HP25.6m/1H4 | 7,12E+04 | 4,12E-05 | 5,79E-10 |
| K_{D}= k_{off} : kₒₙ | | | |

### Auswahl von Antikörperpaaren für die Verwendung im ELISA am menschlichen Stuhl

Diejenigen Antikörper, welche die niedrigsten Nachweisgrenzen bei der Messung aus dem Kulturüberstand zeigten, wurden mittels Oberflächen-Plasmonenresonanz Epitop-Überlappungen bestimmt und Affinitätskonstanten gemessen. Die Kombinationen, die sich bei diesen Messungen vielversprechend zeigten (keine Epitop-Überlappung, hohe Geschwindigkeitskonstante für Adsorption, niedrige Geschwindigkeitskonstante der Desorption) wurden auf ihre Antigen-Nachweisgrenze im Sandwich-Stuhl-ELISA getestet.

### Beispiel 9: Screening von mAk Kulturüberständen auf Patientenproben (gemischtes polyklonales / monoklonales System)

Diejenigen monoklonalen Antikörper, die im Fusionsscreening mittels direktem ELISA (Beispiel 4) eine spezifische Antigenerkennung zeigten, wurden als Kulturüberstände im Sandwich-ELISA hinsichtlich ihrer Patienten-Erkennung und Antigen-Nachweisgrenze untersucht.

Als interne Entwicklungsproben standen Stuhlproben zur Verfügung, deren Infektionsstatus (Gruppe 0 und 4) mittels histologischer Untersuchung und / oder ¹³C Urea Atemtest erhoben wurde. Patienten der Gruppe 0 zeigen ein *H. pylori* negatives Ergebnis, Patienten der Gruppe 4 zeigten ein *H. pylori* positives Ergebnis im Referenztest.

Die Beschichtung der ELISA-Platten (Mikrotiterplatte MaxiSorb; Nunc) erfolgte über Nacht bei 5^{o}C mit 100 µl einer Lösung eines polyklonalen Kaninchen-anti-*H. pylori*-Antikörpers (pAk; ca. 20µg IgG/ml 0,1M Carbonat-Puffer, pH 9,5). Zur Blockade der noch freien Bindungsstellen wurden 200µl 150mM PBS, pH 7,2 mit 0,2% Fischgelatine (w / v) pro Napf pipettiert und 30min bei Raumtemperatur inkubiert. Danach wurde die ELISA-Platte 2x mit 250µl PBS unter Zusatz von 0,025% Tween 20 (Waschpuffer 1) gewaschen. Humanstuhl wurde im Verhältnis 1:10 (w / v) mit 150mM PBS unter Zugabe von 2% Magermilchpulver und 1mM EDTA suspendiert.

Zur Bestimmung der Antigen-Nachweisgrenze wurde eine *H. pylori* negative Stuhlsuspension mit 50 ng/ml Katalase (siehe Beispiel 3) versetzt und in 1:2 Schritten mit einer *H. pylori* negativen Stuhlsuspension verdünnt. Je 100µl der Stuhlsuspension wurden pro Napf für eine Stunde inkubiert (Doppelbestimmung bei Patientenproben). Die ELISA-Platte wurde ausgeschlagen, mit Waschpuffer 2 (PBS mit 0,2% Tween 20) abgespült und 4x mit Waschpuffer 2 gewaschen. Anschließend wurden 100µl Kulturüberstand von Hybridomen (1:5 in PBS verdünnt) zugegeben und für 60min bei Raumtemperatur inkubiert. Die Detektion der gebundenen Antikörper erfolgte durch Zugabe eines Peroxidase-konjugierten Sekundärantikörpers (Kaninchen-anti-Maus IgG-POD, DAKO). Die Peroxidase setzt im nächsten Schritt das zugegebene farblose POD-Substrat Tetramethylbenzidin (TMB, Sigma) in ein blaues Produkt um. Nach 5-10 Minuten, bevorzugt nach 10 Minuten wurde die Enzym-Reaktion durch Zugabe von 1N Schwefelsäure (100 µl/Napf) gestoppt. Die Stärke der Farbreaktion wurde im ELISA-Reader (MWG Spektral) gemessen. Die Messung erfolgt bei 450 nm gegen die Referenzwellenlänge 620 nm, bevorzugt 630 nm. Vor Zugabe des Detektionsantikörpers bzw. der Substratlösung wurde die ELISA-Platte jeweils 3x bzw. 4x mit Waschpuffer 1 gewaschen.

Als Nachweisgrenze wird ein Extinktionswert, der größer oder gleich dem Zweifachen des Null-Wertes (*H. pylori* negative Stuhlprobe ohne Antigen-Zumischung) ist, festgelegt.

Der monoklonale Antikörper HP25.2m/2H10 zeigte im Sandwich-ELISA unter der Verwendung eines polyklonalen Fänger-Antikörpers, der gegen *H. pylori* Lysat gerichtet ist, eine Sensitivität von 68% (von 25 positiven Proben wurden 17 richtig detektiert) und eine Spezifität von 82% (von 17 HP-negativen Proben wurden 3 Proben falsch positiv detektiert). Das Patienten-Erkennungsverhalten weiterer monoklonaler Antikörper (Kulturüberstände) ist aus Tabelle 3 zu entnehmen.

**Tabelle 2:**

| **HP25.2m/2H10: Sensitivität und Spezifität im Sandwich-ELISA (Fang-Antikörper pAK gegen *H. pylori*)** | | | |
|---|---|---|---|
| **Stuhlprobe** | **Infektions- Status des Patienten** | **Fänger-AK: pAK gegen HP Detektions-AK: HP25.2m/2H10 (Kulturüberstand) OD** _{**450-630**} | **Auswertung cut off: 0,1: OD**_{**450-630**}**= 0,1** |
| CX0010 | POSITIV | 0,25 | positiv |
| CX1014 | POSITIV | 0,75 | positiv |
| CX1029 | POSITIV | 0,18 | positiv |
| CX1038 | POSITIV | 0,09 | negativ |
| CX1052 | POSITIV | 0,11 | positiv |
| CX2008 | POSITIV | 0,63 | positiv |
| CX2009 | POSITIV | 0,32 | positiv |
| CX2016 | POSITIV | 0,07 | negativ |
| CX2019 | POSITIV | 0,59 | positiv |
| CX2029 | POSITIV | 0,52 | positiv |
| CX0213 | POSITIV | 0,04 | negativ |
| CX294-1 | POSITIV | 0,14 | positiv |
| CX3098 | POSITIV | 0,13 | positiv |
| CX3146 | POSITIV | 0,05 | negativ |
| CX3148 | POSITIV | 0,08 | negativ |
| CX3234 | POSITIV | 0,18 | positiv |
| CX4003 | POSITIV | 0,17 | positiv |
| CX4006 | POSITIV | 0,25 | positiv |
| CXT001 | POSITIV | 0,23 | positiv |
| CXT002 | POSITIV | 0,53 | positiv |
| CXT003 | POSITIV | 0,12 | positiv |
| CXT004 | POSITIV | 0,03 | negativ |
| CXT005 | POSITIV | 0,03 | negativ |
| CXT006 | POSITIV | 0,31 | positiv |
| CXT007 | POSITIV | 0,08 | negativ |
| CX1008 | NEGATIV | 0,29 | positiv |
| CX1031 | NEGATIV | 0,08 | negativ |
| CX1049 | NEGATIV | 0,7 | positiv |
| CX1051 | NEGATIV | 0,09 | negativ |
| CX0142 | NEGATIV | 0,03 | negativ |
| CX0185 | NEGATIV | 0,03 | negativ |
| CX0189 | NEGATIV | 0,08 | negativ |
| CX0193 | NEGATIV | 0,03 | negativ |
| CX2010 | NEGATIV | 0,08 | negativ |
| CX2018 | NEGATIV | 0,09 | negativ |
| CX0220 | NEGATIV | 0,03 | negativ |
| CX0231 | NEGATIV | 0,03 | negativ |
| CX0258 | NEGATIV | 0,02 | negativ |
| CX3008 | NEGATIV | 0,09 | positiv |
| CX3011 | NEGATIV | 0,08 | negativ |
| CX3033 | NEGATIV | 0,07 | negativ |
| CX3035 | NEGATIV | 0,09 | negativ |
| Abkürzungen: pAK: polyklonaler Antikörper; HP: *H. pylori* | | | |

**Tabelle 3:**

| Charakterisierung der monoklonalen Antikörper gegen Katalase | | | | | |
|---|---|---|---|---|---|
| **Fusion/Klon** | **Isotyp** | **WB (Ag)** | **NWG (ng/ml)** | **Stuhlproben, die korrekt erkannt wurden** | |
| | | | | pos. Proben | neg. Proben |
| HP25.2m/2H1 0 | IgG2a, κ | + | 1,5 | 17 von 25 | 14 von 17 |
| HP25.6m/1G4 | IgG1, κ | + | 1,5 | 4 von 5 | 2 von 2 |
| HP25.6m/1B5 | IgG1, κ | + | 3-6 | 3 von 5 | 2 von 2 |
| HP25.6m/1H4 | IgG1, κ | + | 3-6 | 2 von 5 | 2 von 2 |
| HP25.6m/4E3 | IgG1, κ | + | 6 | 2 von 5 | 2 von 2 |
| HP25.6m/1A5 | IgG1, κ | + | 6 | 2 von 5 | 2 von 2 |
| HP25.6m/5E4 | IgG1, κ | - | 1,5 | 1 von 5 | 2 von 2 |
| HP25.6m/4A12 | IgG1, κ | - | 1,5 | 1 von 5 | 2 von 2 |
| HP25.6m/5F4 | IgG1, κ | - | 1,5 | 1 von 5 | 2 von 2 |
| Abkürzungen: Ag Antigen; WB Westernblot; NWG Nachweisgrenze | | | | | |

### Ergebnisse

Tabelle 3 faßt die Ergebnisse der Isotyp-Bestimmung, der Westernblot-Analysen, der Nachweisgrenzen-Bestimmung und der Patienten-Erkennung für die monoklonalen Antikörper (mAK) gegen Katalase zusammen. Aus den Daten wird ersichtlich, daß eine gute Erkennung der nativen Katalase mittels mAK nicht mit einer guten Patienten-Erkennung korreliert.

Im gemischt polyklonalen / monoklonalen Sandwich-ELISA-System zeigte der mAK HP25.2m/2H10 eine Sensitivität von 68% und eine Spezifität von 82%. Eine Verbesserung von Sensitivität und Spezifität ist durch den Einsatz von gereinigten mAk (statt Kulturüberstand) in einem rein monoklonalen ELISA-System zu erwarten. Dabei können entweder ein monoklonaler Antikörper, der gegen das gleiche Epitop des Antigens gerichtet ist (siehe Beispiel 10), oder zwei verschiedene monoklonale Antikörper, die gegen verschiedene Epitope des gleichen Antigens gerichtet sind (siehe Beispiel 12), als Fänger- und Detektor-Antikörper eingesetzt werden

### Beispiel 10: Detektion von H. pylori im menschlichen Stuhl mittels ELISA (rein monoklonales System)

Für den Test standen Stuhlproben von Patienten aus zehn verschiedenen Kliniken oder gasteroenterologischen Praxen zur Verfügung, deren *H. pylori*-Status mittels ¹³C Urea Atemtest und / oder histologischer Untersuchungen von Magenbiopsien erhoben wurde. Die zu untersuchenden Stuhl-Proben wurden codiert, so daß dem Laborpersonal der Infektionsstatus nicht bekannt war.

### H. pylori-Stuhl-Sandwich-ELISA (Dreischritt-ELISA)

Die Beschichtung der ELISA-Platten (MaxiSorb; Nunc) erfolgte für 1h bei 37°C mit 100 µl einer mAK-Lösung (2.0 µg HP25.2m/2H10 /ml 0.1M Carbonatpuffer, pH 9,5). Zur Blockade der noch freien Bindungsstellen wurden 200µl 150mM PBS mit 0,2% Fischgelatine (w / v) pro Napf pipettiert und 30min bei Raumtemperatur inkubiert. Anschießend folgte ein zweimaliges Waschen mit 250µl Waschpuffer 1 (PBS mit 0,025% Tween). Humanstuhl wurde im Verhältnis 1:10 (w / v) mit 150mM PBS unter Zusatz von 2% Magermilchpulver und 1mM EDTA suspendiert. Zur Bestimmung der Antigen-Nachweisgrenze wurde gereinigte *H. pylori*-Katalase in bekannten Konzentrationen der Stuhlsuspension eines *H. pylori* negativen Patienten zugegeben. Die Stuhlprobensuspensionen wurden 5min bei 7000g abzentrifugiert. Je 100µl des Überstandes wurden pro Napf für 1h inkubiert. Die Platte wurde ausgeschlagen, abgespült und 4x mit Waschpuffer 2 (250µl PBS unter Zusatz von 0,2% Tween) gewaschen. Anschließend wurden 100µl einer Lösung Biotin-gekoppelten mAK (1 µg/ml HP25.2m/2H10-Bio in PBS; 0,1% BSA) zugegeben und für 60min bei Raumtemperatur inkubiert. Die Detektion der gebundenen Antigene erfolgt durch Zugabe eines Konjugats von Streptavidin mit POD (Dianova). Die POD setzt dann im nächsten Schritt das farblose Substrat TMB (Sigma) in ein blaues Produkt um. Nach 5 bis 10 Minuten, bevorzugt 10 Minuten wurde die Enzym-Reaktion durch Zugabe von 1N Schwefelsäure (100 µl/Napf) gestoppt. Die Stärke der Farbreaktion wurde im ELISA-Reader (MWG Spektral) gemessen. Die Messung erfolgt bei 455 nm gegen die Referenzwellenlänge 620nm, oder 630 nm.

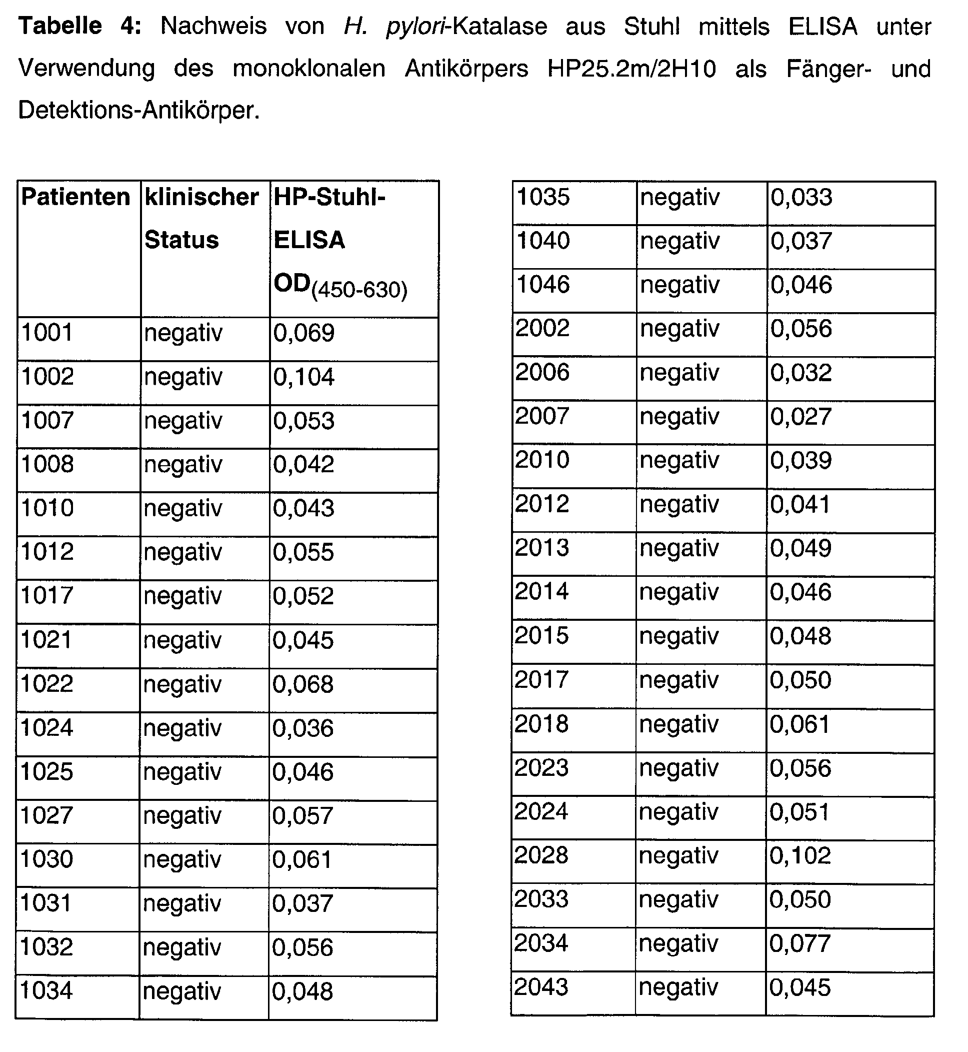

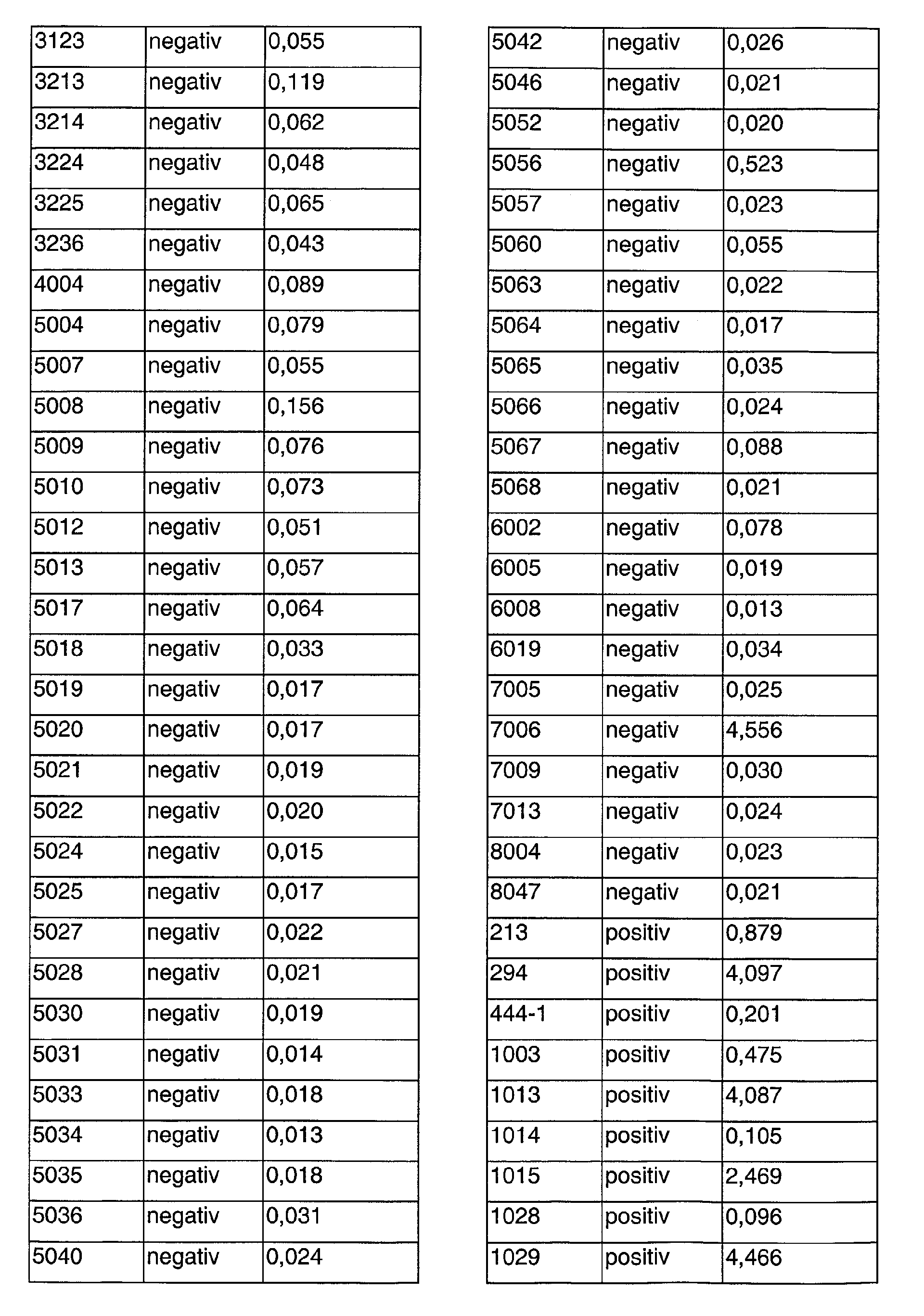

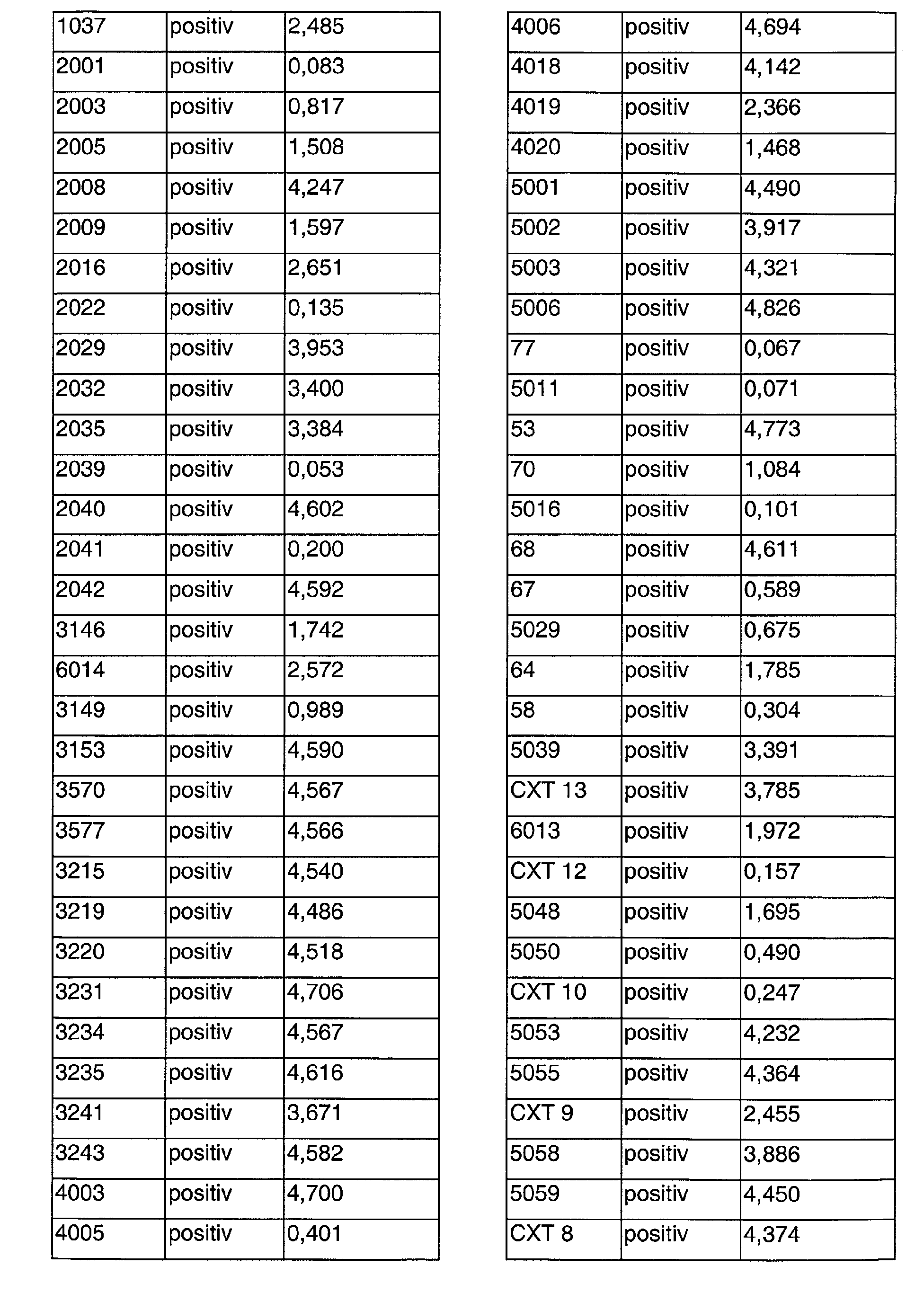

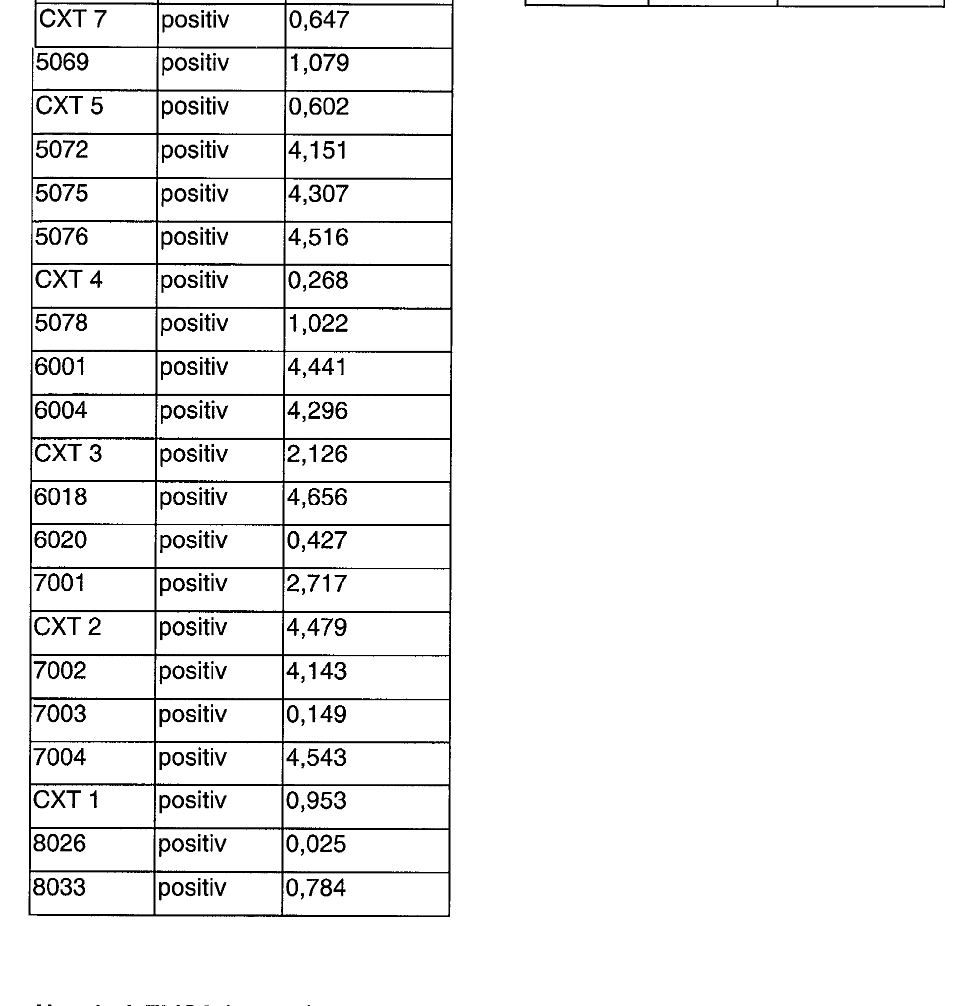

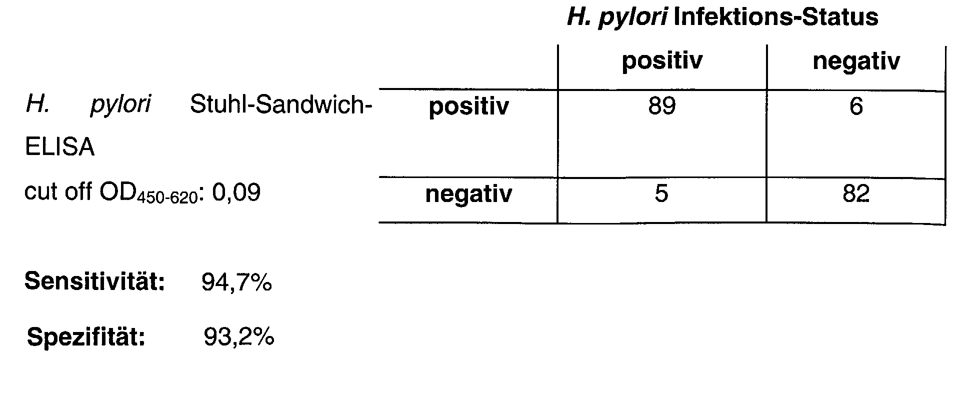

### Ergebnis:

Tabelle 4 zeigt die Ergebnisse der Untersuchung von *H. pylori* negativen und *H. pylori* positiven Stuhlproben mittels eines Stuhl Sandwich ELISA. Dabei werden monoklonale Antikörper, bevorzugt der monoklonale Antikörper HP25.2m/2H10 sowohl als Fänger- als auch als Detektions-Antikörper (POD-markiert) zur Detektion des *H. pylori*-Antigens Katalase aus der Stuhlprobe eingesetzt. Bei der Katalase handelt es sich um ein äußerst stabiles Antigen, welches den Verdauungstrakt weitgehend unverändert passiert und somit in der Stuhlprobe nachgewiesen werden kann. Die Untersuchung von 182 Stuhlproben im rein monoklonalen ELISA-System, das auf nur einem Katalase-spezifischen mAk aufgebaut ist, weist eine Sensitivität von 94,7% und eine Spezifität von 93,2% auf. Diese Sensitivität und Spezifität führt zu so hohen positiven und negativen prädiktiven Werten, daß eine Infektion mit H. pylori mit ausreichender Sicherheit schon alleine durch eine einfache, preisgünstige und nicht invasive Stuhluntersuchung festgestellt werden kann, um über eine Eradikationsbehandlung zu entscheiden. Eine Steigerung von Sensitivitat und Spezifität kann möglicherweise durch eine Kombination von verschiedenen mAks, die gegen unterschiedliche Epitope der Katalase gerichtet sind oder durch eine Kombination zweier Nachweis-Systeme für verschiedene Antigene (z.B. Katalase/Urease) erzielt werden.

Mit der Entwicklung eines Einschritt-ELISA-Tests wurde gegenüber dem Dreischritt-ELISA-Test, der nachfolgend (Beispiel 11 und 12) beschrieben wird, eine erhöhte Anwenderfreundlichkeit erreicht.

### Beispiel 11: Ermittlung von geeigneten Antikörperpaaren im Dreischritt-ELISA

Die Testdurchführung erfolgte gemäß Beispiel 10.

Zur Ermittlung von geeigneten Antikörperpaaren standen die monoklonalen Antikörper gegen Katalase (siehe Tabelle 3) gereinigt und z.T. biotinyliert (siehe Beispiel 7) zur Verfügung. Die Antikörper wurden zur Ermittlung der optimalen Einsatzkonzentration des Fang- und Detektor-Antikörpers zunächst gegeneinander austitriert. Dann wurden mit den so optimierten ELISA-Systemen Patientenstuhlproben getestet, sowie die Nachweisgrenzen von Katalase in humanem *H. pylori* negativem Stuhl (Nullstuhl) bestimmt (Tab. 5).

Geeignete mAK-Kombinationen bezüglich der Patienten-Erkennung und Antigen-Nachweisgrenze sind in Tabelle 5 aufgeführt.

**Tabelle 5:**

| Ergebnisse der Paarfindung der monoklonalen Antikörper gegen Katalase (Dreischritt-ELISA) | | | | | |
|---|---|---|---|---|---|
| | Fänger-Antikörper | | | | |
| biotinylierter Detektor-Antikörper | 25.2m/ 2H10 | 25.6m/ 1B5 | 25.6m/ 1G4 | 25.6m/ 1A5 | 25.6m/ 1H4 |
| 25.2m/2H10 | N: 0,03 G4: 7-8 G0: 2 | 0,1 7 2 | 0,03 8 2 | 0,1 7 2 | 0,03 8 2 |
| 25.6m/1B5 | N: 0,1 G4: 8 G0: 2 | 0,1 7 1 | 0,1 5 2 | 0,03 7 2 | 0,3 8 2 |
| 25.6m/1G4 | N: 0,3 G4: 6-8 G0: 1-2 | 0,1 7 2 | 0,1 8 4 | 0,01 8 2 | 0,1 8 2 |
| 25.6m/1A5 | N: 0,3 G4: 6-7 G0: 2 | 0,1 7 2 | 0,3 5 2 | 0,1 7 2 | 0,3 8 2 |
| 25.6m/1H4 | N: 0,1 G4: 8 G0: 3 | 0,3 | 0,1 4-7 2 | 0,3 7 2 | 0,1 8 2 |
| Patientenerkennung (Detektion von 8 kritisch positiven G4- und 4 klinisch negativen G0-Proben) | | | | | |
| N = Nachweisgrenzen [ng/ml] der Katalase in Nullstuhl | | | | | |
| kritisch positiv = Proben die sich im Nachweis als besonders problematisch erwiesen | | | | | |

### Ermittlung von geeigneten Antikörperpaaren im Einschritt-ELISA

Zur Ermittlung von geeigneten Antikörperpaaren standen monoklonale Antikörper gegen Katalase (siehe Tabelle 3) in gereinigtem Zustand und z.T. Peroxidasemarkiert (siehe Beispiel 7) zur Verfügung.

Die verschiedenen Kombinationen von Fang- und Detektions-Antikörpern (siehe Tabelle 6) wurden im Einschritt-ELISA an 27 *H. pylori*-positiven und 17 *H. pylori*-negativen Patientenproben getestet.

### Einschritt-Sandwich-ELISA:

Die Beschichtung der ELISA-Platte (MaxiSorb Lockwell; Nunc) erfolgte über Nacht bei 2-8 °C mit 100 µl einer mAK-Lösung (2.0 µg Fang-Antikörper / ml Carbonatpuffer, 0,1 M, pH 9,5). Die so beschichteten ELISA-Platten wurden 2x mit PBS gewaschen und zum Blockieren der freien Bindungsstellen mit 200 µl Blockierungspuffer (0,3% BSA; 20% Sorbitol in PBS) je Vertiefung versetzt und bei 2-8 °C über Nacht inkubiert. Diese ELISA-Platten wurden abgesaugt, über Nacht bei 28°C im Umluft-Trockenschrank getrocknet und anschließend mit Trockenmittel-Beutel bei 2-8 °C gelagert.

Patientenstuhl wurde 1:5 (0,1g Stuhlprobe + 500µl Probenpuffer) in Probenpuffer (150mM PBS + 0,5% Tierserum + 1mM EDTA + 0,1% Detergens) ca. 30 sec suspendiert (Vortex) und anschließend 5 min bei 3000 g zentrifugiert. Pro Napf wurden 50 µl des Überstands auf die Platte aufgetragen.

Anschließend wurden 50 µl des in Probenpuffer verdünnten POD-markierten Antikörpers (0,5 nM AK-Dextran-POD bzw. 0,2 µg/ml HP25.2m/2H10-POD-P) direkt in die Stuhlsuspension gegeben. Die Platten wurden 1 Stunde bei Raumtemperatur auf dem Schüttler inkubiert.

Nach fünfmaligen Waschen mit Waschpuffer (75 mM PBS, 0,25% Tween) wurde das Peroxidase-Substrat TMB (Tetramethylbenzidin) Einkomponentensubstrat (Neogen) zugegeben (100 µl/Napf). Nach 10 Minuten wurde die Enzymreaktion durch Zugabe von 1N Salzsäure (100 µl/Napf) gestoppt. Die anschließende Messung der Farbintensität erfolgte bei 450 nm gegen die Referenzwellenlänge 630 nm.

**Tabelle 6:**

| Ergebnisse der Paarfindung der monoklonalen Antikörper gegen Katalase (Einschritt-ELISA) | | | | | | | | | |
|---|---|---|---|---|---|---|---|---|---|
| | Fänger-Antikörper | | | | | | | | |
| POD markierter Detektor-Antikörper | 25.2m/ 2H10 | 25.6m/ 1B5 | 25.6m/ 1A5 | 25.6m/ 4A12 | 25.6m/ 1G4 | 25.6m/ 1H4 | 25.6m/ 3D6 | 25.6m/ 2E12 | 25.6m/ 5E4 |
| 25.2m/2H10 | - | 24 | 24 | 22 | 23 | 24 | 19 | 24 | 22 |
| | | 14 | 11 | 13 | 14 | 12 | 14 | 12 | 15 |
| 25.6m/1B5 | G4: 23 | - | 23 | 20 | 23 | 23 | 18 | 23 | 22 |
| | G0: 12 | | 13 | 14 | 12 | 12 | 12 | 10 | 12 |
| 25.6m/1H4 | G4: 21 | 24 | 24 | - | 24 | - | 20 | 25 | 24 |
| | G0: 9 | 13 | 14 | | 11 | | 15 | 12 | 20 |
| 25.6m/4A12 | G4: 20 | 20 | 20 | - | 20 | 25 | 19 | 20 | 20 |
| | G0: 13 | 12 | 17 | | 13 | 3 | 13 | 13 | 15 |
| 25.6m/3D6 | G4: 17 | 24 | 24 | 21 | 23 | 23 | - | 22 | 22 |
| | G0: 15 | 9 | 12 | 13 | 8 | 13 | | 9 | 14 |
| Patientenerkennung (Detektion von 27 kritisch positiven G4- und 17 klinisch negativen G0-Proben); Cut off OD ₄₅₀₋₆₃₀ₙₘ: 0,15 | | | | | | | | | |

Die Antikörperkombination HP25.6m/1B5 (Fang-Antikörper) und HP25.2m/2H10-POD (Detektions-Antikörper) erwies sich aufgrund der guten Patientenerkennung (korrekte Detektion von 24 aus 27 *H. pylori* positiven und 14 aus 17 *H. pylori* negativen Proben) sowie der hohen Signalstärke zum Nachweis von *H. pylori* Antigenen (Katalase) in Humanstuhl am vorteilhaftesten.

### Beispiel 12: Detektion von H. pylori im menschlichen Stuhl mittels Einschritt-ELISA

Für den Test standen Stuhlproben von Patienten aus zehn verschiedenen Kliniken oder gasteroenterologischen Praxen zur Verfügung, deren *H. pylori* negativ oder *H. pylori* positiv Status) mittels ¹³C Urea Atemtest und / oder histologischer Untersuchungen von Magenbiopsien erhoben wurde.

### H. pylori-Stuhl-Sandwich-ELISA (Einschritt-Test)

Die Beschichtung der ELISA-Platte (MaxiSorb Lockwell; Nunc) erfolgte über Nacht bei 2-8 °C mit 100 µl einer mAK-Lösung (2.0 µg HP25.6m/1B5 / ml Carbonatpuffer, 0,1 M, pH 9,5). Die so beschichteten ELISA-Platten wurden 2x mit PBS gewaschen. Zum Blockieren der freien Bindungsstellen wurden 200 µl Blockierungspuffer (0,3% BSA; 20% Sorbitol in PBS) je Napf zugegeben und bei 2-8 °C über Nacht inkubiert. Diese Platten wurden abgesaugt, über Nacht bei 28°C im Umluft-Trockenschrank getrocknet und anschließend mit Trockenmittel-Beutel bei 2-8 °C gelagert.

Patientenstuhl wurde 1:5 (0,1g Stuhlprobe + 500µl Probenpuffer) in Probenpuffer (150mM PBS + 0,5% Tierserum + 1mM EDTA + 0,1% Detergens) ca. 30 sec suspendiert (Vortex) und anschließend 5 min bei 3000 g zentrifugiert. Pro Napf wurden 50 µl des Überstands (Doppel- bis Dreifachbestimmung) auf die Platte aufgetragen. Anschließend wurden 50 µl des in Probenpuffer-verdünnten POD-markierten Antikörpers HP25.2m/2H10-Dextran-POD direkt in die Stuhlsuspension gegeben. Die Platten wurden 1 Stunde bei Raumtemperatur auf dem Schüttler inkubiert.

Nach fünfmaligen Waschen mit Waschpuffer (75 mM PBS, 0,25% Tween) wurde Peroxidase-Substrat TMB (Tetramethylbenzidin) Einkomponentensubstrat (Neogen) zugegeben (100 µl/Napf). Nach 10 Minuten wurde die Enzymreaktion durch Zugabe von 1N Salzsäure (100 µl/Napf) gestoppt. Die anschließende Messung der Farbintensität erfolgte bei 450 nm gegen die ReferenzVertiefungenlänge 630 nm.

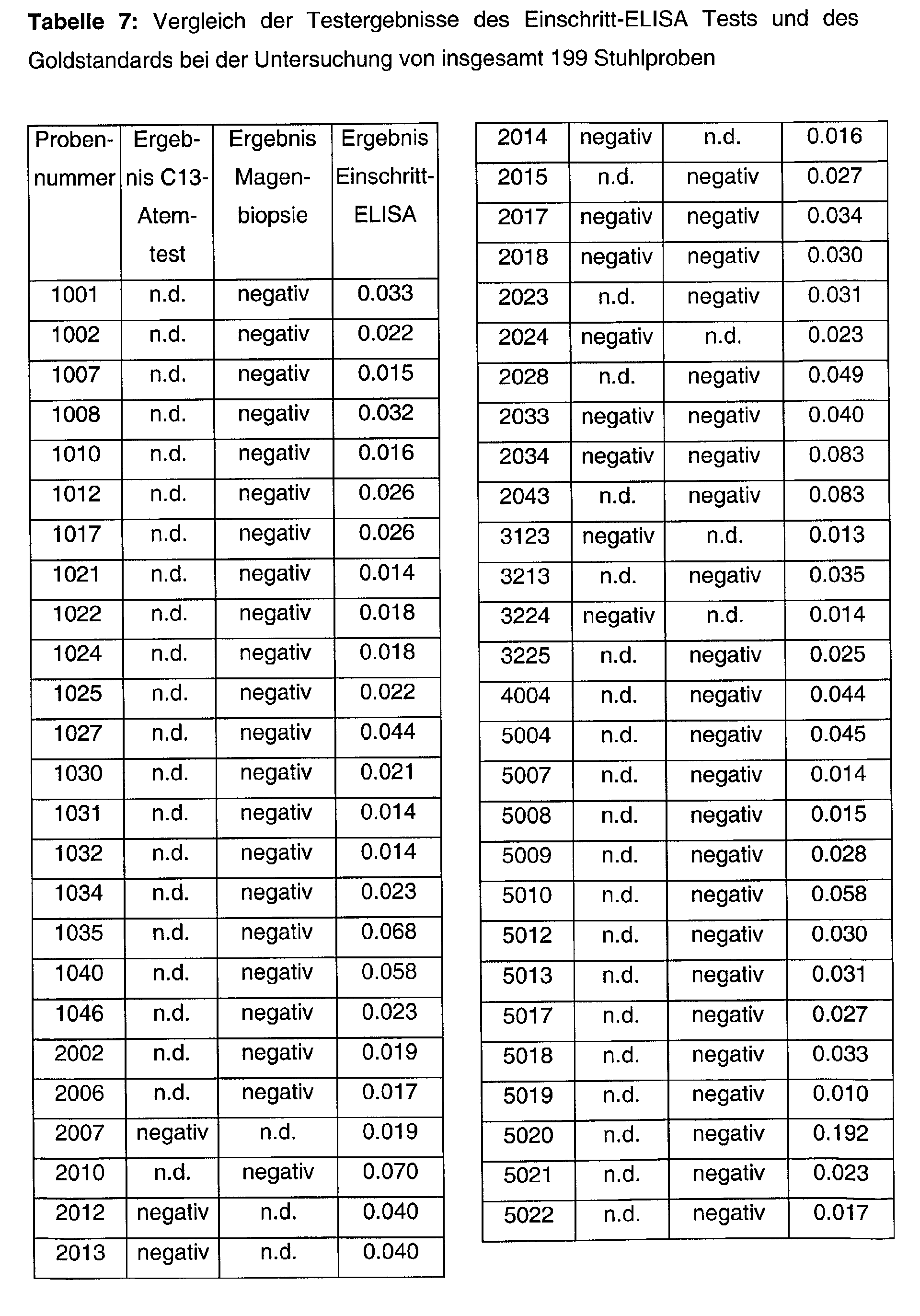

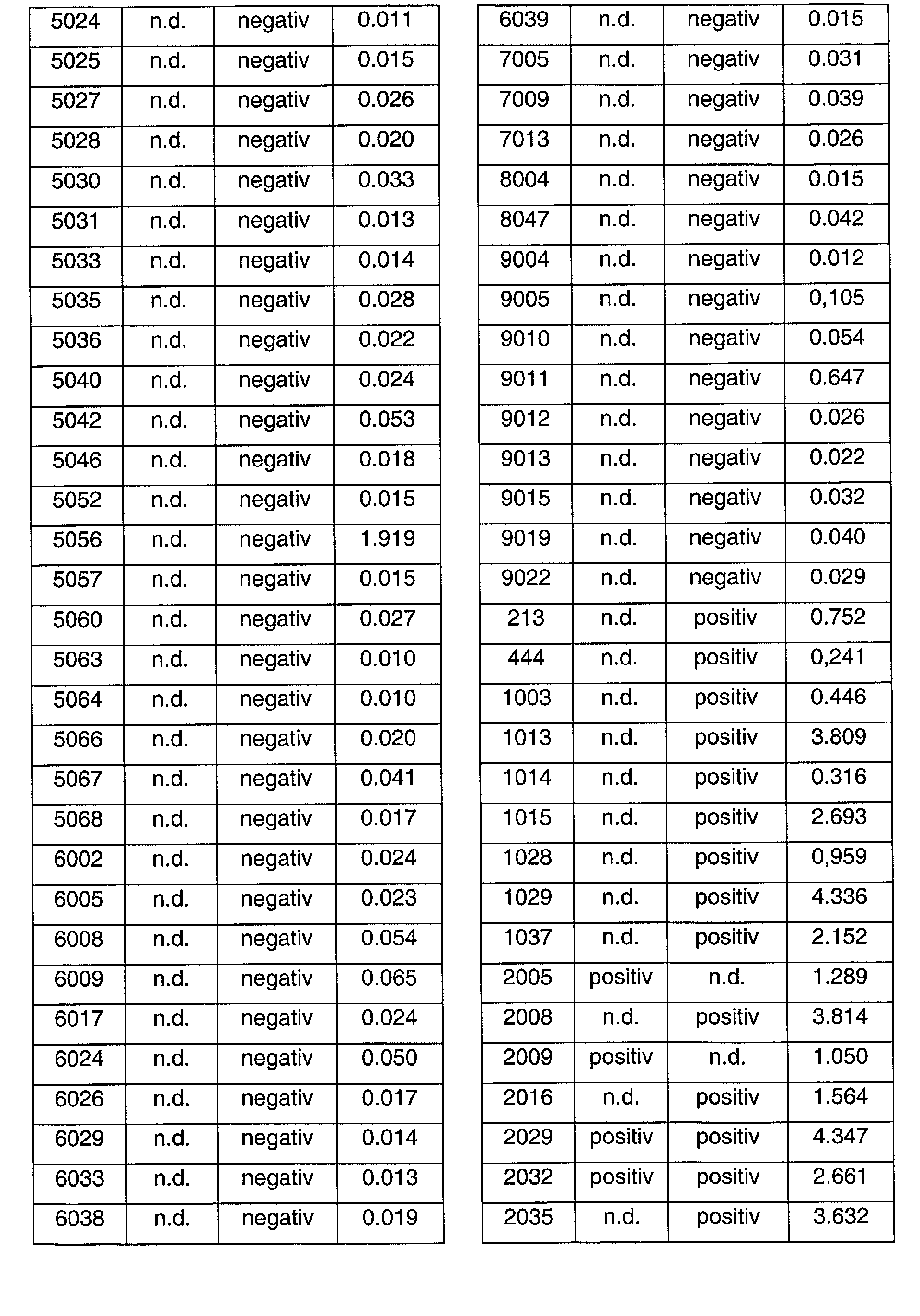

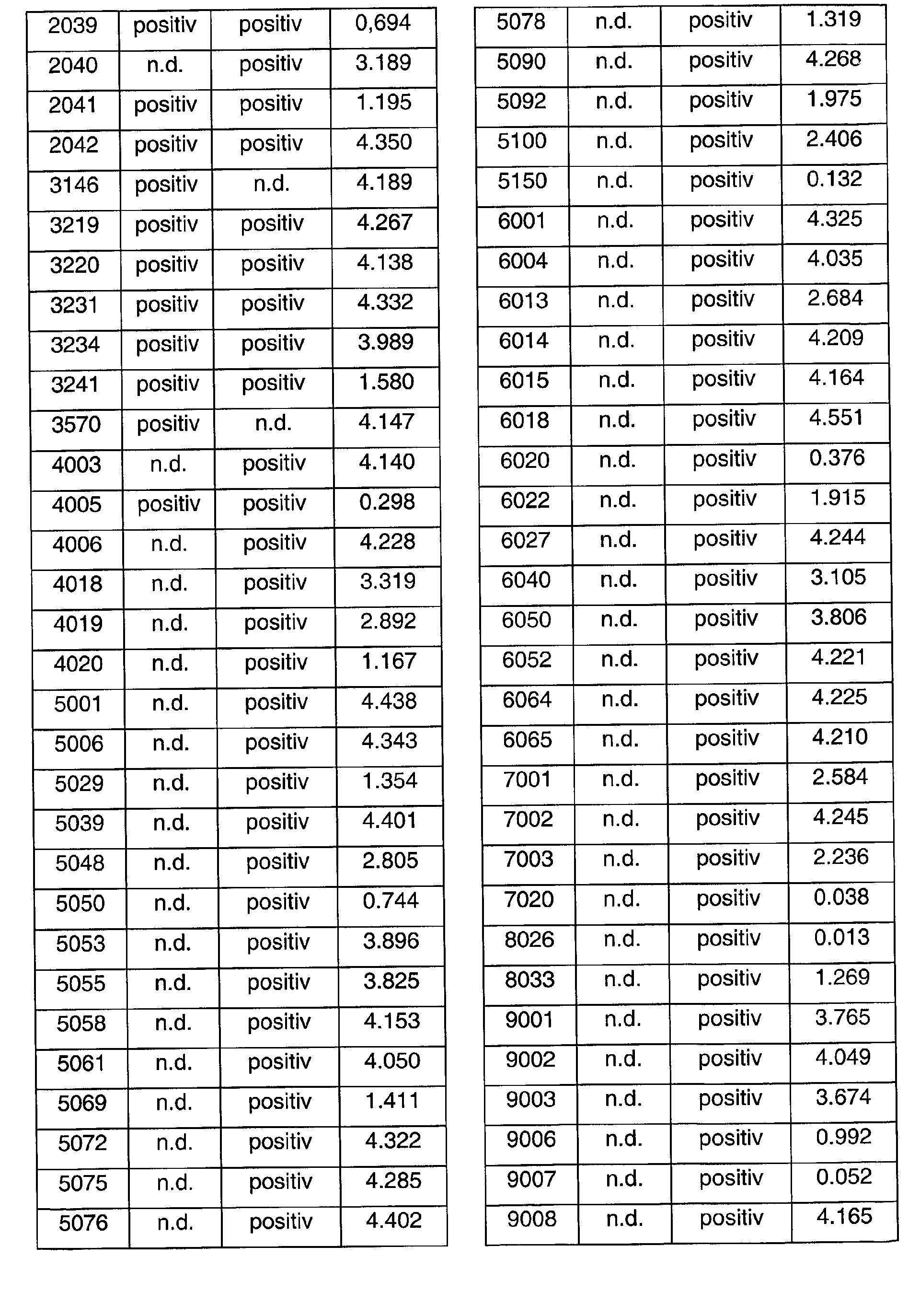

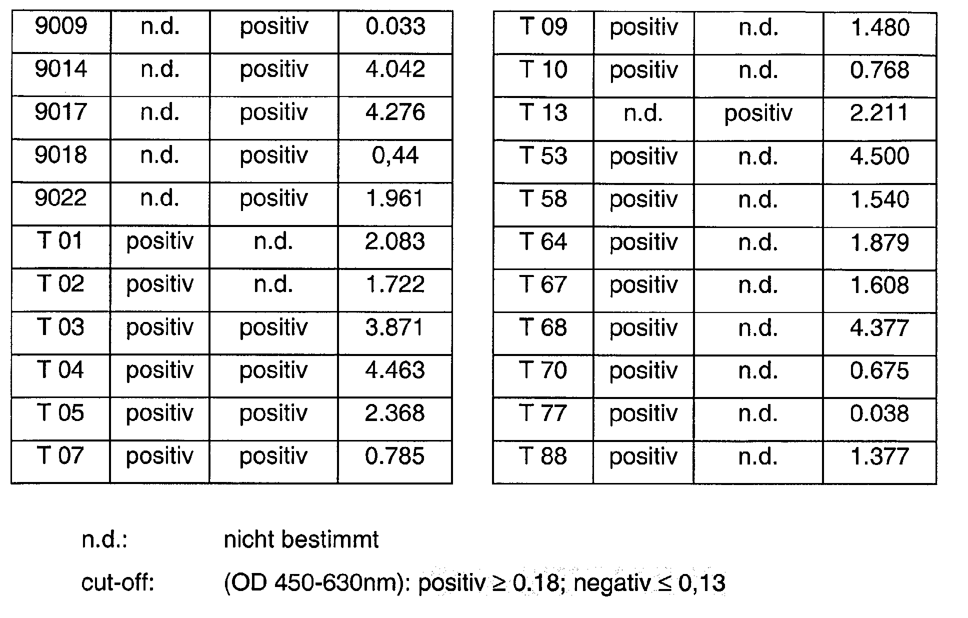

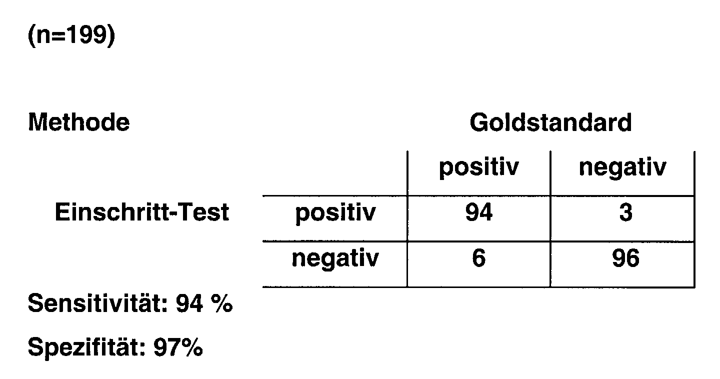

### Ergebnis:

Tabelle 7 zeigt die Ergebnisse der Untersuchung von *H. pylori* negativen und *H. pylori* positiven Stuhlproben mittels eines Einschritt-Stuhl Sandwich ELISA. Dabei wurden die monoklonalen Antikörper (HP25.6m/1B5; HP25.2m/2H10) für die Detektion des *H. pylori*-Antigens Katalase aus der Stuhlprobe eingesetzt. Die Untersuchung von 199 Stuhlproben im rein monoklonalen ELISA-System, das auf oben benannten Katalase-spezifischen mAK aufgebaut ist, weist eine Sensitivität von 94 % und eine Spezifität von 97% auf.

### Beispiel 13: Detektion von H. pylori im menschlichen Stuhl mittels optimiertem Einschritt-ELISA

Für den Test standen 357 Stuhlproben von Patienten aus zehn verschiedenen Kliniken oder gasteroenterologischen Praxen zur Verfügung, deren *H. pylori* Status mittels histologischer Untersuchung von Magenbiopsien erhoben wurde. Die Testdurchführung erfolgte verblindet (die Testproben wurden so codiert, daß dem Laborpersonal der Infektionsstatus der Proben nicht bekannt war) in einem externen GLP-Labor.

### Optimierter Einschritt-Sandwich-ELISA:

Die Beschichtung der ELISA-Platte (MaxiSorb Lockwell; Nunc) erfolgte über Nacht bei 2-8 °C mit 100 µl einer mAK-Lösung (2.0 µg HP25.6m/1B5 / ml Carbonatpuffer, 0,1 M, pH 9,5). Die so beschichteten ELISA-Platten wurden 2x mit PBS gewaschen, zum Blockieren der freien Bindungsstellen mit 200 µl Blockierungspuffer (0,3% BSA; 5% Sorbitol in PBS) je Napf versetzt und bei 2-8 °C über Nacht inkubiert. Diese Platten wurden abgesaugt, über Nacht bei 28°C im Umluft-Trockenschrank getrocknet und anschließend mit Trockenmittel-Beutel bei 2-8 °C gelagert.

Patientenstuhl wurde 1:5 (0,1g Stuhlprobe + 500µl Probenpuffer) in Probenpuffer (150mM PBS + 0,5% Tierserum + 1mM EDTA + 0,1 % Detergens) ca. 30 sec suspendiert (Vortex) und anschließend 5 min bei 3000 g zentrifugiert. Pro Napf wurden 50 µl des Überstands auf die Platte aufgetragen. Anschließend wurden 50 µl des in Probenpuffer verdünnten Peroxidase-markierten Antikörpers (200 femtomol / ml HP25.2m/2H10-Dextran-POD-markiert) direkt zur Stuhlsuspension zugegeben. Die Platten wurden 1 Stunde bei Raumtemperatur auf dem Schüttler inkubiert. Nach fünfmaligen Waschen mit Waschpuffer (75 mM PBS, 0,25% Tween) wurde das Peroxidase-Substrat TMB (Tetramethylbenzidin) Einkomponentensubstrat (Seramun) zugegeben (100 µl/Napf). Nach 10 Minuten wurde die Enzymreaktion durch Zugabe von 1M Schwefelsäure (100 µl/Napf) gestoppt. Die anschließende Messung der Farbintensität erfolgte bei 450 nm gegen die Referenzwellenlänge 630 nm.

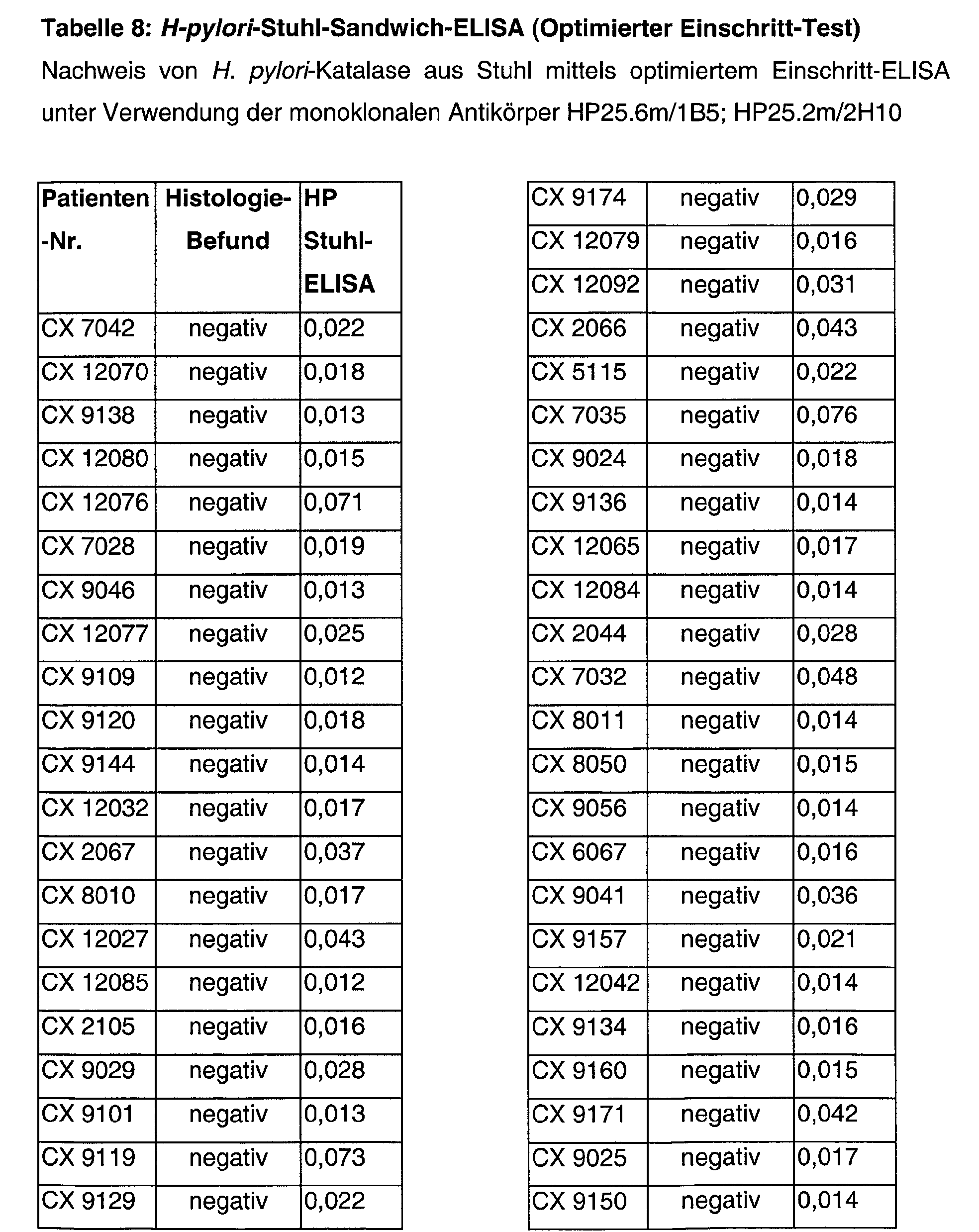

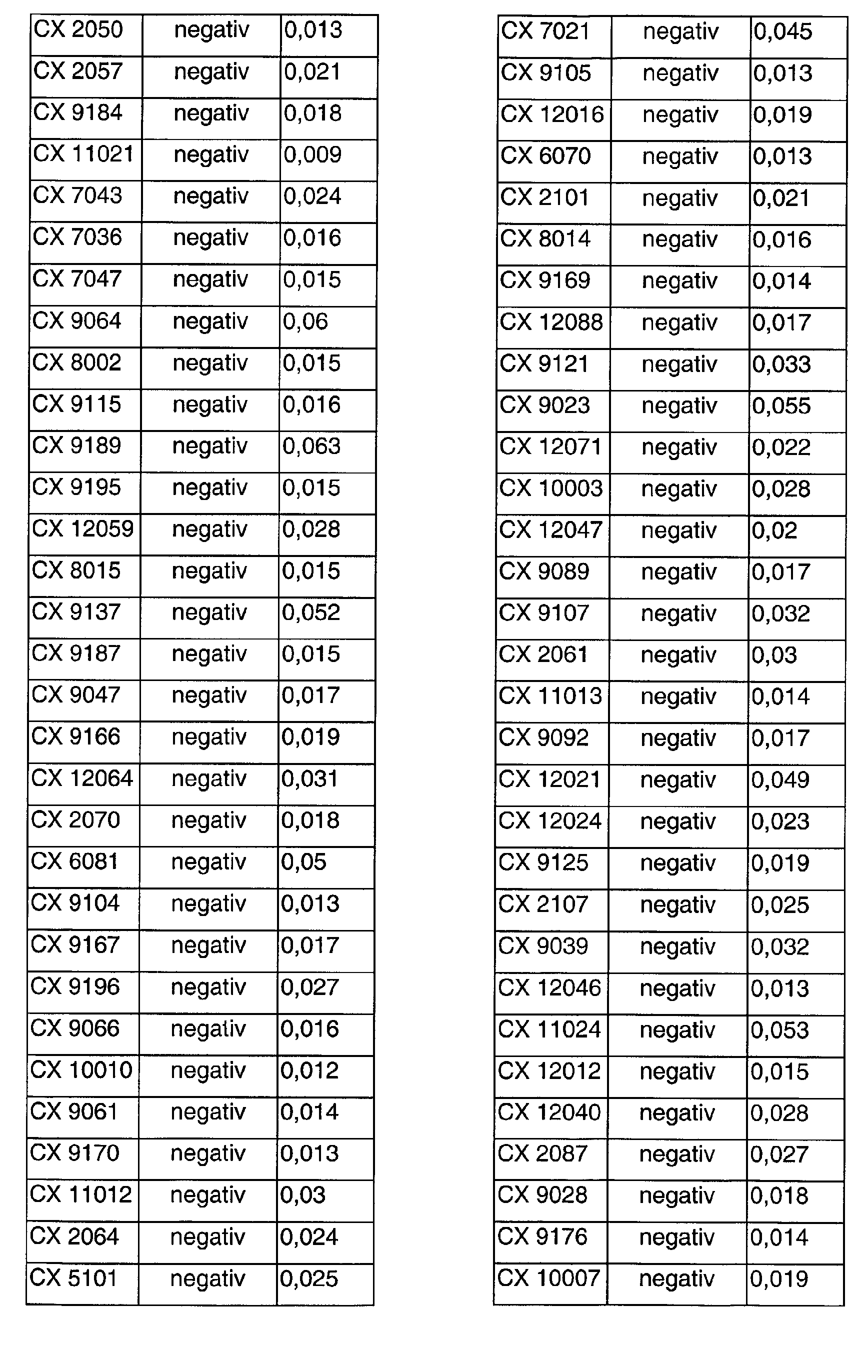

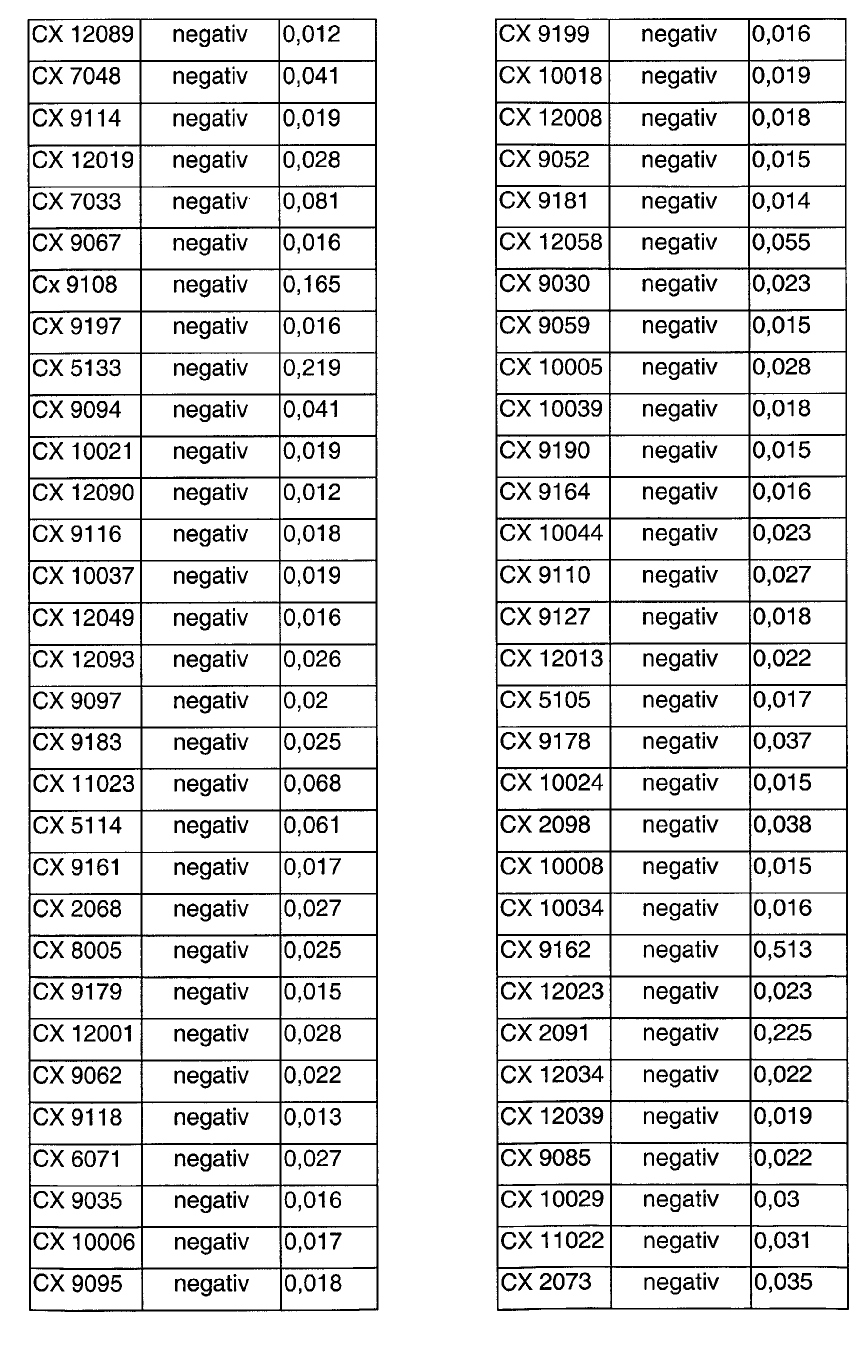

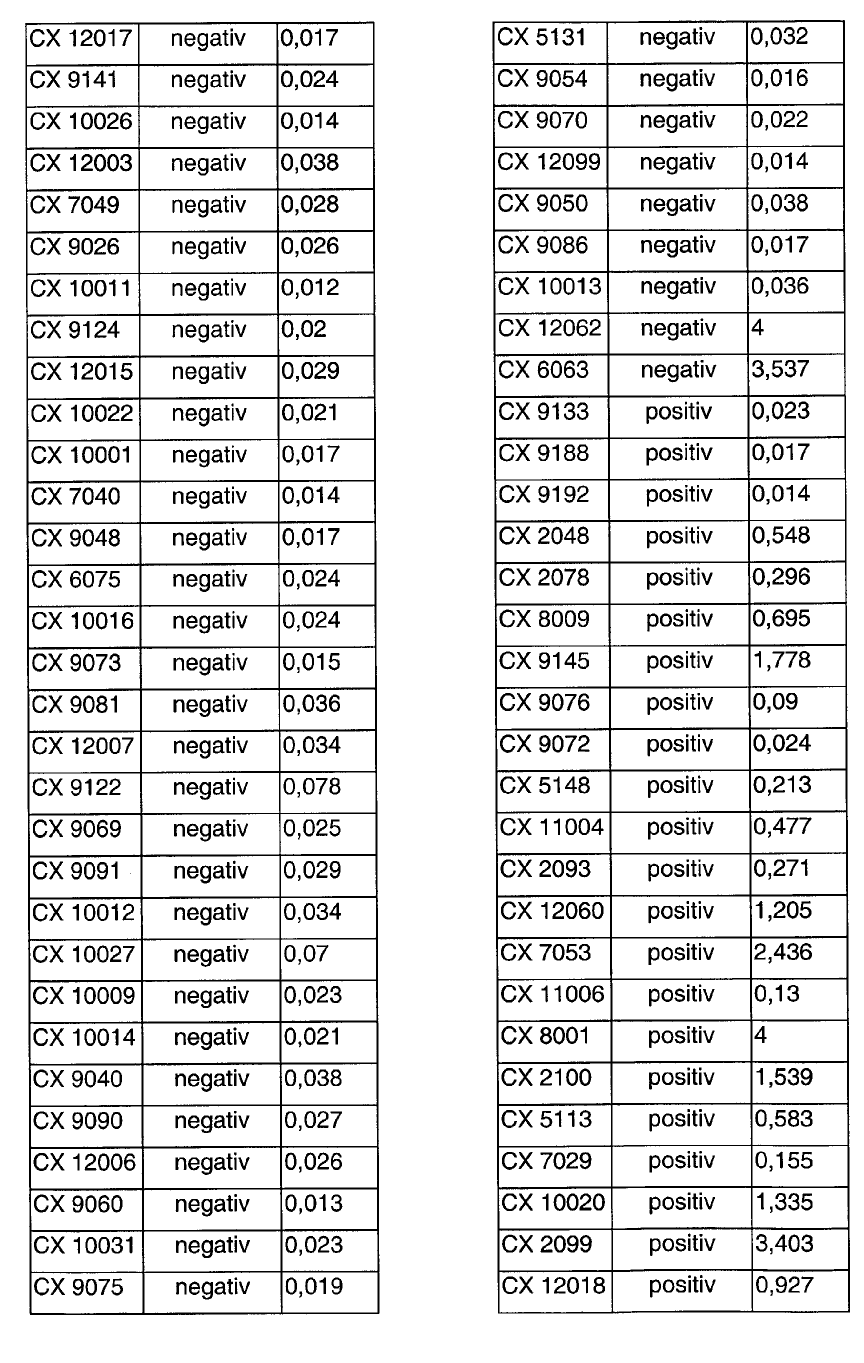

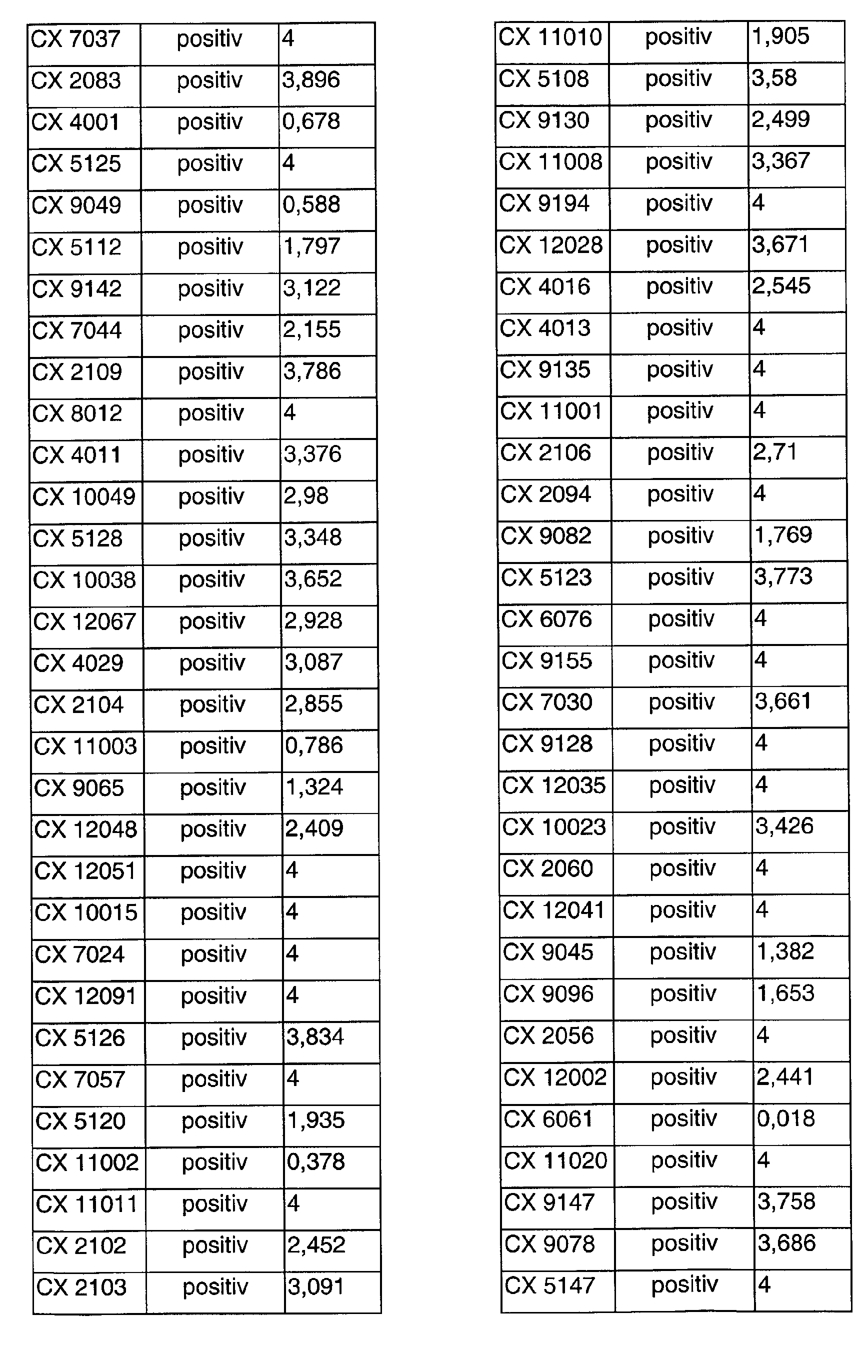

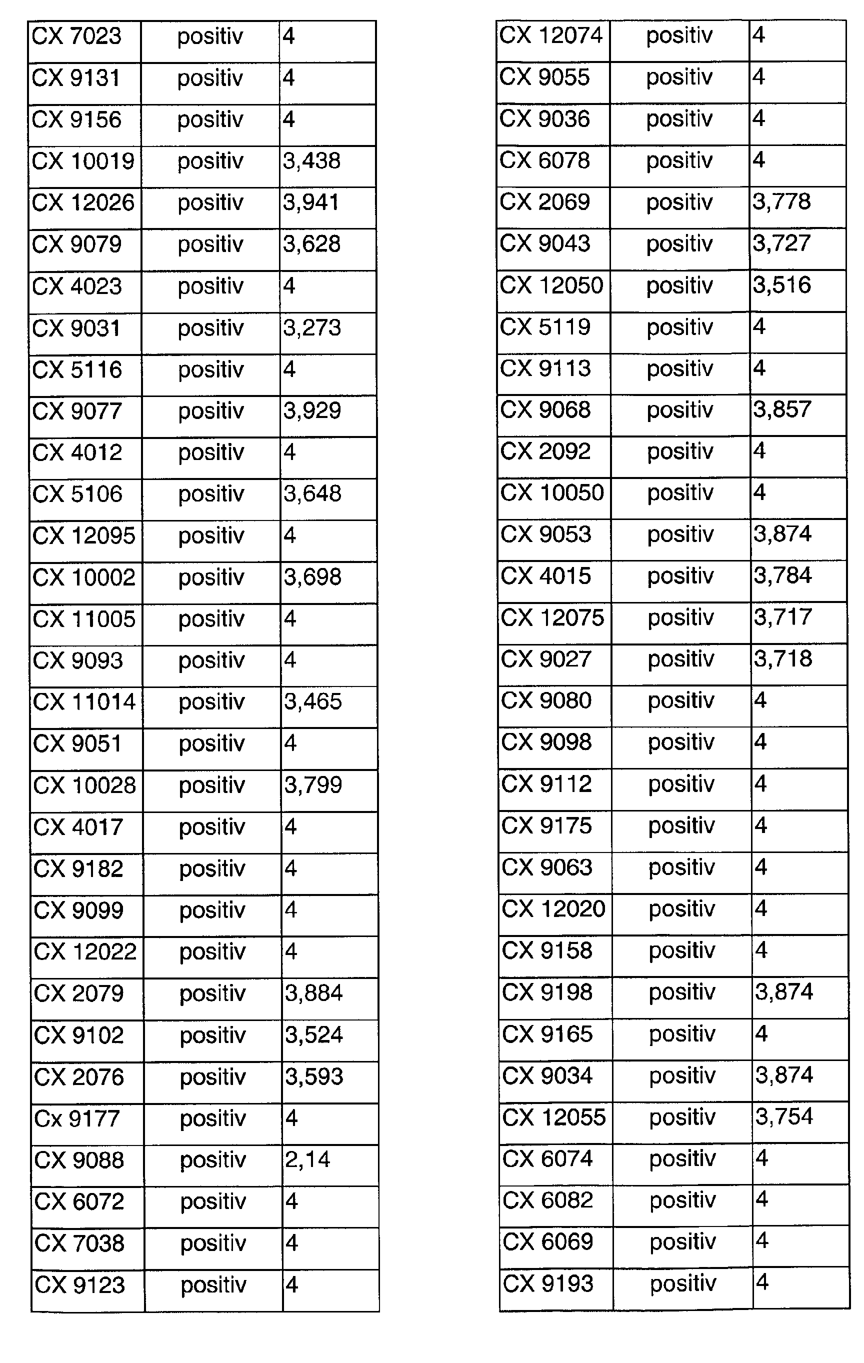

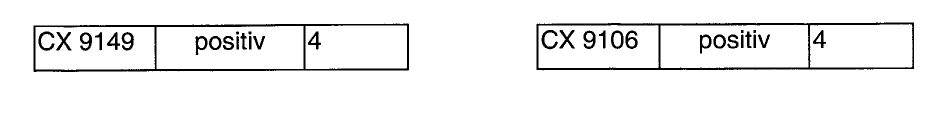

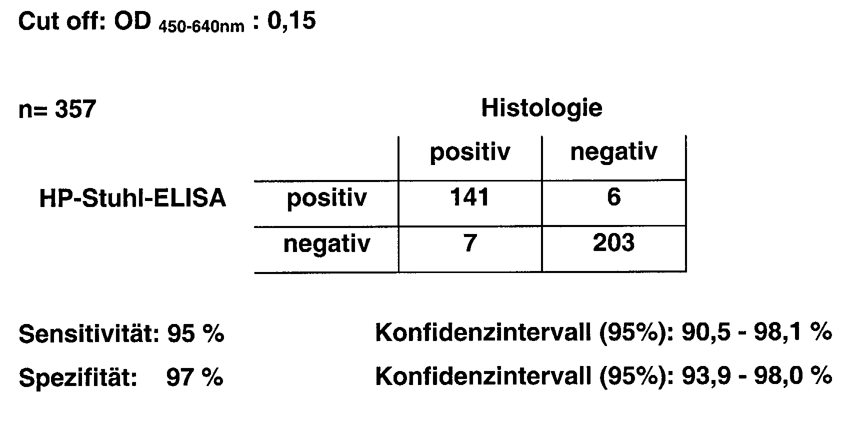

### Ergebnis:

Tabelle 8 zeigt die Ergebnisse der Untersuchung von *H. pylori*-negativen und *H. pylori*-positiven Stuhlproben (Erstdiagnose) mittels eines Stuhl-Sandwich-ELISA. Dabei wurden monoklonale Antikörper (Fang-Antikörper: HP25.6m/1B5; Detektionsantikörper HP25.2m/2H10-POD) für die Detektion des *H. pylori*-Antigens Katalase aus der Stuhlprobe eingesetzt. Die Untersuchung von Stuhlproben im rein monoklonalen ELISA-System, das nur auf Katalase-spezifische mAK aufgebaut ist, weist eine Sensitivität von 95 % und eine Spezifität von 97% auf.

### Beispiel 14: Eradikationsverlauf / Eradikationskontrolle

Eine Eradikationskontrolle ist nur über einen direkten Nachweis von *H. pylori*-Antigenen und nicht von Antikörper im Serum möglich, da *H. pylori*-Antikörper nach einer Infektion noch über viele Monate im Blut präsent sind. Daher bietet der dargestellte Sandwich-Stuhl-ELISA im Gegensatz zu serologischen *H. pylori*-Tests die Möglichkeit, den Erfolg einer Eradikation zu beurteilen.

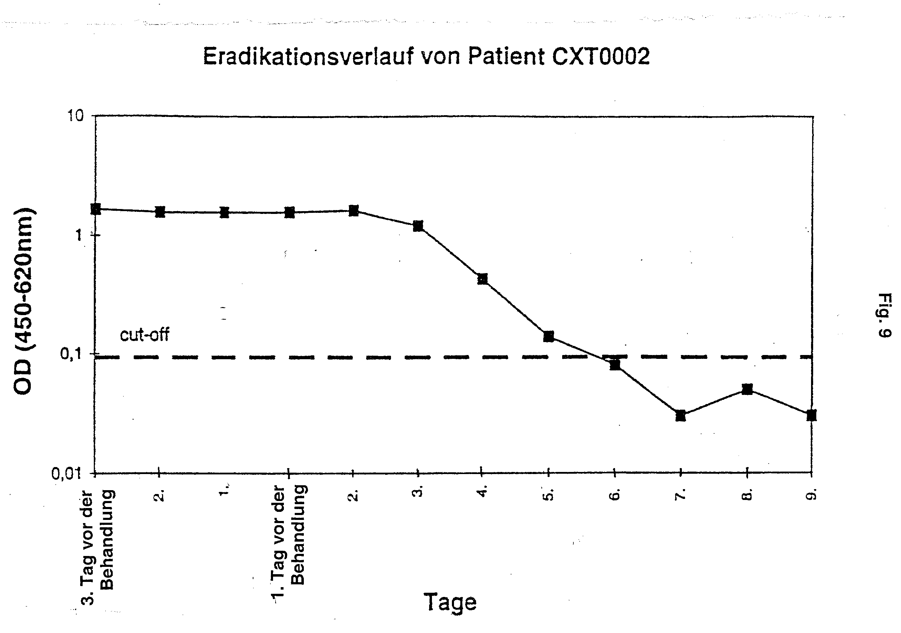

In Figur 9 wird der Verlauf einer Eradikationsbehandlung eines *H. pylori*-positiven Patienten nach Einnahme von Omeprazol, Metronidazol und Clarithromycin gezeigt. 6 Tage nach Beginn der Behandlung konnte kein *H. pylori*-Antigen mehr im Stuhl nachgewiesen werden.

Tabelle 9 zeigt die Ergebnisse des HP-Stuhl ELISAs einer Eradikationsstudie. Die Stuhlproben wurden 4 bis 6 Wochen nach Beendigung der Eradikationstherapie gesammelt. Als Referenztest diente der ¹³C-Atemtest.

Die Testdurchführung erfolgte gemäß Beispiel 12 (Einschritt-ELISA).

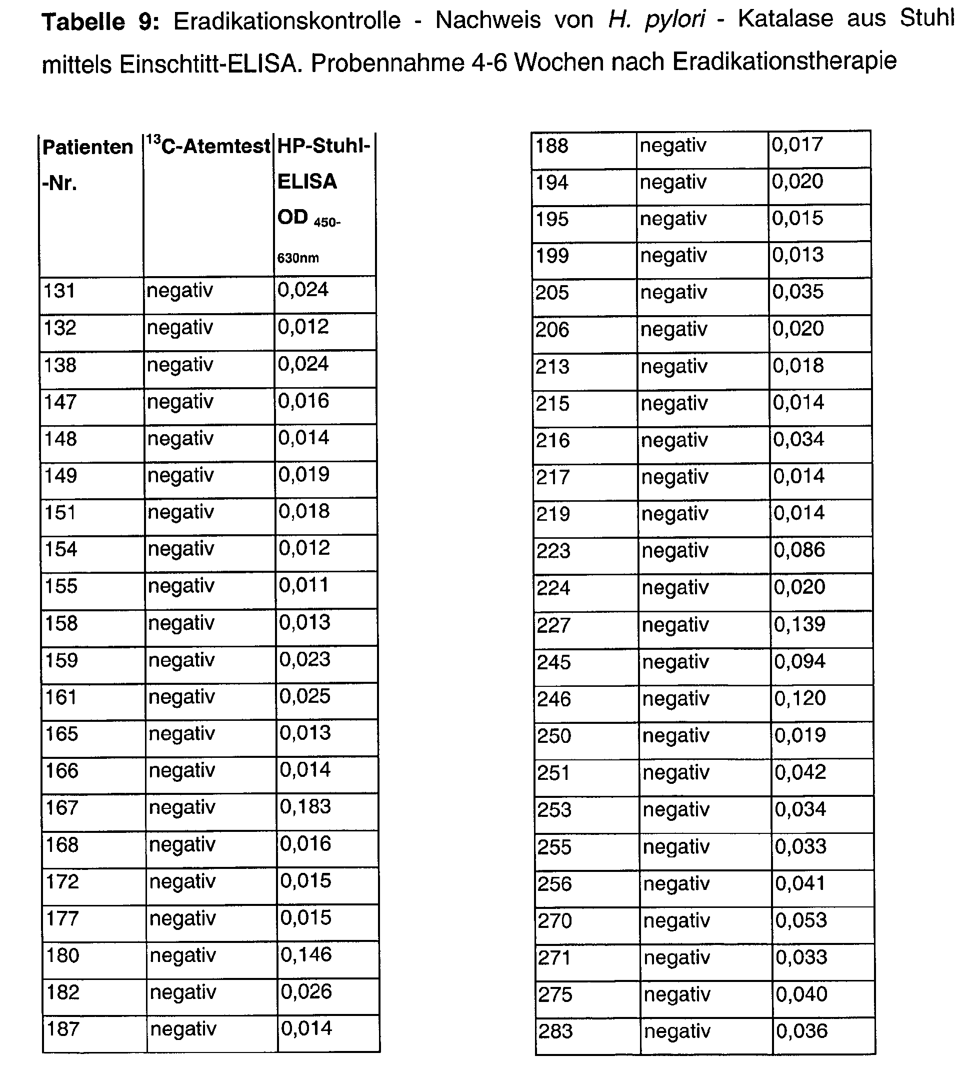

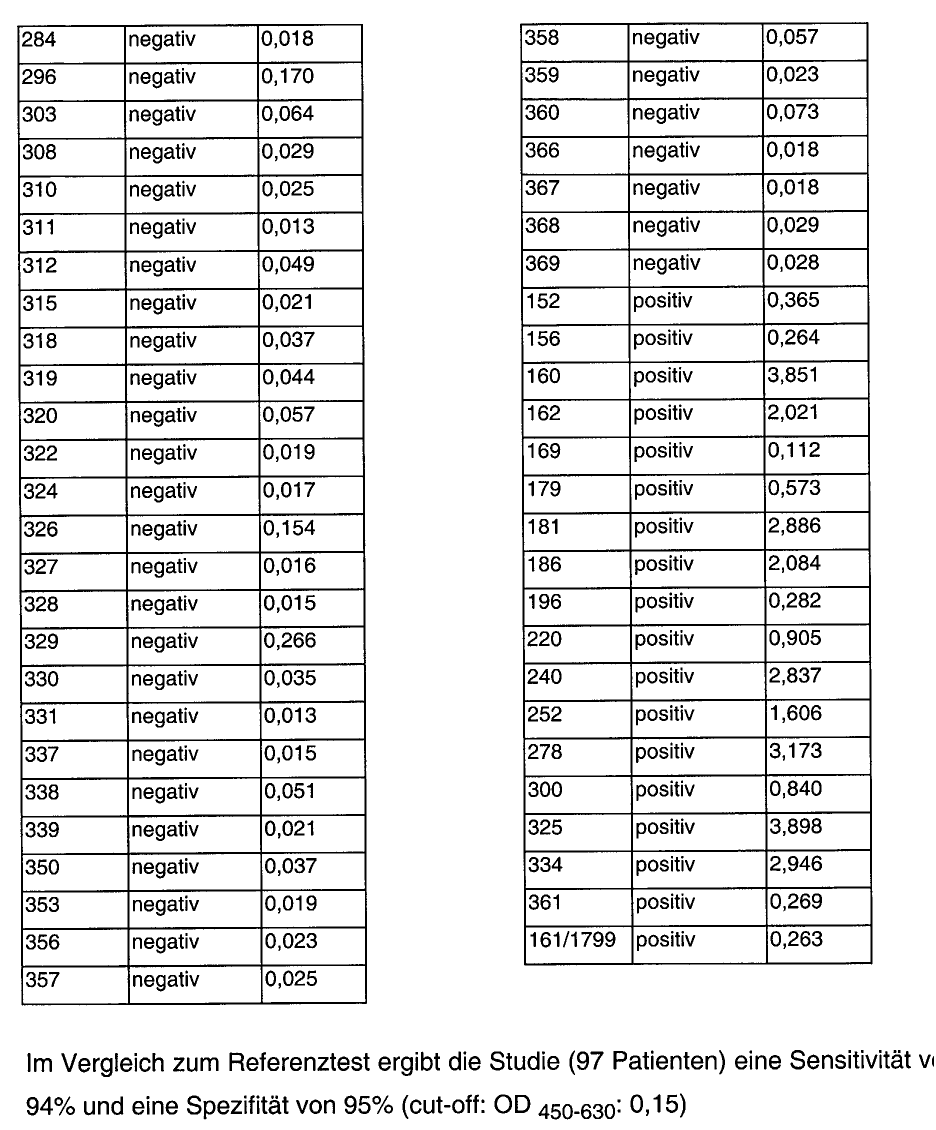

Beispiel 14 zeigt, daß der HP-Stuhl-ELISA nicht nur zum Nachweis einer *H. pylori* Erstdiagnose, sondern auch zur Eradikationskontrolle und zur Dokumentation des Eradikationsverlaufes geeignet ist.

### Beispiel 15: Klonierung und Sequenzbestimmung der funktionellen variablen Bereiche von Immunoglobulinen aus Hybridom-Zellinien

Gesamt-RNA wurde aus Antikörper-produzierenden Hybridom-Zellinien nach Chomczynski (Chomczynski, 1987) isoliert. Die entsprechende cDNA wurde nach Standard-Methoden synthetisiert (Sambrook et al., 1989)

Die DNA-Regionen, welche die kappa leichte Kette sowie das schwere Kette-Fd-Segment (VH bzw. CH1) der jeweiligen Antikörper kodieren, wurden mittels PCR amplifiziert. Dabei kam das in Tabelle 10 aufgeführte Oligunukleotid-Primer Set zur Anwendung. Die aus den einzelnen Hybridom-Zellinien isolierte cDNA diente als Matritze.

Das eingesetzte Primer-Set führt zu je einer 5'-*Xho* I und einer 3'-*Spe I* Schnittstelle in den schwere Kette-Fd-Fragmenten sowie je einer 5'-*Sac* I und einer 3'-*Xba* I-Schnittstelle in den kappa leichten Ketten. Zur PCR-Amplifikation der schwere Kette-Fd-kodierenden DNA-Fragmente wurden 11 verschiedene 5'-VH-Primer (MVH 1-8 und MULH1-3) jeweils kombiniert mit dem 3'-VH-Primer MlgG2a (HP25.2m/2H10) bzw. mit dem 3'-VH-Primer MlgG1 (HP25.6m/1B5) verwendet. Zur Amplifikation der DNA-Fragmente, welche für die kappa leichten Ketten codieren, wurden 11 verschiedene 5'-VK-Primer (MUVK 1-7 und MULK1-4) jeweils mit dem 3'-VK-Primer 3'MUCK kombiniert.

Das folgende Temperaturprogramm kam bei allen PCR-Amplifikationen zur Anwendung: Denaturierung bei 94°C für 30 s, Primer-Anlagerung bei 52°C für 60 s, Polymerisation bei 72°C für 90 s. Dieses Programm wurde für 40 Zyklen beibehalten, gefolgt von einer 10 min abschließenden Vervollständigung der Fragmente bei 72°C.

Die Ergebnisse der PCR-Amplifikationen wurden mittels Agarose-Gel-Elektrophorese aufgetrennt und DNA-Banden des erwarteten Molekulargewichts isoliert.

Für den Antikörper 25.2m/2H10 wurden die isolierten Banden anschließend einem Restriktionsverdau unter Einsatz der Enzyme *Xho* I und *Spe* I (schwere Ketten) bzw. *Sac* I und *Xba* I (leichte Ketten) unterzogen und die erhaltenenen Fragmente in den Plasmid-Vektor Bluescript KS (Stratagene) kloniert, nachdem dieser zunächst mit den Restriktionsenzymen *Xho* I und *Spe* I bzw. *Sac* I und *Xba* I gespalten worden war.

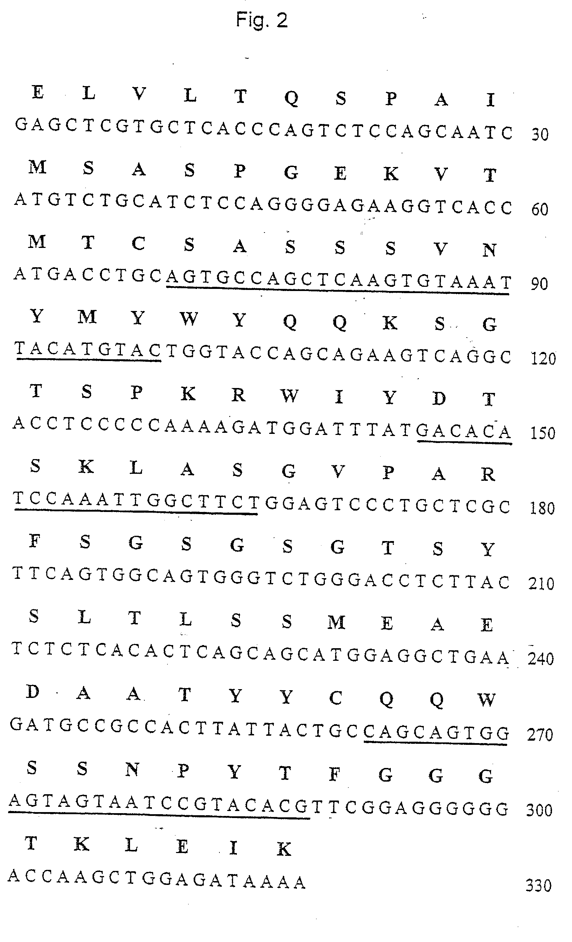

Plasmid-Präparationen der klonierten schwere und leichte Ketten-Fragmente wurden daraufhin sequenzanalysiert. Es wurden Sequenzen ausgewählt, die für funktionelle variable Bereiche der Immunglobulin schwere und leichte Kette (VH bzw. VL) codieren. Auf diese Weise konnte für diese Hybridom-Zellinie genau ein funktioneller VH- und ein funktioneller VL-Bereich identifiziert werden. Die funktionellen VH- und VL-Sequenzen sind in Fig. 1/Fig. 2 wiedergegeben. Die ersten vier Aminosäuren der VH-Region wurden durch Umklonierung ergänzt. Klonierung und Sequenzierung wurden nach Standardmethoden durchgeführt (Sambrook et al., 1989).

Für den Antikörper HP25.6m/1B5 wurden die isolierten Banden anschließend direkt sequenziert und eine funktionelle leichte und eine funktionelle schwere Kette identifiziert. Das schwere-Ketten-Fd-Fragment und die leichte Kette wurden anschließend einem Restriktionsverdau unter Einsatz der Enzyme Xhol und Spel (Schwere Kette) bzw. Sacl und Xbal (Leichte Kette) unterzogen und die erhaltenenen Fragmente in den Plasmid-Vektor pBSIIIHisEx (Connex) kloniert, nachdem dieser mit den Restriktionsenzymen *Xho* I und *Spe* I bzw. *Sac* I und *Xba* I gespalten worden war, und erneut sequenziert.

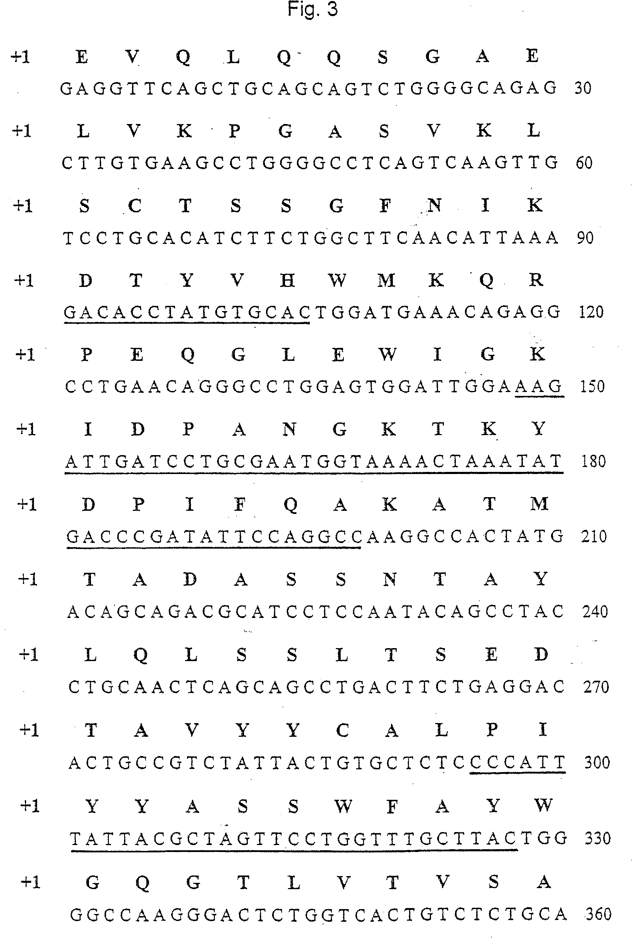

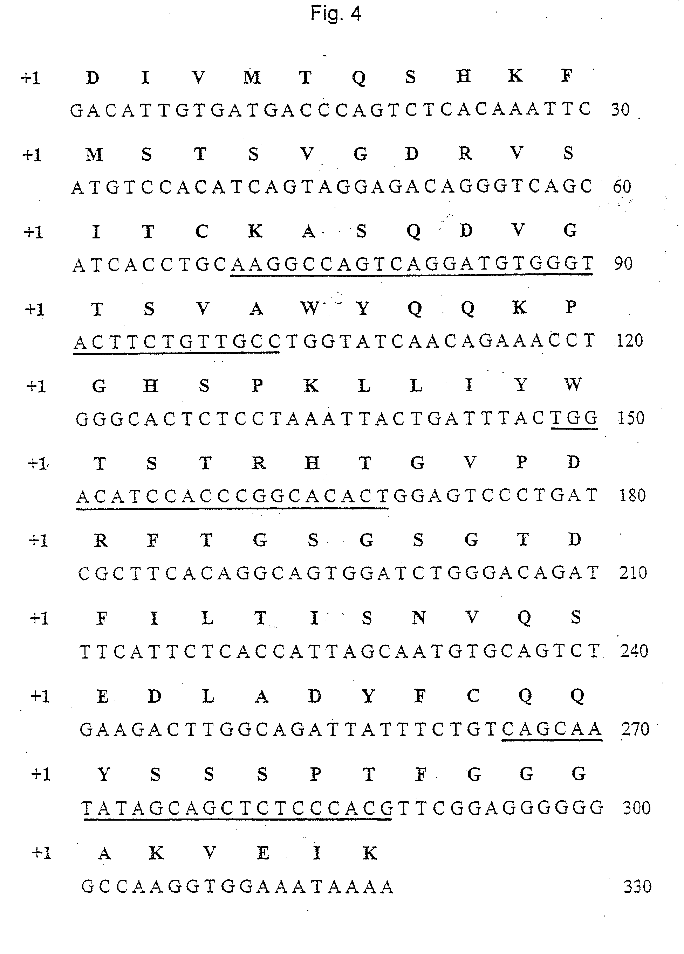

Auf diese Weise konnte für diese Hybridom-Zellinie genau ein funktioneller VH- und ein funktioneller VL-Bereich identifiziert werden. Die funktionellen VH- und VL-Sequenzen sind in Fig. 3/Fig. 4 wiedergegeben. Bei den VH- und VL Sequenzen sind die reifen N-Termini dargestellt, wie sie durch die Sequenzierung mittels Leaderprimer ermittelt wurden. Klonierung und Sequenzierung wurden nach Standardmethoden durchgeführt (Sambrook et al., 1989).

Tabelle 10: Liste der für die PCR-Amplifikation der funktionellen variablen Bereiche
von schweren und leichten Immunglobulin-Ketten verwendeten Primer
(Orientierung 5' - 3')

MVH1 | (GC)AG GTG CAG CTC GAG GAG TCA GGA CCT
MVH2 | GAG GTC CAG CTC GAG CAG TCT GGA CCT
MVH3 | CAG GTC CAA CTC GAG CAG CCT GGG GCT
MVH4 | GAG GTT CAG CTC GAG CAG TCT GGG GCA
MVH5 | GA(AG) GTG AAG CTC GAG GAG TCT GGA GGA
MVH6 | GAG GTG AAG CTT CTC GAG TCT GGA GGT
MVH7 | GAA GTG AAG CTC GAG GAG TCT GGG GGA
MVH8 | GAG GTT CAG CTC GAG CAG TCT GGA GCT
MULK1 | GGG GAG CTC CAC CAT GGA GAC AGA CAC ACT CCT GCT AT
MULK2 | GGG GAG CTC CAC CAT GGA TTT TCA AGT GCA GAT TTT CAG
MULK3 | GGG GAG CTC CAC CAT GGA GWC ACA KWC TCA GGT CTT TRT A
MULK4 | GGG GAG CTC CAC CAT GKC CCC WRC TCA GYT YCT KGT
MIgG1 | TAT GCA ACT AGT ACA ACC ACA ATC CCT GGG
MIgG2a | GAG AGA GGG GTT CTG ACT AGT GGG CAC TCT GGG CTC
MUVK1 | CCA GTT CCG AGC TCG TTG TGA CTC AGG AAT CT
MUVK2 | CCA GTT CCG AGC TCG TGT TGA CGC AGC CGC CC
MUVK3 | CCA GTT CCG AGC TCG TGC TCA CCC AGT CTC CA
MUVK4 | CCA GTT CCG AGC TCC AGA TGA CCC AGT CTC CA
MUVK5 | CCA GAT GTG AGC TCG TGA TGA CCC AGA CTC CA
MUVK6 | CCA GAT GTG AGC TCG TCA TGA CCC AGT CTC CA
MUVK7 | CCA GTT CCG AGC TCG TGA TGA CAC AGT CTC CA
MULH1 | GGG CTC GAG CAC CAT GGR ATG SAG CTG KGT MAT SCT CTT
MULH2 | GGG CTC GAG CAC CAT GRA CTT CGG GYT GAG CTK GGT TTT
MULH3 | GGG CTC GAG CAC CAT GGC TGT CTT GGG GCT GCT CTT CT
3`MUCK | GCG CCG TCT AGA ATT AAC ACT CAT TCC TGT TGA A

### Literatur:

**Coller & Coller, 1983:** Coller, H.A., Coller, B.S., Meth. Enzymol. **121**:412-417 **Harlow & Lane, 1988:** Harlow, E., Lane, D., Antibodies: A laboratory manual, Cold Spring Harbour Laboratory, New York
**Kearney et al., 1979:** Kearney, J. Immunol. **123**: 1548-1550
**Laemmli, 1970:** Laemmli, E.K., Nature **227**: 680-685
**Peters & Baumgarten, 1990:** Peters, J.H., Baumgarten, H. (Hrsg.), Monoklonale Antikörper, Springer Verlag, Berlin
**Fägerstam et al., 1990:** Fägerstam, L.G. et al., J. Mol. Recognit. **3**: 208-214
**Malmqvist, 1996:** Methods **9:** 525-532
**Eschweiler et al., 1993:** Eschweiler, B., et al., Zentralbl. F. Bakt. **280**: 73-85 **Pharmacia Biotech, 1994:** Monoclonal Antibody Purification Handbook
**Chomczynski, 1987:** Anal. Biochem. **162:** 156-159
**Sambrook et al., 1989:** Molecular cloning: A laboratory manual, Cold Spring Harbour Laboratory Press, second edition
**Vaughan et al., 1998:** Nature Biotechnology **16:** 535-539
**Orlandi et al., 1989:** Proc. Natl. Acad. Sci USA **86**: 3833-3837
**Janeway & Travers, 1997:** Immunologie, 2. Auflage, Spektrum Akademischer Verlag GmbH, Heidelberg
**Osborne et al., 1997:** Curr. Opin. Chem. Biol. **1:** 5-9
**Stull und Szoka, 1995:** Pharm. Res. **12:** 465-483
**Frens, 1973:** Nat. Phys. Sci. **241,** 20-23
**Geoghegan and Ackerman, 1977:** J. of Histochemistry and Cytochemistry, **25(11)**, 1187-1200
**Slot, 1985:** Eur. J. Cell Biol. **38**, 87-93
**Manos et al., 1998:** Helicobacter **3** (1), 28-38
**Haque, 1993:** J. Infect. Dis. **167:** 247-9
**Park, 1996:** J. Clin. Microbiol. **34**: 988-990
**Hasan, 1994:** FEMS Microbiol. Lett. 120: 143-148
**Koopmans, 1993:** J. Clin. Microbiol. 31: 2738-2744
**Machnicka,1996:** Appl. Parasitol. 37: 106-110

## Patentansprüche

1. Verfahren zum Nachweis einer Infektion eines Säugers mit einem Säure-resistenten Mikroorganismus der Gattung Helicobacter wobei man
(a) eine Stuhlprobe des Säugers unter Verwendung (aa) eines Rezeptors unter Bedingungen inkubiert, die eine Komplexbildung eines Antigens aus dem Säure-resistenten Mikroorganismus mit dem Rezeptor erlauben; oder (ab) zwei unterschiedliche Rezeptoren unter Bedingungen inkubiert, die eine Komplexbildung eines Antigens aus dem Säure-resistenten Mikroorganismus mit den beiden Rezeptoren erlauben und wobei der Rezeptor gemäß (aa) oder die Rezeptoren gemäß (ab) ein Antigen spezifisch bindet/binden, das zumindest bei einem Teil der Säuger nach der Darmpassage eine Struktur aufweist, die der nativen Struktur oder der Struktur entspricht, gegen die ein Säuger nach Infektion oder Immunisierung mit dem Säure-resistenten Mikroorganismus oder einem Extrakt oder Lysat davon oder einem Protein daraus oder einem Fragment davon oder einem synthetischen Peptid Antikörper produziert; und
(b) die Bildung mindestens eines Antigen-Rezeptorkomplexes gemäß (a) nachweist.

2. Verfahren nach Anspruch1, wobei das Bakterium ein Bakterium der Spezies *Helicobacter pylori oder Helicobacter hepaticus,* ist.

3. Verfahren nach einem der Ansprüche 1 oder 2, wobei das Antigen das Antigen einer Katalase oder einer Metalloproteinase, ist.

4. Verfahren nach Anspruch 3, wobei das Antigen ein Antigen von H. pylori ist.

5. Verfahren nach einem der Ansprüche 1 bis 4, wobei der Rezeptor/die Rezeptoren (ein) Antikörper, (ein) Fragment(e) oder Derivat(e) davon oder (ein) Aptamer(e) ist/sind.

6. Verfahren nach einem der Ansprüche 1 bis 5, wobei für den Nachweis zusätzlich ein Gemisch von Rezeptoren eingesetzt wird, wobei das Gemisch von Rezeptoren als Fänger des Antigens fungiert, wenn der Rezeptor als Detektor des Antigens eingesetzt wird und das Gemisch als Detektor des Antigens fungiert, wenn der Rezeptor als Fänger des Antigens eingesetzt wird.

7. Verfahren nach Anspruch 6, wobei das Gemisch von Rezeptoren ein polyklonales Antiserum ist.

8. Verfahren nach Anspruch 7, wobei das polyklonale Antiserum gegen ein Lysat des Mikroorganismus gewonnen wurde.

9. Verfahren nach Anspruch 8, wobei das Lysat ein Lysat mit angereichertem Antigen ist.

10. Verfahren nach Anspruch 8 oder 9, wobei das Lysat ein Lysat mit abgereicherten immundominanten Antigenen ist.

11. Verfahren nach Anspruch 7, wobei das polyklonale Antiserum gegen ein aufgereinigtes oder ein (semi)synthetisch hergestelltes Antigen gewonnen wurde.

12. Verfahren nach einem der Ansprüche 1 bis 11, wobei der Rezeptor und/oder das Gemisch von Rezeptoren (ein) Konformationsepitop(e) bindet/n.

13. Verfahren nach einem der Ansprüche 5 bis 12, wobei die schwere Kette des ein Katalase-Epitop bindenden Antikörpers mindestens eine der folgenden CDRs, und vorzugsweise die CDR3 aufweist:

CDR1: NYWIH
CDR2: YINPATGSTSYNQDFQD
CDR3: EGYDGFDS

14. Verfahren nach Anspruch 13, wobei die schwere Kette alle drei genannten CDRs aufweist.

15. Verfahren nach Anspruch 13 oder 14, wobei die die schwere Kette des Antikörpers kodierende DNA-Sequenz mindestens eine der folgenden CDRs, vorzugsweise die CDR3 und weiter bevorzugt alle drei folgenden CDRs aufweist:

CDR1: AACTACTGGA TTCAC
CDR2: TACATTAATC CTGCCACTGG TTCCACTTCT TACAATCAGG
ACTTTCAGGA C
CDR3: GAGGGGTACG ACGGGTTTGA CTCC

16. Verfahren nach einem der Ansprüche 5 bis 12, wobei die leichte Kette des ein Katalase-Epitop bindenden Antikörpers mindestens eine der folgenden CDRs, vorzugsweise die CDR3 und weiter bevorzugt alle drei folgenden CDRs aufweist:

CDR1: SASSSVNYMY
CDR2: DTSKLAS
CDR3: QQWSSNPYT

17. Verfahren nach Anspruch 16, wobei die die leichte Kette des Antikörpers kodierende DNA-Sequenz mindestens eine der folgenden CDRs, vorzugsweise die CDR3 und weiter bevorzugt alle drei folgenden CDRs aufweist:

CDR1: AGTGCCAGCT CAAGTGTAAA TTACATGTAC
CDR2: GACACATCCA AATTGGCTTC T
CDR3: CAGCAGTGGA GTAGTAATCC GTACACG

18. Verfahren bzw. Test nach einem der Ansprüche 5 bis 12, wobei die schwere Kette des ein Katalase-Epitop bindenden Antikörpers mindestens eine der folgenden CDRs, vorzugsweise die CDR3 und weiter bevorzugt alle drei folgenden CDRs aufweist:

CDR1: DTYVH
CDR2: KIDPANGKTKYDPIFQA
CDR3: PIYYASSWFAY

19. Verfahren nach Anspruch 18, wobei die die schwere Kette des Antikörpers kodierende DNA-Sequenz mindestens eine der folgenden CDRs, vorzugsweise die CDR3 und weiter bevorzugt alle drei folgenden CDRs aufweist:

CDR1: GACACCTATGTGCAC
CDR2: AAGATTGATCCTGCGAATGGTAAAACTAAATAT
GACCCGATATTCCAGGCC
CDR3: CCCATTTATTACGCTAGTTCCTGGTTTGCTTAC

20. Verfahren nach einem der Ansprüche 5 bis 12, wobei die die leichte Kette des ein Katalase-Epitop bindenden Antikörpers mindestens eine der folgenden CDRs, vorzugsweise die CDR3 und weiter bevorzugt alle drei folgenden CDRs aufweist:

CDR1: KASQDVGTSVA
CDR2: WTSTRHT
CDR3: QYSSSPT

21. Verfahren nach Anspruch 20, wobei die die leichte Kette des Antikörpers kodierende DNA-Sequenz mindestens eine der folgenden CDRs, vorzugsweise die CDR3 und weiter bevorzugt alle drei folgenden CDRs aufweist:

CDR1: AAGGCCAGTCAGGATGTGGGTACTTCTGTTGCC
CDR2: TGGACATCCACCCGGCACACT
CDR3: CAGCAATATAGCAGCTCTCCCACG

22. Verfahren nach einem der Ansprüche 5 bis 21, wobei die Antikörper in den variablen Regionen der leichten und schweren Ketten die in den Figuren 1 und 2, 3 und 4, dargestellten Aminosäuresequenzen aufweisen.

23. Verfahren nach einem der Ansprüche 5 bis 22, wobei die kodierenden Bereiche der variablen Regionen der leichten und schweren Ketten die in den Figuren 1 und 2, 3 und 4, dargestellten DNA-Sequenzen aufweisen.

24. Verfahren nach einem der Ansprüche 1 bis 23, wobei mit der Stuhlprobe vor der Inkubation mit den Antikörpern folgende Schritte durchgeführt werden:
(a) Resuspendieren der Stuhlprobe 1:3 bis 1:25, vorzugsweise etwa 1:5 bis 1:10, besonders bevorzugt 1:5 in Resuspendierungspuffer und
(b) Mischen auf einem Vortexmixer.

25. Verfahren nach einem der Ansprüche 1 bis 24, wobei der Nachweis der Bildung des mindestens einen Antigen-Rezeptorkomplexes/Antigen-Rezeptor-Rezeptorgemischkomplexes in Schritt (b) mittels eines immunologischen Verfahrens erfolgt.

26. Verfahren nach einem der Ansprüche 1 bis 25, wobei der Nachweis der Bildung des mindestens einen Antigen-Rezeptorkomplexes/Antigen-Rezeptor-/Rezeptorgemischkomplexes in Schritt (b) mittels ELISA, RIA, Western Blot oder eines immunchromatographischen Verfahrens erfolgt.

27. Verfahren nach Anspruch 25 oder 26, wobei im RIA oder im ELISA der gleiche Rezeptor zur Bindung an die Festphase wie zum Nachweis des Epitops eingesetzt wird.

28. Verfahren nach einem der Ansprüche 1 bis 27, wobei der Rezeptor an einen Träger fixiert ist.

29. Verfahren nach einem der Ansprüche 1 bis 28, wobei der Rezeptor ein monoklonaler Maus-Antikörper ist.

30. Verfahren nach einem der Ansprüche 1 bis 29, bei dem das Verfahren ein Einschritt-ELISA ist.

31. Verfahren nach einem der Ansprüche 1 bis 29, bei dem das Verfahren ein Dreischritt-ELISA ist.

32. Verfahren nach Anspruch28, wobei das Trägermaterial des Trägers ein poröses Trägermaterial ist.

33. Verfahren nach Anspruch 28 und32, wobei das Trägermaterial ein Teststreifen ist.

34. Verfahren nach Anspruch 28, 32 oder 33, wobei das Trägermaterial aus Zellulose oder einem Zellulosederivat besteht.

35. Verfahren nach einem der Ansprüche 1 bis 34, bei dem anstelle einer Stuhlprobe Atemkondensat, Magengase, Schleimhautabstriche, Vollblut oder
Serum zum Nachweis eingesetzt wird.

36. Verfahren nach einem der Ansprüche 1 bis 35, bei dem es sich um ein automatisiertes Verfahren handelt.

37. Verfahren nach einem der Ansprüche 1 bis 36, wobei der Säuger ein Mensch ist.

38. Verfahren nach einem der Ansprüche 1, 2, 4, bis 12, 24 bis 37 wobei das Antigen das Antigen einer Urease ist.

39. Verfahren nach einem der Ansprüche 5 bis 14, wobei die schwere Kette des ein Epitop der β-Urease bindenden Antikörpers mindestens eine der folgenden CDRs, vorzugsweise die CDR3 und weiter bevorzugt alle drei folgenden CDRs aufweist:

CDR1: GFTFSSHFMS
CDR2: SISSGGDSFYPDSLKG
CDR3: DYSWYALDY
oder:

CDR1: GYAFSTSWMN
CDR2: RIYPGDGDTNYNGKFKG
CDR3: EDAYYSNPYSLDY

40. Verfahren nach Anspruch 23, wobei die die schwere Kette kodierende DNA-Sequenz des Antikörpers mindestens eine der folgenden CDRs, vorzugsweise die CDR3 und weiter bevorzugt alle drei folgenden CDRs aufweist:

CDR1: GG CTACGCATTC AGTACCTCCT GGATGAAC
CDR2: CGGATTTATC CTGGAGATGG AGATACTAAC TACAATGGGA
AGTTCAAGGG C
CDR3: GAG GATGCCTATT ATAGTAACCC CTATAGTTTG GACTAC
oder:
CDR1: GG ATTCACTTTC AGTAGCCATT TCATGTCT
CDR2: TCCATTAGTA GTGGTGGTGA CAGTTTCTAT CCAGACAGTC
TGAAGGGC
CDR3: GACTAC TCTTGGTATG CTTTGGACTA C

41. Verfahren nach einem der Ansprüche 5 bis 14, wobei die leichte Kette des ein Epitop der β-Urease bindenden Antikörpers mindestens eine der folgenden CDRs, vorzugsweise die CDR3 und weiter bevorzugt alle drei folgenden CDRs aufweist:

CDR1: RASQSIGTRIH
CDR2: YGSESIS
CDR3: QQSNTWPLT
oder:
CDR1: HASQNINVWLS
CDR2: KASNLHT
CDR3: QQGRSYPLT

42. Verfahren nach Anspruch 25, wobei die die leichte Kette des Antikörpers kodierende DNA-Sequenz mindestens eine der folgenden CDRs, vorzugsweise die CDR3 und weiter bevorzugt alle drei folgenden CDRs aufweist:

CDR1: A GGGCCAGTCA GAGCATTGGC ACAAGAATAC AC
CDR2: TAT GGTTCTGAGT CTATCTCT
CDR3: CAACAA AGTAATACCT GGCCGCTCAC G
oder:
CDR1: C ATGCCAGTCA GAACATTAAT GTTTGGTTAA GC
CDR2: AAG GCTTCCAACT TGCACACA
CDR3: CAACAG GGTCGAAGTT ATCCTCTCAC G

43. Monoklonaler Antikörper, Fragment oder Derivat davon, der/das eine V-Region aufweist, die eine Kombination der in einem der Ansprüche 13 bis 21 dargestellten CDRs aufweist.

44. Monoklonaler Antikörper, Fragment oder Derivat davon nach Anspruch 43, der/das mindestens eine der in den Figuren 1 und 2, 3 und 4, dargestellten V-Regionen aufweist.

45. Monoklonaler Antikörper, Fragment oder Derivat davon nach Anspruch 43 oder 44, der ein Maus-Antikörper oder ein Fragment oder Derivat davon oder ein chimärer, vorzugsweise ein humanisierter Antikörper oder ein Fragment oder Derivat davon ist.

46. Aptamer, das dasselbe Epitop wie der monoklonale Antikörper, das Fragment oder Derivat davon nach einem der Ansprüche 43 bis 45 spezifisch bindet.

47. Epitop, das von einem monoklonaler Antikörper, Fragment oder Derivat davon nach einem der Ansprüche 43 bis 45 oder dem Aptamer nach Anspruch 46 spezifisch gebunden wird.

48. Antikörper, Fragment oder Derivat davon, der/das ein Epitop nach Anspruch 47 spezifisch bindet.

49. Diagnostische Zusammensetzung enthaltend mindestens einen Rezeptor wie in einem der Ansprüche 3 bis 37, 43 bis 45 definiert, gegebenenfalls fixiert an ein Trägermaterial, die gegebenenfalls ferner ein Gemisch von Rezeptoren wie in einem der vorstehenden Ansprüche definiert enthält, gegebenenfalls fixiert an ein Trägermaterial.

50. Testvorrichtung zum Nachweis mindestens eines wie in einem der vorstehenden Ansprüchen definierten Epitops, umfassend
(a) mindestens einen Rezeptor wie in einem der Ansprüche 3 bis 37, 43 bis 45 definiert, fixiert an ein Trägermaterial;
(b) eine Vorrichtung zur Aufbereitung und Analyse von Stuhlproben; und gegebenenfalls
(c) ein Gemisch von Rezeptoren wie in einem der vorstehenden Ansprüche definiert.

51. Testvorrichtung, zum Nachweis mindestens eines wie in einem der vorstehenden Ansprüchen definierten Epitops, umfassend
(a) mindestens einen Rezeptor wie in einem der Ansprüche 3 bis 37, 43 bis 45 definiert, wobei der Rezeptor konjugiert ist mit kolloidalem Gold, Latexpartikeln oder anderen farbgebenden Partikeln, deren Größe typischerweise im Bereich zwischen 5nm und 100nm, vorzugsweise zwischen 20nm und 60nm liegt; besonders bevorzugt zwischen 40nm und 60nm (Gold) bzw. 200 nm bis 500nm (Latex)
(b) eine Vorrichtung zur Aufbereitung und Analyse von Stuhlproben; und gegebenenfalls
c) ein Gemisch von Rezeptoren wie in einem der vorstehenden Ansprüche definiert.

52. Kit enthaltend
(a) mindestens einen Rezeptor wie in einem der Ansprüche 3 bis 37, 43 bis 45 definiert, gegebenenfalls fixiert an ein Trägermaterial; gegebenenfalls ferner
(b) eine Vorrichtung zur Aufbereitung und Analyse von Stuhlproben; und gegebenenfalls
c) ein Gemisch von Rezeptoren wie in einem der vorstehenden Ansprüche definiert.

53. Diagnostische Zusammensetzung zur Verwendung in dem Verfahren nach einem der Ansprüche 1 bis 42 enthaltend mindestens einen Rezeptor der mit einem Antigen aus dem säureresistenten Bakterium einen Komplex bildet, wie in einem der Ansprüche 1 bis 42 beschrieben, gegebenenfalls fixiert an ein Trägermaterial, die gegebenenfalls ferner ein Gemisch von Rezeptoren wie in einem der vorstehenden Ansprüche definiert enthält, gegebenenfalls fixiert an ein Trägermaterial.

54. Testvorrichtung zur Verwendung in dem Verfahren nach einem der Ansprüche 1 bis 42 zum Nachweis mindestens eines wie in einem der vorstehenden Ansprüchen definierten Epitops, umfassend
(a) mindestens einen Rezeptor der mit einem Antigen aus dem säureresistenten Bakterium einen Komplex bildet, wie in Anspruch 1-42 beschrieben), fixiert an ein Trägermaterial;
(b) eine Vorrichtung zur Aufbereitung und Analyse von Stuhlproben; und gegebenenfalls
(c) ein Gemisch von Rezeptoren wie in einem der vorstehenden Ansprüche definiert.

55. Testvorrichtung zur Verwendung in dem Verfahren nach einem der Ansprüche 1 bis 42 zum Nachweis mindestens eines wie in einem der vorstehenden Ansprüchen definierten Epitops, umfassend
(a) mindestens einen Rezeptor der mit einem Antigen aus dem säureresistenten Bakterium einen Komplex bildet, wie in Anspruch 1-42 beschrieben, wobei der Rezeptor konjugiert ist mit kolloidalem Gold, Latexpartikeln oder anderen farbgebenden Partikeln, deren Größe typischerweise im Bereich zwischen 5nm und 100nm, vorzugsweise zwischen 20nm und 60nm liegt; besonders bevorzugt zwischen 40nm und 60nm (Gold) bzw. 200 nm bis 500nm (Latex)
(b) eine Vorrichtung zur Aufbereitung und Analyse von Stuhlproben; und gegebenenfalls
c) ein Gemisch von Rezeptoren wie in einem der vorstehenden Ansprüche definiert.

56. Kit zur Verwendung in dem Verfahren nach einem der Ansprüche 1 bis 42 enthaltend
(a) mindestens einen Rezeptor der mit einem Antigen aus dem säureresistenten Bakterium einen Komplex bildet, wie in Anspruch 1-42 beschrieben, gegebenenfalls fixiert an ein Trägermaterial; gegebenenfalls ferner
(b) eine Vorrichtung zur Aufbereitung und Analyse von Stuhlproben; und gegebenenfalls
c) ein Gemisch von Rezeptoren wie in einem der vorstehenden Ansprüche definiert.

57. Zusammensetzung, vorzugsweise Arzneimittel enthaltend mindestens einen der vorstehend beschriebenen Rezeptoren gegebenenfalls in Kombination mit einem pharmazeutisch verträglichen Träger und/oder Verdünnungsmittel.

58. Packung enthaltend die diagnostische Zusammensetzung nach Anspruch 49 oder 53 die Testvorrichtung nach Anspruch 50, 51, 54 oder 55 oder den Kit nach Anspruch 52 oder 56.
